# EUROPEAN PATENT SPECIFICATION

(11) **EP 1 930 007 B1**
(45) Date of publication and mention of the grant of the patent: **09.06.2010**
(21) Application number: 08004276.5
(22) Date of filing: 04.04.2003
(51) Int. Cl.: A61K 31/495, A61P 9/06

(54) **Use of ranolazine for the preparation of a medicament for the treatment of early afterdepolarisations (EADs)**
Verwendung von Ranolazin zur Herstellung eines Medikaments zur Behandlung von frühen Nachdepolarisationen (EADs)
Utilisation de ranolazine pour la préparation d'un médicament pour le traitement des postdépolarizations précoces (EADs)

(30) Priority: 04.04.2002 US 370150 P; 05.09.2002 US 408292 P; 30.10.2002 US 422589 P
(43) Date of publication of application: 11.06.2008
(62) Divisional of application: 03723907.6
(73) Proprietor: Gilead Palo Alto, Inc., Foster City CA 94404 (US)
(72) Inventor: Belardinelli, Luiz, Palo Alto CA 94306 (US); Antzelevitch, Charles, New Hartford NY 13413 (US); Blackburn, Brent, Los Altos CA 94022 (US)
(74) Representative: Schnappauf, Georg

(56) References cited:
- WO-A-00/13687
- WO-A-01/60348
- US-A- 4 567 264
- US-B1- 6 303 607
- GRALINSKI MR, LIGUO CHI ET AL.: "Protective Effects of Ranolazine on Ventricular Fibrillation Induced by Activation of the ATP-Dependent Potassium Channel in the Rabbit Heart" J CARDIOVASC PHARMACOL THERAPEUT, vol. 1, no. 2, 1996, pages 141-148, XP009014600
- VISKIN S S: "Long QT syndromes and torsade de pointes" LANCET, XX, XX, vol. 354, no. 9190, 6 November 1999 (1999-11-06), pages 1625-1633, XP004262825 ISSN: 0140-6736
- DATABASE HCAPLUS [Online] 17 June 1991 (1991-06-17), SHIBUYA M, TAKAHASHI Y: "Preparation of piperazine derivatives as antiarrhythmics" XP002250235 Database accession no. 1991-656226 & PATENT ABSTRACTS OF JAPAN vol. 015, no. 357 (C-0866), 10 September 1991 (1991-09-10) & JP 03 141258 A (KOWA), 17 June 1991 (1991-06-17)

## Description

### Field of the Invention

This invention relates to claim 1.

### Background Information

The heart is, in essence, a pump that is responsible for circulating blood throughout the body. In a normally functioning heart such circulation is caused by the generation of electrical impulses that, for example, increase or decrease the heart rate and/or the force of contraction in response to the demands of the circulatory system.

The electrical impulses of the heart can be electrically sensed and displayed (the electrocardiogram, EKG), and the electrical waveform of the EKG is characterized by accepted convention as the "PQRST" complex. The PQRST complex includes the P-wave, which corresponds to the atrial depolarization wave; the QRS complex, corresponding to the ventricular depolarization wave; and the T-wave, which represents the re-polarization of the cardiac cells. Thus, the P wave is associated with activity in the heart's upper chambers, and the QRS complex and the T wave both reflect activity in the lower chambers.

If the electrical signal becomes disturbed in some way, the efficient pumping action of the heart may deteriorate, or even stop altogether. Disturbance in the regular rhythmic beating of the heart is one of the most common disorders seen in heart disease. Irregular rhythms (arrhythmia) can be a minor annoyance, or may indicate a serious problem. For example, arrhythmias may indicate an underlying abnormality of the heart muscle, valves or arteries, and includes the situation where the heart is beating too slowly (bradycardia) and also where the heart is beating too rapidly (tachycardia).

Tachycardias come in two general varieties: supraventricular tachycardias and ventricular tachycardias.

Supraventricular tachycardias include paroxysmal supraventricular tachycardia (PSVT), atrial fibrillation, atrial flutter, AV node reentry, and Wolff-Parkinson White syndrome (WPW). Supraventricular tachycardia (SVT)) is a condition in which electrical impulses traveling through the heart are abnormal because of a cardiac problem somewhere above the lower chambers of the heart. SVT can involve heart rates of 140 to 250 beats per minute (normal is about 70 to 80 beats per minute).

The ventricular tachycardias include ventricular tachycardia itself, as well as ventricular fibrillation and Torsade de Pointes (TdP). Ventricular tachycardia (VT) is a rapid heart rhythm originating within the ventricles. VT tends to disrupt the orderly contraction of the ventricular muscle, so that the ventricle's ability to eject blood is often significantly reduced. That, combined with the excessive heart rate, can reduce the amount of blood actually being pumped by the heart during VT to dangerous levels. Consequently, while patients with VT can sometimes feel relatively well, often they experience - in addition to the ubiquitous palpitations - extreme lightheadedness, loss of consciousness, or even sudden death. As a general rule, VT does not occur in patients without underlying cardiac disease. For people who have underlying cardiac disease, it is generally true that the worse the left ventricular function, the higher the risk of developing life-threatening ventricular tachycardias.

Ventricular tachycardias can arise in myocardial ischemia situations such as unstable angina, chronic angina, variant angina, myocardial infarction, acute coronary syndrome and, additionally in heart failure, both acute and chronic.

There is a condition known as abnormal prolongation of repolarization, or long QT Syndrome (LQTS), which is reflected by a longer than average interval between the Q wave and the T wave as measured by an EKG. Prolongation of the QT interval renders patients vulnerable to a very fast, abnormal heart rhythm (an "arrhythmia") known as Torsade de Pointes. When an arrhythmia occurs, no blood is pumped out from the heart, and the brain quickly becomes deprived of blood, causing sudden loss of consciousness (syncope) and potentially leading to sudden death.

LQTS is caused by dysfunction of the ion channels of the heart or by drugs. These channels control the flow of potassium ions, sodium ions, and calcium ions, the flow of which in and out of the cells generate the electrical activity of the heart. Patients with LQTS usually have no identifiable underlying structural cardiac disease. LQTS may be inherited, with the propensity to develop a particular variety of ventricular tachycardia under certain circumstances, for example exercise, the administration of certain pharmacological agents, or even during sleep. Alternatively, patients may acquire LQTS, for example by exposure to certain prescription medications.

The acquired form of LQTS can be caused by pharmacological agents. For example, the incidence of Torsade de Pointes (TdP) in patients treated with quinidine is estimated to range between 2.0 and 8.8%. DL-sotalol has been associated with an incidence ranging from 1.8 to 4.8%. A similar incidence has been described for newer class III anti-arrhythmia agents, such as dofetilide and ibutilide. In fact, an ever-increasing number of non-cardiovascular agents have also been shown to aggravate and/or precipitate TdP. Over 50 commercially available drugs have been reported to cause TdP. This problem appears to arise more frequently with newer drugs and a number have been withdrawn from the market in recent years (e.g. prenylamine, terodiline, and in some countries terfenadine, astemizole and cisapride). Drug-induced TdP has been shown to develop largely as a consequence of an increase in dispersion of repolarization secondary to augmentation of the intrinsic electrical heterogeneities of the ventricular myocardium.

The majority of pharmacological agents that are capable of producing prolonged repolarization and acquired LQTS can be grouped as acting predominantly through one of four different mechanisms (1) a delay of one or both K currents I_{Ks} and I_{Kr}. Examples are quinidine, N-acetylprocainamide, cesium, sotalol, bretylium, clofilium and other new Class III antiarrhythmic agents (this action could possibly be specifically antagonized by drugs that activate the K channel, such as pinacidil and cromakalin); (2) suppression of Iₜₒ, as in the case of 4-aminopyridine, which was shown to prolong repolarization and induce EADs preferentially in canine subepicardial M cells, which are reported to have prominent Iₜₒ; (3) an increase in I_{Ca}, as in the case of Bay K 8644 (this action could be reversed by Ca channel blockers); (4) a delay of I_{Na} inactivation, as in the case of aconitine, veratridine, batrachotoxin, DPI, and the sea anemone toxins (ATX) anthopleurin-A (AP-A) and ATX-II (this action could be antagonized by drugs that block I_{Na}, and/or slowly inactivate Na current, such as lidocaine and mexiletine). Because these drugs (e.g., lidocaine and mexiletine) can shorten prolonged repolarization, they can also suppress EADs induced by the first two mechanisms.

The list of drugs causing LQTS and TdP is continually increasing. Literally, any pharmacological agent that can prolongate QT can induce LQTS. The incidence of TdP has not been correlated with the plasma concentrations of drugs known to precipitate this arrhythmia. However, high plasma concentrations, resulting from excessive dose or reduced metabolism of some of these drugs, may increase the risk of precipitating TdP. Such reduced metabolism may result from the concomitant use of other drugs that interfere with cytochrome P₄₅₀ enzymes. Medications reported to interfere with the metabolism of some drugs associated with TdP include systemic ketoconazole and structurally similar drugs (fluconazole, itraconazole, metronidazole); serotonin re-uptake inhibitors (fluoxetine, fluvoxamine, sertraline), and other antidepressants (nefazodone), human immunodeficiency virus (HIV) protease inhibitors (indinavir, ritonavir, saquinavir); dihydropyridine calcium channel blockers (felodipine, nicardipine, nifedipine) and erythromycin, and other macrolide antibiotics. Grapefruit and grapefruit juice may also interact with some drugs by interfering with cytochrome P₄₅₀ enzymes. Some of the drugs have been associated with TdP, not so much because they prolong the QT interval, but because they are inhibitors primarily of P4503A4, and thereby increase plasma concentration of other QT prolonging agents. The best example is ketoconazole and itraconazole, which are potent inhibitors of the enzyme and thereby account for TdP during terfenadine, astemizole, or cisapride therapy. On the other hand, the incidence of drug associated TdP has been very low with some drugs: diphyhydramine, fluconazole, quinine, lithium, indapamide, and vasopressin. It should also be noted that TdP may result from the use of drugs causing QT prolongation in patients with medical conditions, such as hepatic dysfunction or congenital LQTS, or in those with electrolyte disturbances (particularly hypokalemia and hypomagnesemia).

However, there are anti-arrhythmic drugs that are known to prolong the QT interval but do not induce TdP. It has been discovered that a property common to such drugs is the ability to concurrently inhibit other ion currents such as I_{Na} channels, and/or the I_{Ca} channel.

The inherited form of LQTS occurs when a mutation develops in one of several genes that produce or "encode" one of the ion channels that control electrical repolarization. There are at least five different forms of inherited LQTS, characterized as LQT1, LQT2, LQT3, LQT4, and LQT5. They were originally characterized by the differing shape of the EKG trace, and have subsequently been associated with specific gene mutations. The LQT1 form, from KCNQ1 (KVLQT1) or KCNE1 (MinK) gene mutations, is the most frequent, accounting for approximately 55-60% of the genotyped patients. LQT2, from HERG or KCNE2 (MiRP1) mutations, is next at about 35-40%, and LQT3, from SCN5A mutations accounts for about 3-5%. Patients with two mutations seem to account for less than 1% of all patients, but this may change as more patients are studied with the newer genetic techniques.

The mutant gene causes abnormal channels to be formed, and as these channels do not function properly, the electrical recovery of the heart takes longer, which manifests itself as a prolonged QT interval. For example, an inherited deletion of amino-acid residues 1505-1507 (KPQ) in the cardiac Na+ channel, encoded by SCN5A, causes the severe autosomal dominant LQT3 syndrome, associated with fatal ventricular arrhythmias. Fatal arrhythmias occur in 39% of LQT3 patients during sleep or rest, presumably because excess late Na+ current abnormally prolongs repolarization, particularly at low heart rates, and thereby favors development of early afterdepolarizations (EADs) and ectopic beats. Preferential slowing of repolarization in the midmyocardium might further enhance transmural dispersion of repolarization and cause unidirectional block and reentrant arrhythmias. In another 32% of LQT3 patients, fatal cardiac events are triggered by exercise or emotion.

It was recently reported that a variant of the cardiac sodium channel gene SCN5A was associated with arrhythmia in African-Americans. Single-strand conformation polymorphism (SCCP) and DNA sequence analyses revealed a heterozygous transversion of C to A in codon 1102 of SCN5A causing a substitution of serine (S1102) with tyrosine (Y1102). S1102 is a conserved residue located in the intracellular sequences that link domains II and III of the channel. These researchers found that the Y1102 allele increased arrhythmia susceptibility. The QT_{c} (corrected QT) was found to be markedly prolonged with amiodarone, leading to Torsade de Pointes ventricular tachycardia.

There is a need for an agent to treat or prevent inherited or acquired LQTS in a manner that reduces the risk of arrhythmia and TdP. Ranolazine has previously been demonstrated to be an effective agent for the treatment of angina causing no or minimal effects on heart rate or blood pressure. Now, surprisingly, we have discovered that ranolazine and related compounds are effective agents for the prophylaxis and/or treatment of inherited or acquired arrhythmia.

Surprisingly, we have discovered that compounds that inhibit I_{Kr}, I_{Ks}, and late I_{Na} ion channels exhibit this preferred spectrum of activity. Such compounds prolong the ventricular action potential duration, increase the ventricular effective refractory period, decrease TDR, increase APD, and do not produce EADs. For example, ranolazine, which is known to be useful in the treatment of angina and congestive heart failure, has been found to be useful in the treatment of ventricular tachycardia by virtue of its ability to inhibit I_{Kr}, I_{Ks}, and late I_{Na} ion channels at dose levels that do not block calcium channels. This is particularly surprising, in that U.S. Patent No. 4,567,264discloses that ranolazine is a cardioselective drug that inhibits calcium ion channels, and suggests that as a consequence of its effect to block calcium channels it might be useful in the treatment of a multitude of disease states including arrhythmia. However, we have discovered that ranolazine acts as an effective anti-arrhythmic agent at levels that have little or no effect on the calcium channel. The lack of or minimal effect on calcium channel activity at therapeutic dose levels is beneficial in that it obviates the well-known effects of calcium ion channel inhibitors (e.g., changes in blood pressure) that are undesirable when treating arrhythmia in a patient. We have also discovered that ranolazine is effective in suppressing EADs and triggered activity that are a side effect of administration of drugs such as quinidine and sotalol.

Accordingly, a novel and effective method of treating VT is provided that restores sinus rhythm while being virtually free of undesirable side effects, such as changes in mean arterial pressure, blood pressure, heart rate, or other adverse effects.

### SUMMARY OF THE INVENTION

It is an object of this invention to provide an effective method of suppressing early after depolarization (EADS) in a mammal. Accordingly, in a first aspect, the invention relates to a compound of the Formula I: 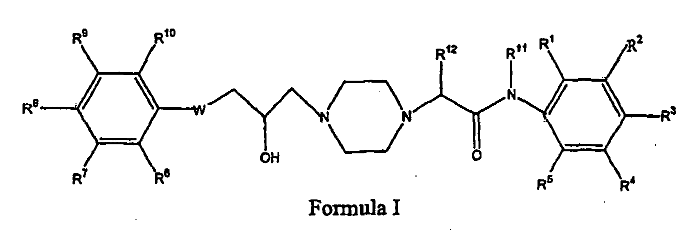 wherein:
R¹, R², R³, R⁴ and R⁵ are each independently hydrogen, lower alkyl, lower alkoxy, cyano, trifluoromethyl, halo, lower alkylthio, lower alkyl sulfinyl, lower alkyl sulfonyl, or N-optionally substituted alkylamido, provided that when R¹ is methyl, R⁴ is not methyl;
or R² and R³ together form -OCH₂O-;
R⁶, R⁷, R⁸, R⁹ and R¹⁰ are each independently hydrogen, lower acyl, aminocarbonylmethyl, cyano, lower alkyl, lower alkoxy, trifluoromethyl, halo, lower alkylthio, lower alkyl sulfinyl, lower alkyl sulfonyl, or di-lower alkyl amino; or
R⁶ and R⁷ together form -CH=CH-CH=CH-; or
R⁷ and R⁸ together form -O-CH₂O--;
R¹¹ and R¹² are each independently hydrogen or lower alkyl; and
W is oxygen or sulfur;
or an isomer thereof, or a pharmaceutically acceptable salt or ester of the compound of Formula I or its isomer, for use in suppressing early after depolarization in a mammal.

A preferred compound is ranolazine, which is named N-(2,6-dimethylphenyl)-4-[2-hydroxy-3-(2-methoxyphenoxy)propyl]-1-piperazineacetamide {also known as 1-[3-(2-methoxyphenoxy)-2-hydroxypropyl]-4-[(2,6-dimethylphenyl)-anunocarbonylmethyl]-piperazine}, as a racemic mixture, or an isomer thereof, or a pharmaceutically acceptable salt thereof. It is preferably administered at dose levels that inhibit Iₖᵣ, Iₖₛ. and late I_{Na} ion channels but does not inhibit calcium channels or other ion channels. Ranolazine, as a racemic mixture or an isomer, may be formulated either as the free base or as a pharmaceutically acceptable salt. If formulated as a pharmaceutically acceptable salt, the dihydrochloride salt is preferred.

In a second aspect, the invention relates to a method of suppressing early after depolarization in a mammal, comprising administering an effective amount of ranolazine, or an isomer thereof, or a pharmaceutically acceptable salt of the compound or its isomer, to a mammal in need thereof.

In a third aspect, the invention relates to a method of suppressing early after depolarization in a mammal comprising administration of ranolazine, or an isomer thereof, or a pharmaceutically acceptable salt of the compound or its isomer, at a dose level that inhibits late I_{Na} ion channels. Preferred is a therapeutic amount that inhibits I_{Kr}, I_{Ks}, and late I_{Na} ion channels More preferred is a therapeutic amount that inhibits I_{Kr}, I_{Ks}, and late I_{Na} ion channels but does not inhibit calcium channels.

In one preferred embodiment, the compounds of the invention are administered in a manner that provides plasma level of the compound of Formula I of at least 350±30 ng/mL for at least 12 hours.

In a second preferred embodiment, the compounds of the invention are administered as a sustained release formulation that maintains plasma concentrations of the compound of Formula I at less than a maximum of 4000 ng/mL, preferably between about 350 to about 4000 ng base/mL, for at least 12 hours.

In a third preferred embodiment, the compounds of the invention are administered in a formulation that contains between about 10 mg and 700 mg of a compound of Formula I. A preferred compound of Formula I is ranolazine, or an isomer thereof, or a pharmaceutically acceptable salt of the compound or an isomer thereof.

In a fourth preferred embodiment, the compounds of the invention are administered in a formulation that provides a dose level of about 1 to about 30 micromoles per liter of the formulation. Preferred is the administration of a formulation that provides a dose level of about 1 to about 10 micromoles per liter of the formulation.

In a fourth aspect, this invention relates to a method of suppressing early after depolarisation in a mammal comprising administration of a therapeutic amount of a compound of Formula I at dose levels that inhibit I_{Kr}, I_{Ks}, and late I_{Na} ion channels but does not inhibit calcium channels.

In a fifth aspect, this invention relates to a method of suppressing early after depolarization in a mammal by administration of a compound of Formula I as a bolus in a manner that provides a plasma level of the compound of Formula I of at least 350±30 ng/mL for at least 12 hours.

In a sixth aspect, this invention relates to a method of suppressing early after depolarization in a mammal by administration of a compound of Formula I as a sustained release formulation in a manner that maintains a plasma level of the compound of Formula I of at a less than a maximum of 4000 ng/ml, preferably between about 350 to about 4000 ng base/mL for at least 12 hours.

In a seventh aspect, this invention relates to methods of suppressing early after depolarization in a mammal wherein a compound of Formula I or an isomer thereof, or a pharmaceutically acceptable salt or ester of the compound or its isomer is administered by bolus or sustained release composition.

In a eighth aspect, this invention relates to methods of suppressing early after depolarization in a mammal wherein a compound of Formula I or an isomer thereof, or a pharmaceutically acceptable salt or ester of the compound or its isomer is administered intravenously.

In a nineth aspect, this invention relates to use of a compound of Formula I or an isomer thereof, or a pharmaceutically acceptable salt or ester of the compound or its isomer for the manufacture of a pharmaceutical composition for suppressing early after depolarization in a mammal.

### ABBREVIATIONS:

- APD:: Action potential duration
- BCL:: basic cycle length
- EAD:: Early after depolarizations.
- ECG and EKG:: Electrocardiogram
- I_{Kr}:: rapid potassium channel rectifying current
- I_{Ks}:: slow potassium channel rectifying current
- I_{Na,L}: late sodium channel current
- epi cells:: Epicardial Cells
- endo cells:: Endocardial Cells
- LQTS:: long term QT syndrome
- M cells:: cells derived from the midmyocardial region of the heart
- RMP:: resting membrane potential
- TdP:: Torsade de Pointes
- TDR: transmural dispersion of repolarization
- VT:: ventricular tachycardia

### FIGURE LEGENDS

Figure 1. The relationship between a hypothetical action potential from the conducting system and the time course of the currents that generate it.
Figure 2. Normal impulse propagation.

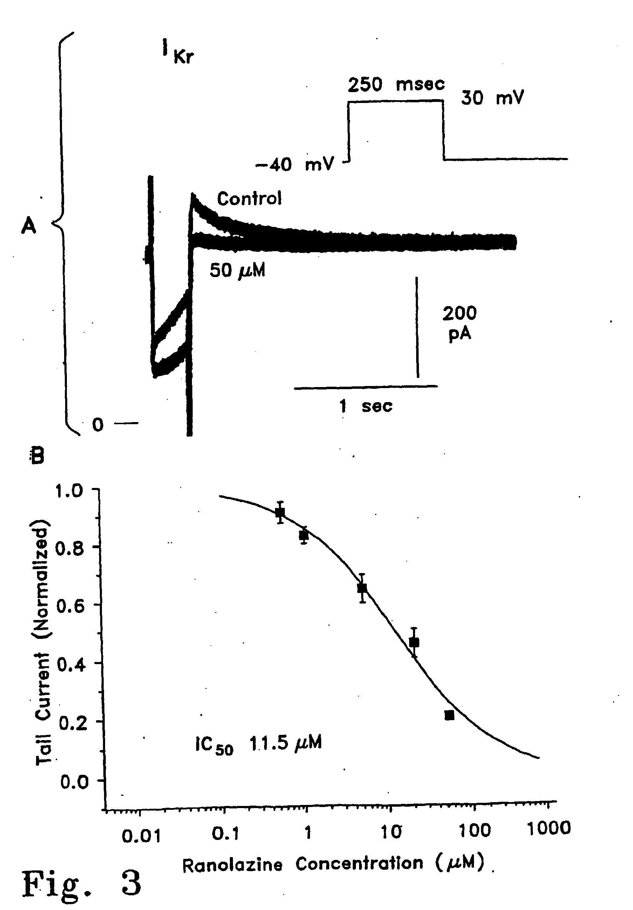

Figure 3. Effect of ranolazine on the rapidly activating component of the delayed rectifier current (I_{Kr}) in canine left ventricular myocytes. A: representative current traces recorded during 250 msec pulses to 30 mV from a holding potential of - 40 mV and repolarization back to -40 mV before and after ranolazine (50)µM). Cells were bathed in Tyrode's solution containing 5 µM nifedipine. B: Concentration-response curves for the inhibitory effects of ranolazine on I_{Kr}. I_{Kr} was measured as the tail current on repolarization to -40 mV after a 250 msec depolarizing pulse to 30 mV (n = 5-8).

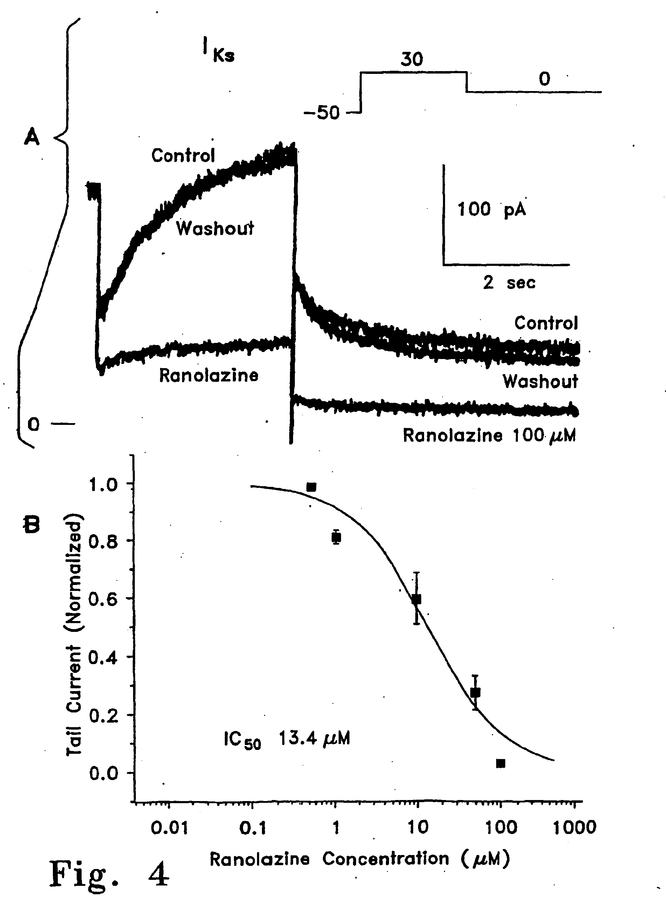

Figure 4. Ranolazine inhibits the slowly activating component of the delayed rectifier current (I_{Ks}). A: Representative I_{Ks} current traces recorded from a typical experiment in canine left ventricular epicardial myocytes in the presence and absence of 100 µM ranolazine. Currents were elicited by a depolarization step to 30 mV for 3 sec from a holding potential of -50 mV followed by a repolarization step to 0 mV (4.5 sec). I_{Ks} was measured as the tail current recorded following the repolarization step. Ranolazine (100 µM), almost completely blocked I_{Ks} and the inhibitory effect was completely reversed on washout. B: Concentration-response curve for the inhibitory effect of ranolazine on I_{Ks} (measured as the tail current elicited by the repolarization step to 0 mV after a 3 sec depolarizing step to 30 mV) (n = 5-14) in the presence of 5 µM, E-4031 and 5 µM, nifedipine. Values represent mean ± SEM of normalized tail current. Ranolazine inhibited I_{Ks} with an IC₅₀ of 13.4 µM.
Figure 5. Ranolazine does not affect I_{K1} in canine ventricular myocytes. A: Shown are representative current traces recorded before and after exposure to ranolazine (100 µM) during voltage steps from a holding potential of - 40 mV to 900 msec test potentials ranging between - 100 and 0 mV. B: Steady state I-V relations constructed by plotting the current level measured at the end of the 900 msec pulse as a function of the test voltages. Ranolazine up to a concentration of 100 µM, did not alter I_{K1}. Data are presented as mean ± S.E.M. (n = 6).
Figure 6. Effects of ranolazine on epicardial and M cell action potentials at a basic cycle length (BCL) of 2000 msec ([K⁺]₀ = 4 mM). A: Shown are superimposed transmembrane action potentials recorded under baseline conditions and following addition of progressively higher concentrations of ranolazine (1-100 µM). B and C: Graphs plot the concentration-dependent effect of ranolazine on action potential duration (APD₅₀ and APD₉₀). Data presented are mean±SD. * - p<0.05 vs. control.
Figure 7. Effect of ranolazine on epicardial and M cell action potential duration (APD₅₀ and APD₉₀) at a basic cycle length of 500 msec ([K⁺]₀ = 4 mM). Graphs plot the concentration-dependent effect of ranolazine on action potential duration (APD₅₀ and APD₉₀). Data presented are mean±SD. *- p<0.05 vs. control.
Figure 8. Effect of ranolazine on the rate of rise of the upstroke of the action potential (Vₘₐₓ). Shown are superimposed action potentials (B) and corresponding differentiated upstrokes (dV/dt, A) recorded under baseline conditions and in the presence of 10 and 100 µM ranolazine (BCL=500 msec). C: Concentration-response relationship of ranolazine's effect to reduce Vmax.
Figure 9. Effects of ranolazine on epicardial and M cell action potentials recorded at a basic cycle length of 2000 msec and [K⁺]ₒ = 2 mM. A: Shown are superimposed transmembrane action potentials recorded in the absence and presence of ranolazine (1-100 µM). B and C: Graphs plot the concentration-dependent effect of ranolazine on action potential duration (APD₅₀ and APD₉₀). Data presented as mean±SD. * - p<0.05 vs. control.
Figure 10. Effects of ranolazine on epicardial and M cell action potential duration (APD₅₀ and APD₉₀) at a basic cycle length of 500 msec ([K+]₀ = 2 mM). Graphs plot the concentration-dependent effect of ranolazine on action potential duration (APD₅₀ and APD₉₀). Data presented as mean±SD. * - p<0.05 vs. control.
Figure 11. Each panel shows, from top to bottom, an ECG trace and transmembrane action potentials recorded from the midmyocardium (M region) and epicardium (Epi) of the arterially perfused canine left ventricular wedge preparation at a basic cycle length (BCL) of 2000 msec. The superimposed signals depict baseline conditions (Control) and the effect of ranolazine over a concentration range of 1-100 µM. A: Performed using Tyrode's solution containing 4 mM KCl to perfuse the wedge. B: Performed using Tyrode's solution containing 2 mM KCl.
Figure 12. Composite data graphically illustrating APD₉₀ (of Epi and M) and QT interval values (A, C) and of APD₅₀ values (B, D) before and after exposure to ranolazine (1-100 µM). A, B: 4 mM KCl. C, D: 2 mM KCl. BCL= 2000 msec.
Figure 13. Effect of ranolazine to suppress d-sotalol-induced early afterdepolarizations (EAD) in M cell preparations. A and B: Superimposed transmembrane action potentials recorded from two M cell preparations under control conditions, in the presence of I_{Kr} block (100 µM d-sotalol), and following the addition of stepwise increased concentrations of ranolazine (5, 10, and 20 µM) in the continued presence of d-sotalol. Basic cycle length = 2000 msec.
Figure 14. Block of late I_{Na} by ranolazine recorded using perforated patch voltage clamp technique. A: TTX-sensitive currents are shown in control solution (black trace) and after 20 µM ranolazine (red trace). B: Summary plot of the concentration-response curve for 2 - 8 cells.
Figure 15. Effects of ranolazine on Iₜₒ. Currents were recorded during 100 ms steps to -10 (small outward current), 0, and 10 mV. Itₒ recorded in control solution (left, black traces), and 4 min after addition of 50 uM ranolazine (right, red traces).
Figure 16. Summarized data for the effects of ranolazine on Itₒ at 3 test potentials for concentrations of 10 µM (9 cells), 20 µM (9 cells), 50 uM (6 cells), and 100 µM (7 cells).
Figure 17. Normalized Iₜₒ and the effects of ranolazine. These data are the same as those presented in figure 4.
Figure 18. Top panel shows superimposed traces of I_{Na}-Ca in control solution, 4 min after addition of 100 µM ranolazine, and after returning to control solution (red trace). The lower panel of figure shows the concentration-response curve.
Figure 19. Concentration-response curves for I_{Kr}, I_{Ks}, I_{Ca}, I_{Na}, late, and I_{NaCa} in a single plot. I_{Kr}, I_{Ks}, and late I_{Na} showed similar sensitivities to ranolazine, whereas I_{NaCa} and I_{Ca} were considerably less sensitive.
Figure 20. Effects of ranolazine on Purkinje fiber action potential. A and B: Graphs plot concentration-dependent effects of ranolazine (1-100 µM) on action potential duration (APD₅₀ and APD₉₀) at a BCL of 500 (A) and 2000 (B) msec. C and D: Superimposed transmembrane action potentials recorded under baseline conditions and after the addition of progressively higher concentrations ofranolazine at a BCL of 500 (C) and 2000 (D) msec. ([K+]₀ = 4 mM). Data are presented as mean ± SD. * - p<0.05 vs. control.
Figure 21. Concentration-dependent effects of ranolazine on the rate of rise of the upstroke of the action potential (Vₘₐₓ). Shown are superimposed action potentials (B) and corresponding differentiated upstrokes (dV/dt, A) recorded in the absence and presence of ranolazine (1-100 µM) (BCL=500 msec). C: Concentration-response relationship of ranolazine's effect to reduce Vₘₐₓ.
Figure 22. Effects of ranolazine on Purkinje fiber action potential in the presence of low [K⁺]ₒ. A and B: Graphs plot concentration-dependent effects of ranolazine (1-100 µM) on action potential duration (APD₅₀ and APD₉₀) at a BCL of 500 (A) and 2000 (B) msec. ([K⁺]₀ = 3 mM). Data are presented as mean ± SD. * - p<0.05 vs. control.
Figure 23. Effect of ranolazine to suppress d-sotalol-induced early afterdepolarization (EAD) in a Purkinje fiber preparation. Shown are superimposed transmembrane action potentials recorded from a Purkinje fiber preparation in the presence of I_{Kr} block (100 µM d-sotalol), and following addition of stepwise increased concentration of ranolazine (5 and 10 µM) in the continued presence of d-sotalol. Basic cycle length = 8000 msec.
Figures 24A and B. Overall electrophysiological data for sotalol. Shown are the effects of sotalol on right and left ventricular ERP in ms.
Figures 25 A and B. Overall electrophysiological data for sotalol. Shown are the effects of sotalol on QT and QRS intervals in ms.
Figure 26. Overall electrophysiological data for ranolazine. Shown are the effects of ranolazine on right and left ventricular ERP in ms.
Figure 27. Overall electrophysiological data for ranolazine. Shown are the effects of ranolazine on mean ERP-LV.
Figure 28. Overall electrophysiological data for ranolazine. Shown are the effects of ranolazine on QT interval in ms.
Figure 29. Overall electrophysiological data for ranolazine. Shown are the effects of ranolazine on QRS interval.
Figure 30. Block of late I_{Na} by ranolazine recorded using action potential voltage clamp technique. A: TTX-sensitive currents are shown in control solution and after 20 µM ranolazine. Measurements were made at the two cursors, corresponding to voltages of 20 mV and -28 mV. Inhibition was greatest at 20 mV, but some TTX-sensitive current remains at -28 mV in the presence of ranolazine. TTX-sensitive current also remains early in the action potential in the presence of ranolazine.
Figure 31. Block of I_{Na,late} by ranolazine. 2000 ms BCL. Summary plot of the concentration-response curve. Error bars are ±s.e.m., number of cells 3-11 cells.
Figure 32. Block of I_{Na,late} by ranolazine. 300 ms BCL. Summary plot of the concentration-response curve. Error bars are ±s.e.m., number of cells 6-10 cells.
Figure 33. Summarized data for the effects of ranolazine on I_{Na,late} at slow and rapid rates of stimulation. Error bars are i-s.e.m., number of cells 6-12 cells.
Figure 34. The effect of ranolazine at 3, 10, and 30 µmol/L on action potential duration of myocytes.
Figure 35. The effects of ranolazine at 30 µmol/L on a myocyte paced first at 2 Hz and then at 0.5 Hz.
Figure 36. The comparisons of APD₅₀ and APD₉₀ measured in the absence and presence of 3, 10, and 30 µmol/L ranolazine at pacing frequencies of 0.5, 1 and 2 Hz.
Figure 37. Effects of ranolazine, shortening the APD₅₀ and APD₉₀ at various pacing frequencies. Normalized as percentage of control.
Figure 3 8. Effect of quinidine at 5 µmol/L on duration of action potential of a myocyte paced at 0.25 Hz. Ranolazine at 10 µmol/L attenuated the effect of quinidine.

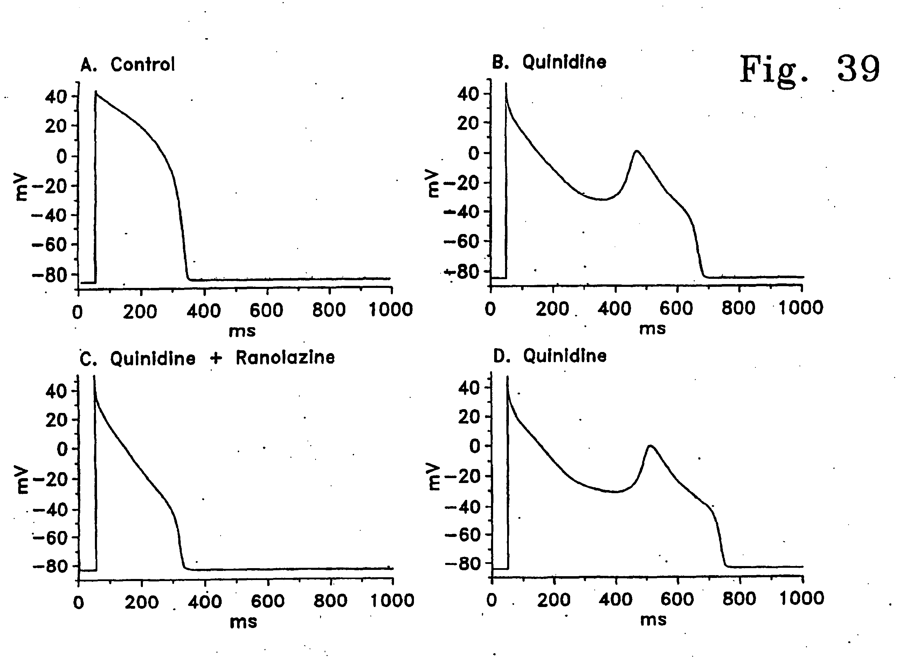

Figure 39. Effects of quinidine and/or ranolazine on EADs. Ranolazine at 10 µmol/L was found to be effective in suppressing EADs induced by quinidine.

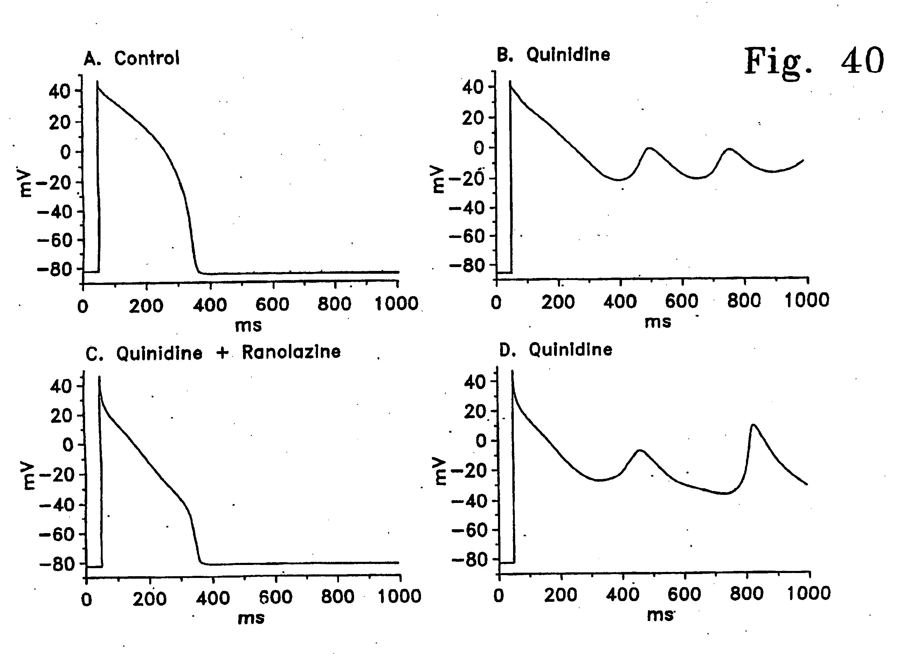

Figure 40. Effects of quinidine and/or ranolazine on triggered activity. Ranolazine at 10 µmol/L was found to be effective in suppressing triggered activity induced by quinidine.

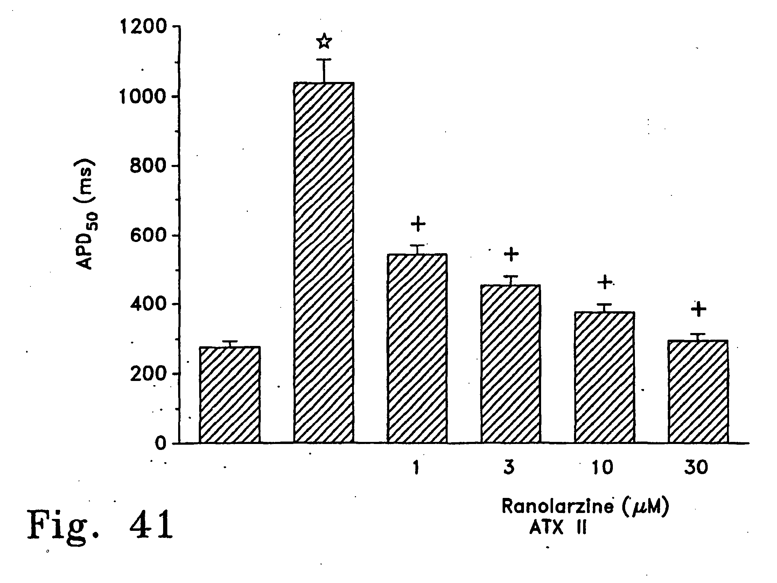

Figure 41. Effects of ATXII and/or ranolazine at 1, 3, 10, and 30 µmol/L on action potential duration in guinea pig ventricular myocytes.

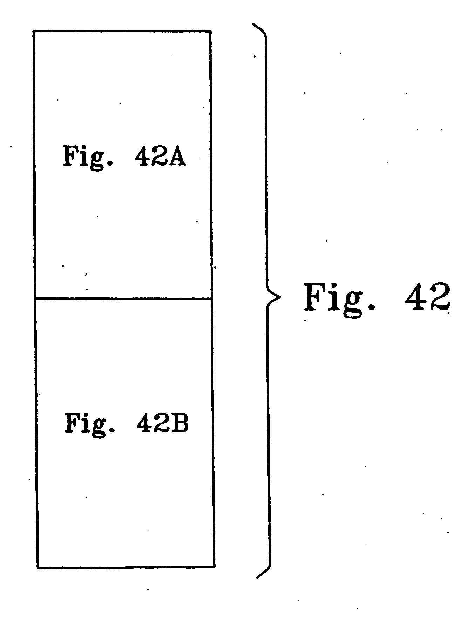

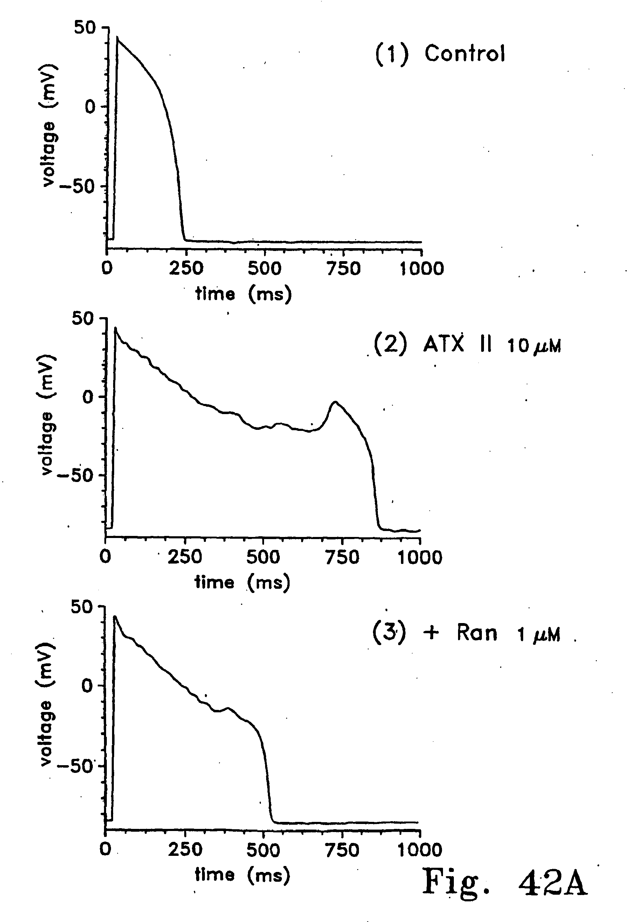

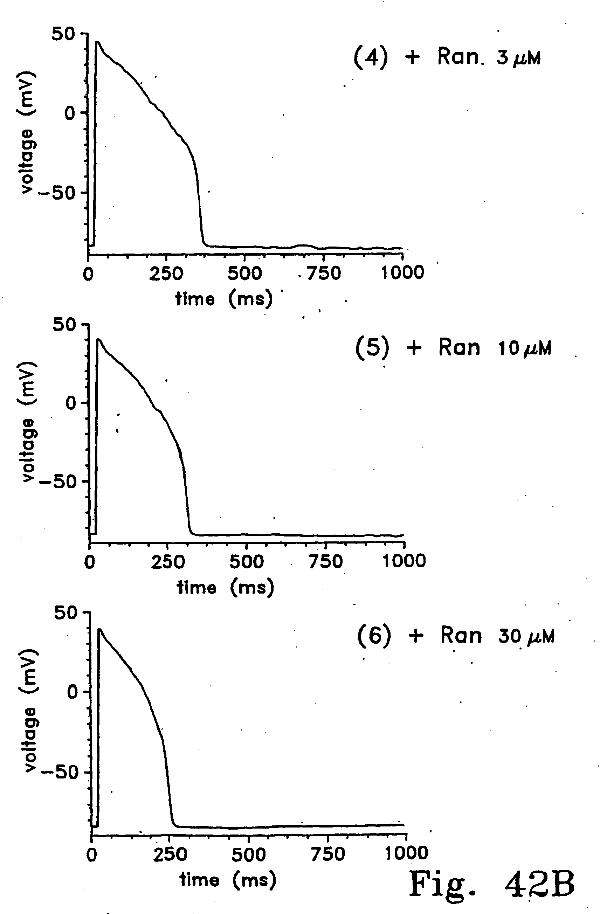

Figure 42. Effects of ATXII and/or ranolazine at 1, 3, 10, and 30 µmol/L on action potential duration in guinea pig ventricular myocytes. Ranolazine at a concentration as low as 1 µmol/L effectively abolished ATXU-induced EADs and triggered activity.

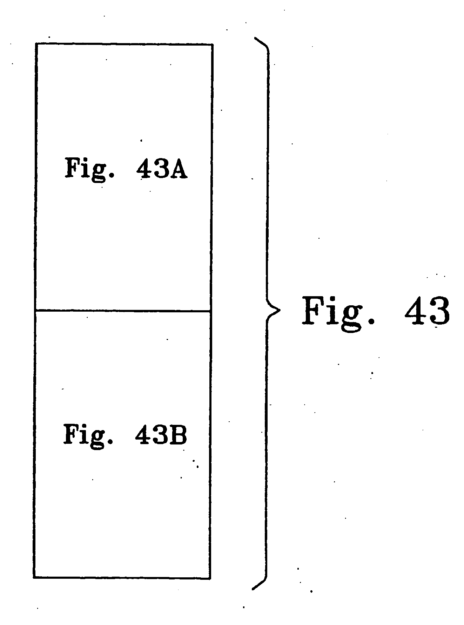

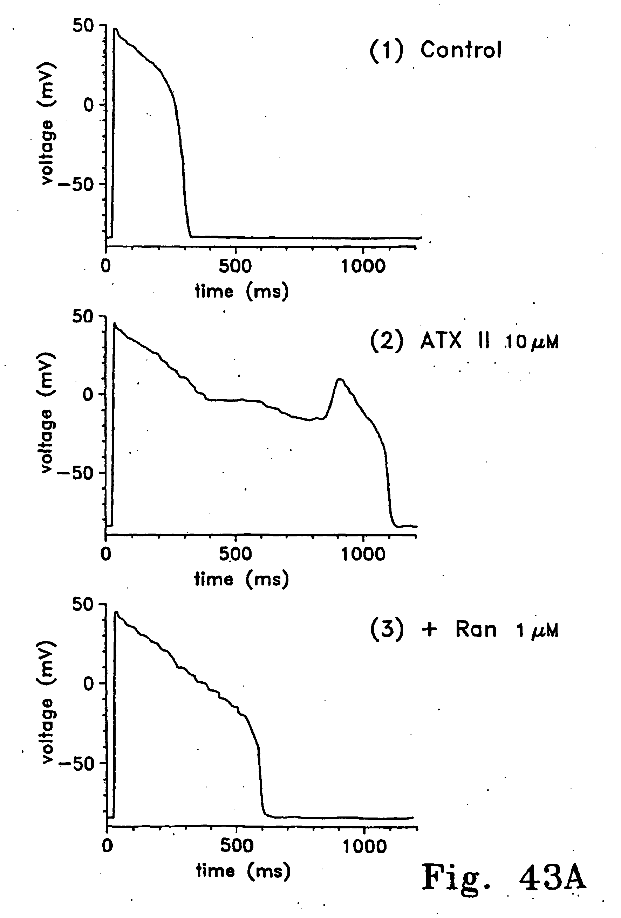

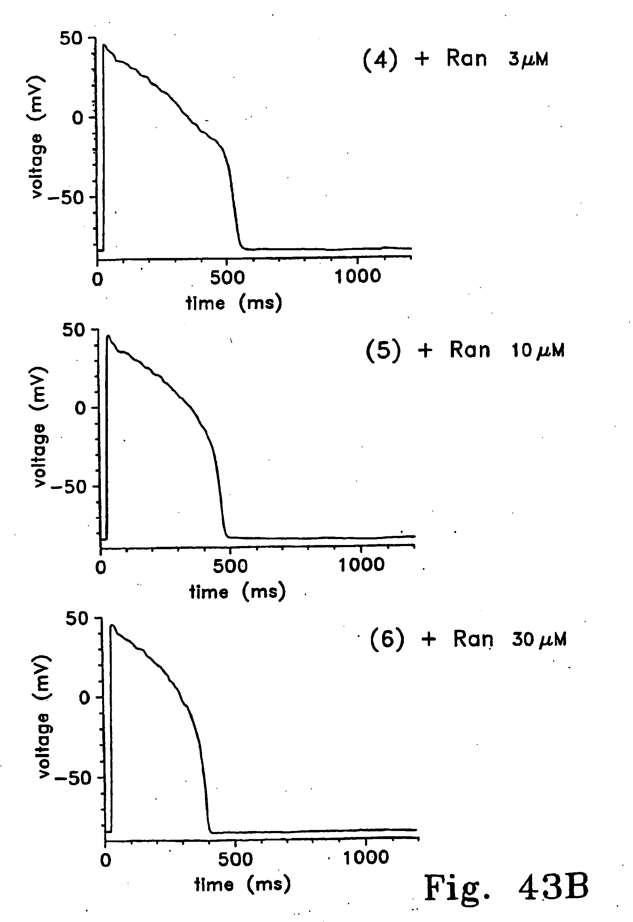

Figure 43. Effects of ATXII and/or ranolazine at 1, 3, 10, and 30 µmol/L on action potential duration in guinea pig ventricular myocytes. Ranolazine at a concentration as low as 1 µmol/L effectively abolished ATXII-induced EADs and triggered activity.

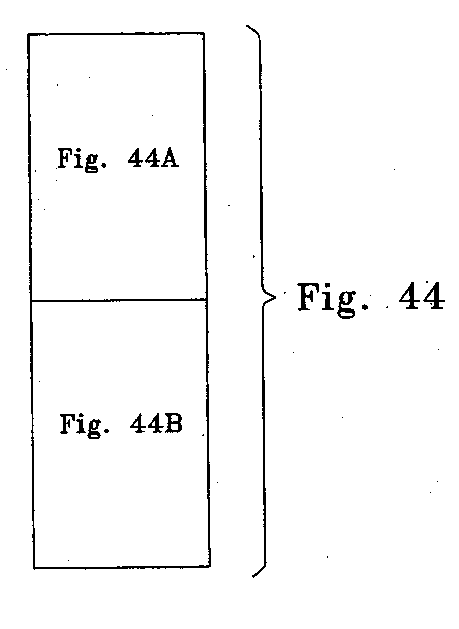

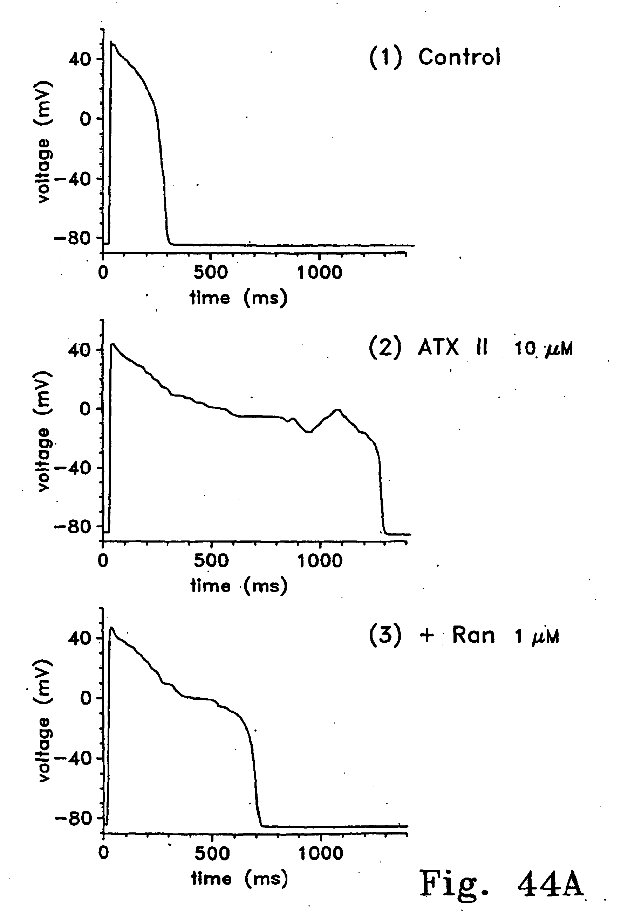

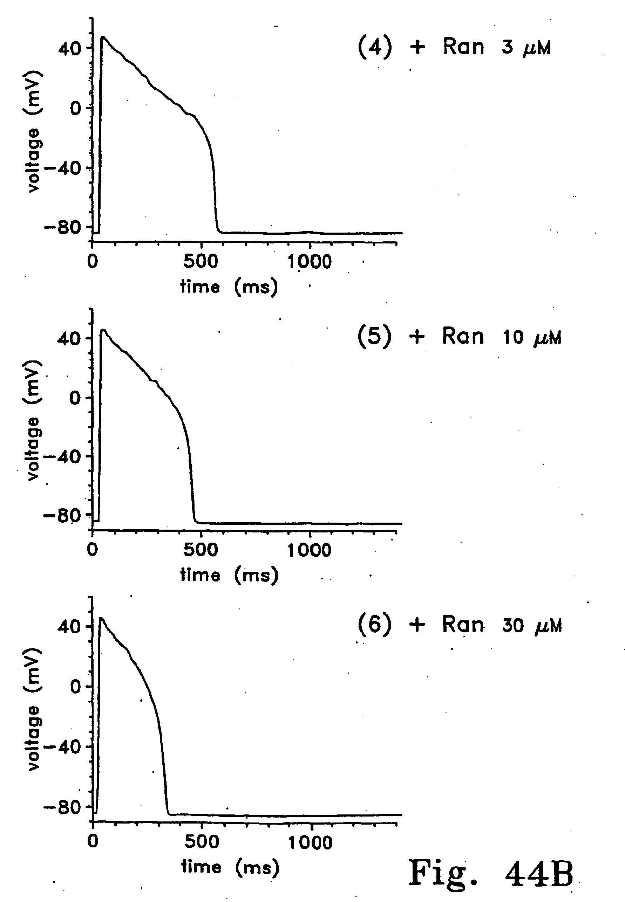

Figure 44. Effects of ATXH and/or ranolazine at 1, 3, 10, and 30 µmol/L on action potential duration in guinea pig ventricular myocytes. Ranolazine at a concentration as low as 1 µmol/L effectively abolished ATXII-induced EADs and triggered activity.

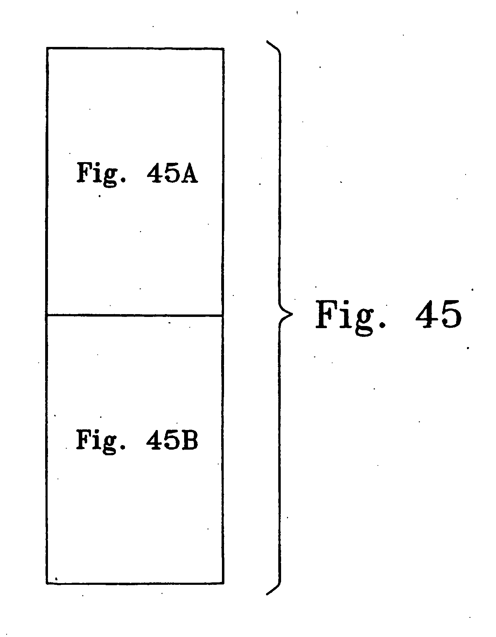

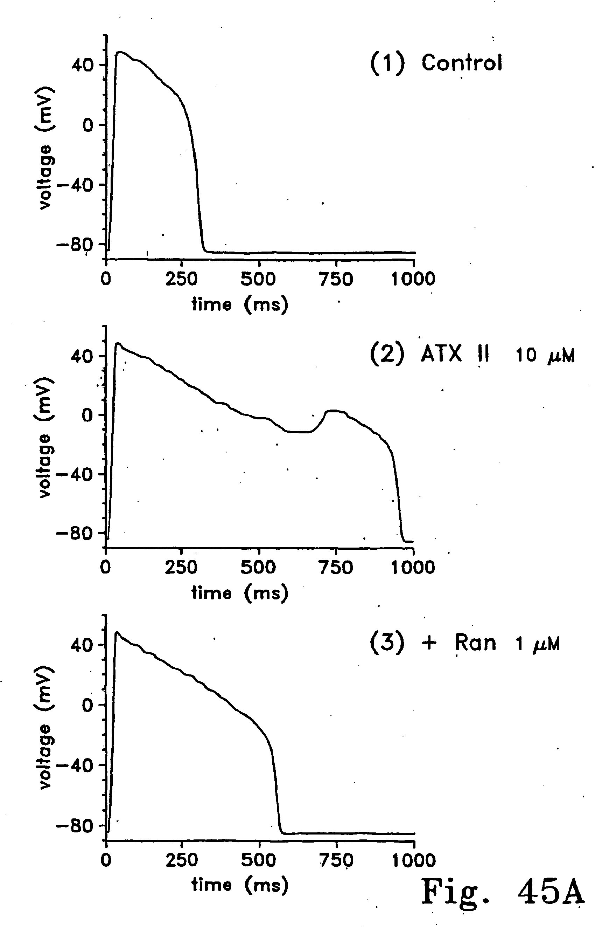

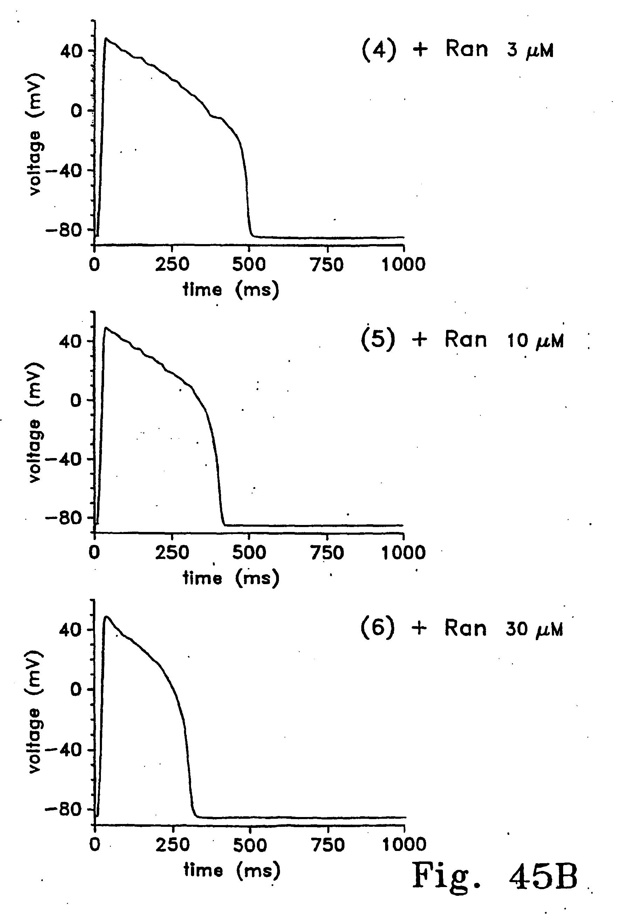

Figure 45. Effects of ATXII and/or ranolazine at 1, 3, 10, and 30 µmol/L on action potential duration in guinea pig ventricular myocytes. Ranolazine at a concentration as low as 1 µmol/L effectively abolished ATXII-induced EADs and triggered activity.

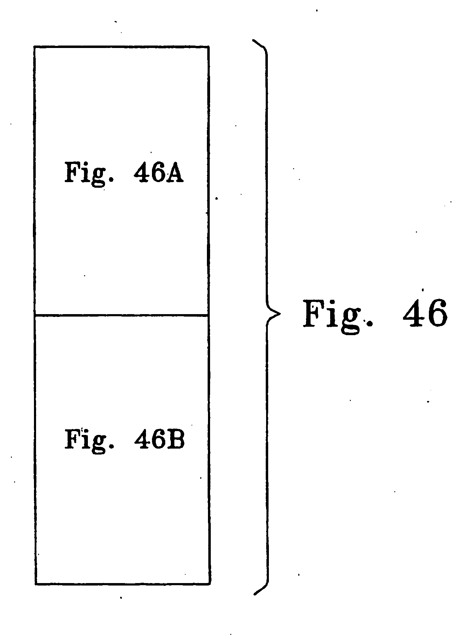

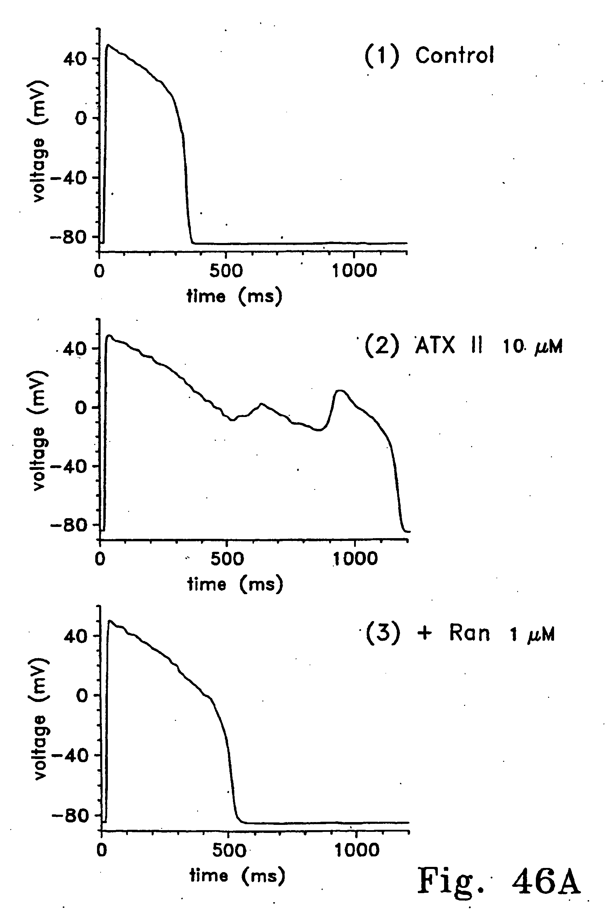

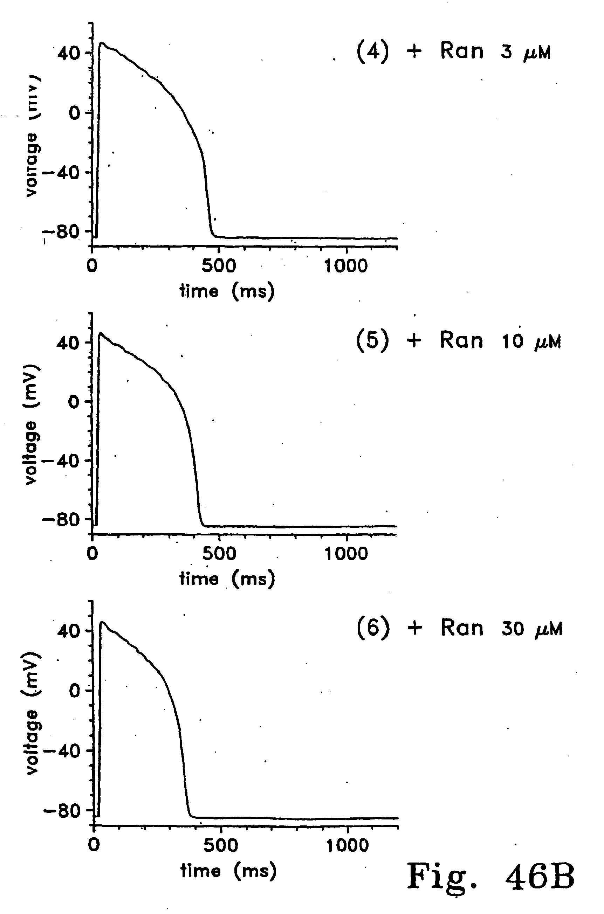

Figure 46. Effects of ATXII and/or ranolazine at 1, 3, 10, and 30 µmol/L on action potential duration in guinea pig ventricular myocytes. Ranolazine at a concentration as low as 1 µmol/L effectively abolished ATXII-induced EADs and triggered activity.

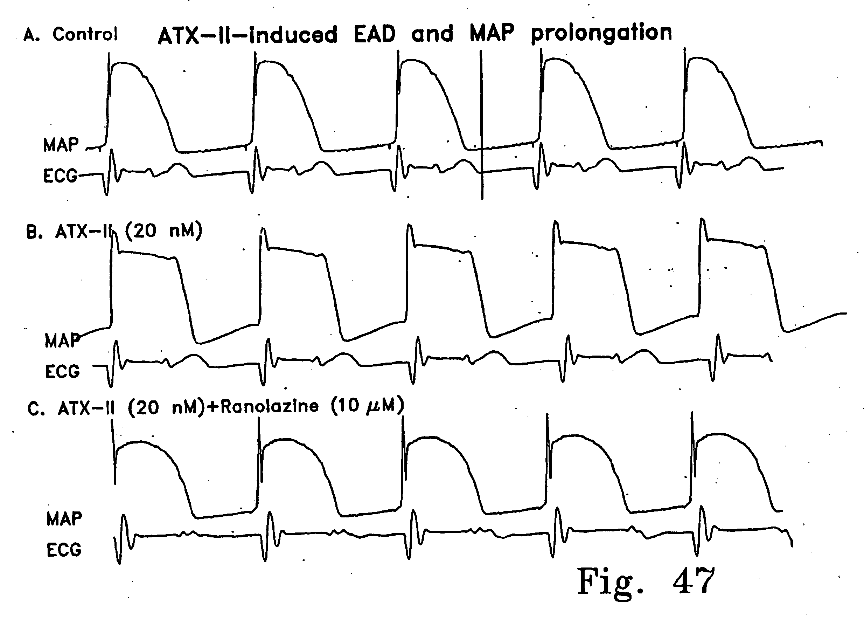

Figure 47. Effects of ATXII and ranolazine at 10 µM on induced EAD and MAP prolongation in the K-H buffer perfused guinea pig isolated heart model. Ranolazine at a concentration as low as 10 µM reduced or effectively abolished ATXII-induced EADs and MAP prolongation.

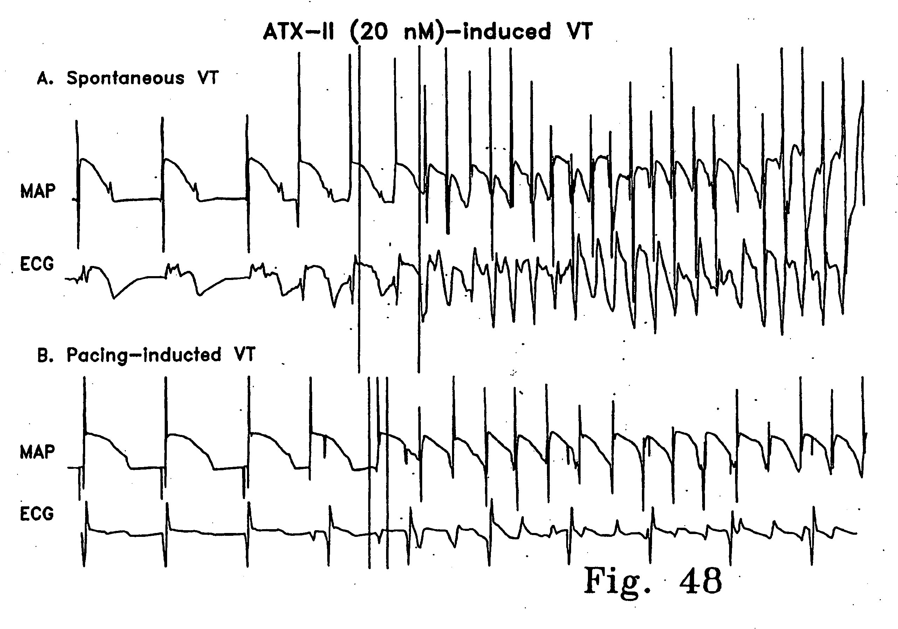

Figure 48. Effects of ATXII on VT. ATXII (20 nM) induced VT, both spontaneous VT and pacing-induced VT.

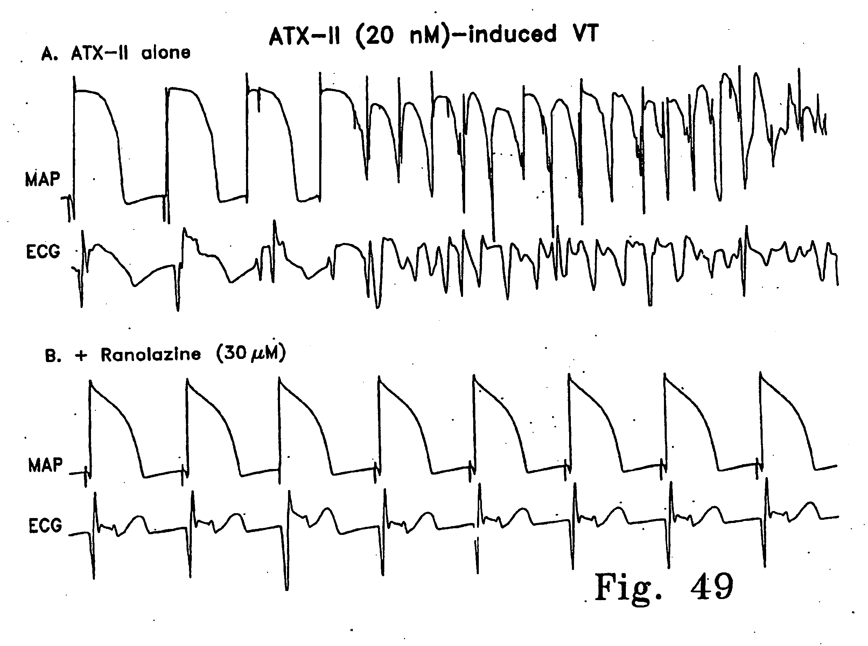

Figure 49. Effects of ATXII (20 nM) and ranolazine on induced VT. Ranolazine at a concentration of 30 µM reduced or effectively abolished ATXII-induced VT.

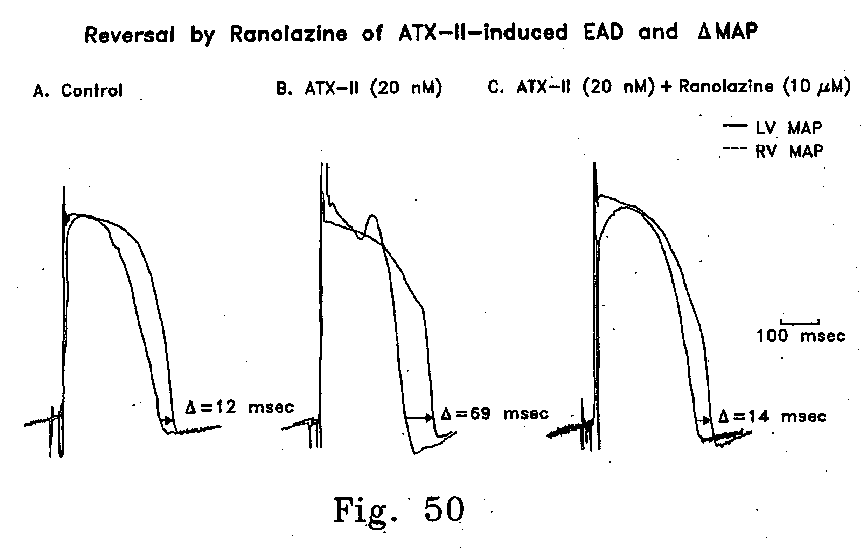

Figure 50. Effects of ATXII (20 nM) and ranolazine on induced EAD and ΔMAP.

### DETAILED DESCRIPTION OF THE INVENTION

The invention provides a means ofsuppressing early after depolarization in a mammal.

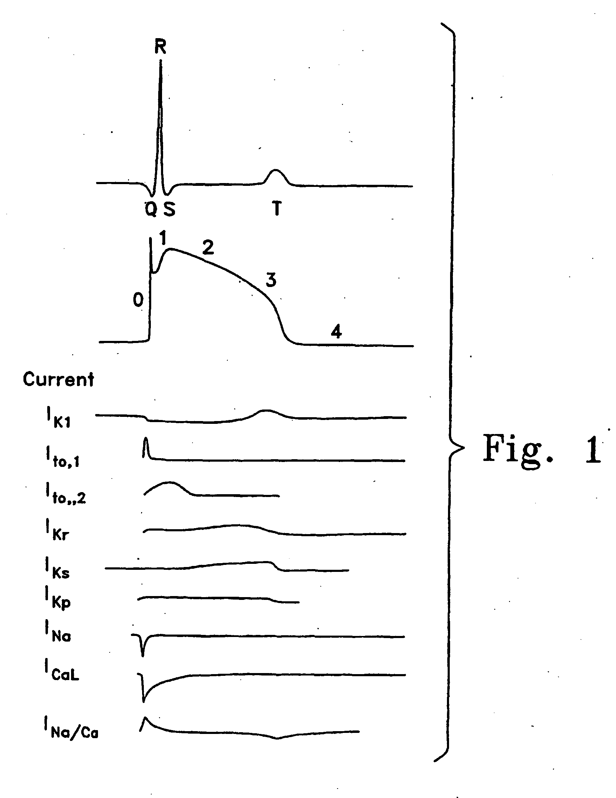

Normal heart rhythm (sinus rhythm) results from action potentials (APs), which are generated by the highly integrated electrophysiological behavior of ion channels on multiple cardiac cells. Sodium, calcium and potassium channels are the most important channels for determining the shape and the duration of the cardiac action potential. Briefly, activation of sodium and calcium channels leads to the influx of positively charged ions into individual cardiac cells, causing depolarization of the membrane. Conversely, the opening of potassium channels allows the flow of positive charge out of the cells and, in large part, terminates the action potential and repolarizes the cell (Figure. 1).

APs are propagated from their origin in the pacemaker, through the sinoatrial node, through the atrial muscle, then through the atrioventricular node (AV), through the Purkinje conduction system, and finally to the ventricle.

Arrhythmia, a disruption in the normal sequence of impulse initiation and propagation in the heart, may result from primary cardiovascular disease, pulmonary disorders, autonomic disorders, systemic disorders, drug-related side effects, inherited effects (mutations of genes), or electrolyte imbalances.

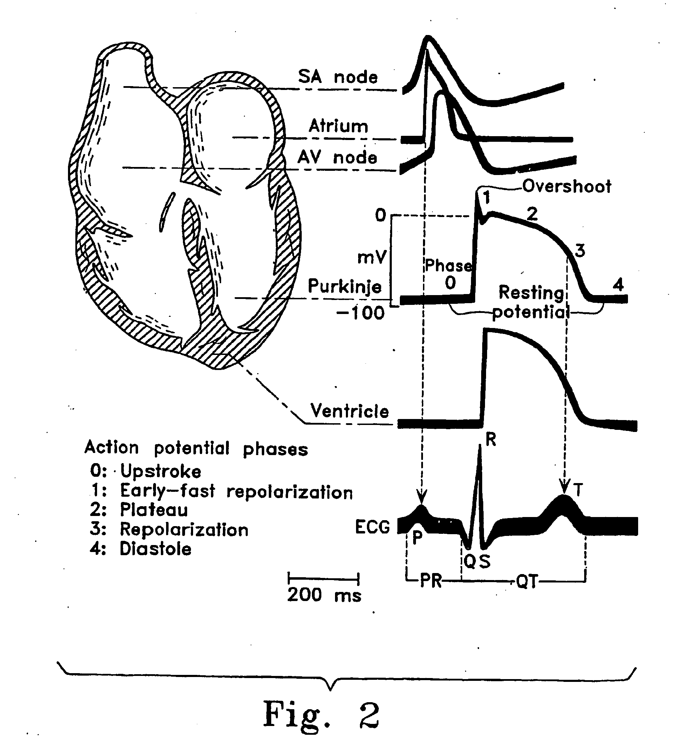

Normal sinus rhythm and arrhythmias are visualized on electrocardiograms (ECGs). An ECG is a graphic tracing of the variations in electrical potential caused by the excitation of the heart muscle and detected at the body surface. From the electrocardiograms heart rate, PR interval duration, a reflection of AV nodal conduction time, QRS duration, a reflection of conduction time in the ventricle, and QT interval, which is a measure of ventricular action potential duration, can be measured. A representation of the ECG generated during sinus rhythm is shown in Figure 2.

Ventricular tachycardias are caused by enhanced automaticity, afterdepolarizations and triggered automaticity and reentry. Enhanced automaticity occurs in cells that normally display spontaneous diastolic depolarization. B-adreneric stimulation, hypokalemia, and mechanical stretch of cardiac muscle cells increase phase 4 slope and so accelerate pacemaker rate, whereas acetylcholine reduces pacemaker rate both by decreasing phase 4 slope and by hyperpolarization. When impulses propagate from a region of enhanced normal or abnormal automaticity to excite the rest of the heart arrhythmias result.

Afterdepolarizations and triggered automaticity occur under some pathophysiological conditions in which a normal cardiac action potential is interrupted or followed by an abnormal depolarization. If this abnormal depolarization reaches threshold, it may, in turn, give rise to secondary upstrokes, which then can propagate and create abnormal rhythms. These abnormal secondary upstrokes occur only after an initial normal, or "triggering," upstroke and so are termed triggered rhythms. Two major forms of triggered rhythms are recognized: (1) delayed afterpolarization (DAD) that may occur under conditions of intracellular calcium overload (myocardial ischemia, adrenergic stress, etc). If this afterdepolarization reaches threshold, a secondary triggered beat or beats may occur and; (2) early afterdepolarizations (EADs) often occur when there is a marked prolongation of the cardiac action potential. When this occurs, phase 3 repolarization may be interrupted by an EAD. EAD-mediated triggering in vitro and clinical arrhythmias are most common when the underlying heart rate is slow, extracellular K+ is low, and certain drugs that prolong action potential duration are present. EADs result from an increase in net inward current during the repolarization phase of the action potential.

TdP is a common and serious side effect of treatment with many different types of drugs; and could be caused by EADs and the resultant triggering. However, there are other conditions that measure the risk of TdP, including hypokalemia, hypomagnesemia, hypocalcemia, high-grade AV block, congenital disorders and severe bradycardia.

Long QT Syndrome (LQTS) is caused by dysfunction of protein structures in the heart cells called ion channels. These channels control the flow of ions like potassium, sodium and calcium molecules. The flow of these ions in and out of the cells produces the electrical activity of the heart. Abnormalities of these channels can be acquired or inherited. The acquired form is usually caused by prescription medications.

The inherited form occurs when a mutation develops in one of several genes that produce or "encode" one of the ion channels that control electrical repolarization. The mutant gene produces abnormal channels to be formed, and as these abnormal channels are not as efficient as the normal channels, the electrical recovery of the heart takes longer. This is manifest on the electrocardiogram (ECG, EKG) by a prolonged QT interval. QT prolongation makes the heart vulnerable to polymorphic VTs, one kind of which is a fast, abnormal heart rhythm known as "Torsade de Pointes".

The congenital LQTS is caused by mutations of at least one of six genes

| Disease | Gene | Chromosome | Ion Channel |
|---|---|---|---|
| | | | |
| LQT1 | KVLQT1* | 11p15.5 | I_{Ks} subunit |
| LQT2 | HERG | 7q3 5-36 | I_{Kr} |
| LQT3 | SCNSA | 3q2I-24 | Na |
| LQT4 | E1425G | 4q25-27 | Ca²⁺ |
| LQT5 | MinK | 21 | I_{Ks} subunit |

| | | | |
|---|---|---|---|
| *Homozygous carriers of novel mutations of KVLQT1 have Jervell, Lange-Nielsen syndrome. KVLQT1 and MinK coassemble to form the I_{Ks} channel. | | | |

The LQT diseases and ion channels listed in the table above are the same for acquired LQTS as they are for inherited LQTS.

It should be noted that if the inherited or acquired form of LQTS is present in a mammal, and symptoms of a VT have appeared, then administration of a compound of Formula I, especially ranolazine, reduces the occurrence and/or frequency of VT. If the inherited or acquired form of LQTS is present, but there are no symptoms of VT, then administration of a compound of Formula I, especially ranolazine, prevents the occurrence of VT.

Sodium pentobarbital is known to prolong QT interval, but also reduces the transmural dispersion of repolarization. It does this by inhibiting I_{Kr}, I_{Ks} and I_{Na} most prominently. Transmural dispersion reduction is shown by a greater prolongation of APD in epi and endo cells than in M cells. Sodium pentobarbital also suppresses d-sotalol-induced EAD activity in M cells. Thus, despite its actions to prolong QT, pentobarbital does not induce TdP.

Amiodarone is known to prolong QT and at low instances induce TdP. It was found that amiodarone reduces transmural dispersion of repolarization by exhibiting a greater prolongation of APD in epi and endo cells than in M cells. Amiodarone blocks the sodium, potassium and calcium channels in the heart. When administered chronically (30-40 mg/kg/day orally for 30-45 days) it also suppresses the ability of the I_{Kr} blocker, d-sotalol, to induce a marked dispersion of repolarization or EAD activity.

In arterially-perfused wedge preparations from the canine left ventricle ranolazine was found to preferentially prolong APD₉₀ of epicardial (epc) cells. The reduction in transmural dispersion was found to be more pronounced at higher concentrations because ranolazine also abbreviates the APD₉₀ of the M cells while prolonging that of the epi cells.

Tests also were carried out in isolated myocytes from canine left ventricle to determine if ranolazine induces EADs and whether ranolazine's action on late sodium current and calcium current can antagonize EAD induction by d-sotalol in Purkinje fibers. EADs were not observed in the presence of ranolazine. Ranolazine was found to suppress EADs induced by d-sotalol at concentrations as low as 5 micromolar/L.

It was also found that ranolazine blocks the calcium channel, but does so at a concentration (296 micromolar/L) very much higher than the therapeutic concentration of the drug (- 2 to 8 µM).

Thus, even if ranolazine exhibits a prolonged QT interval, it does not induce EADs or TdP.

Because ranolazine may cause a prolonged QT interval, ranolazine may increase the duration of APD of ventricular myocytes. The QT interval of surface EKG reflects the duration of ventricular repolarization.

It was found that ranolazine decreased the APD of guinea pig myocytes (reversible on washout). Ranolazine also was found to reduce APD in the presence of quinidine. Quinidine is known to trigger EADs and TdP. Ranolazine was found to suppress EADs and other triggered activity induced by quinidine

ATXII (a sea anemone toxin) slows the inactivation of the open state of the sodium channel, triggers EADs, prolongs QT interval, and causes a sharp rise in transmural dispersion of repolarization as a result of greater prolongation of APD in M cells. Data shows that ranolazine causes a decrease in APD in the presence of ATXII. Therefore, ranolazine suppresses EADs induced by ATXII. ATXII is a sodium ion activator that mimics LTQ3 syndrome (which leads to TdP). Thus, ranolazine does not lead to TdP, instead suppresses TdP caused by ATX. Definitions

As used in the present specification, the following words and phrases are generally intended to have the meanings as set forth below, except to the extent that the context in which they are used indicates otherwise.

"Aminocarbonylmethyl" refers to a group having the following structure: 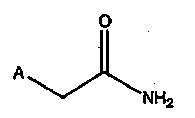 where A represents the point of attachment.

"Halo" or "halogen" refers to fluoro, chloro, bromo or iodo.

"Lower acyl" refers to a group having the following structure: 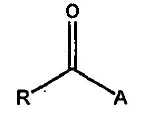 where R. is lower alkyl as is defined herein, and A represents the point of attachment, and includes such groups as acetyl, propanoyl, n-butanoyl and the like.

"Lower alkyl" refers to a unbranched saturated hydrocarbon chain of 1-4 carbons, such as methyl, ethyl, n-propyl, and n-butyl.

"Lower alkoxy" refers to a group -OR wherein R is lower alkyl as herein defined.

"Lower alkylthio" refers to a group -SR wherein R is lower alkyl as herein defined.

"Lower alkyl sulfinyl" refers to a group of the formula: 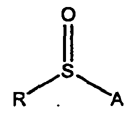 wherein R is lower alkyl as herein defined, and A represents the point of attachment.

"Lower alkyl sulfonyl" refers to a group of the formula: 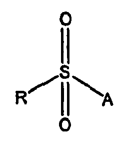 wherein R is lower alkyl as herein defined., and A represents the point of attachment.

"N-Optionally substituted alkylamido" refers to a group having the following structure: 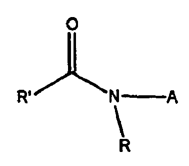 wherein R is independently hydrogen or lower alkyl and R' is lower alkyl as defined herein, and A represents the point of attachment

The term "drug" or "drugs" refers to prescription medications as well as over-the-counter medications and all pharmacological agents.

"Isomers" refers to compounds having the same atomic mass and atomic number but differing in one or more physical or chemical properties. All isomers of the compound of Formula I are within the scope of the invention.

"Optional" or "optionally" means that the subsequently described event or circumstance may or may not occur, and that the description includes instances where said event or circumstance occurs and instances in which it does not.

The term "therapeutically effective amount" refers to that amount of a compound of Formula I that is sufficient to effect treatment, as defined below, when administered to a mammal in need of such treatment The therapeutically effective amount will vary depending upon the subject and disease condition being treated, the weight and age of the subject, the severity of the disease condition, the manner of administration and the like, which can readily be determined by one of ordinary skill in the art.

The term "treatment" or "treating" means any treatment of a disease in a mammal, including:
(i) preventing the disease, that is, causing the clinical symptoms of the disease not to develop;
(ii) inhibiting the disease, that is, arresting the development of clinical symptoms; and/or
(iii) relieving the disease, that is, causing the regression of clinical symptoms.

Arrhythmia refers to any abnormal heart rate. Bradycardia refers to abnormally slow heart rate whereas tachycardia refers to an abnormally rapid heart rate. As used herein, the treatment of arrhythmia is intended to include the treatment of supra ventricular tachycardias such as atrial fibrillation, atrial flutter, AV nodal reentrant tachycardia, atrial tachycardia, and the ventricular tachycardias (VTs), including idiopathic ventricular tachycardia, ventricular fibrillation, pre-excitation syndrome, and Torsade de Pointes (TdP),

Sinus rhythm refers to normal heart rate.

The term "cardiac compromised mammal" means a mammal having cardiopathological disease state, for example angina, congestive heart failure, ischemia and the like.

In many cases, the compounds of this invention are capable of forming acid and/or base salts by virtue of the presence of amino and/or carboxyl groups or groups similar thereto. The term "pharmaceutically acceptable salt" refers to salts that retain the biological effectiveness and properties of the compounds of Formula I, and which are not biologically or otherwise undesirable. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocyclic amines, diheterocyclic amines, triheterocyclic amines, mixed di- and tri-amines where at least two of the substituents on the amine are different and are selected from the group consisting of alkyl, substituted alkyl, alkenyl, substituted alkenyl, cycloalkyl, substituted cycloalkyl, cycloalkenyl, substituted cycloalkenyl, aryl, heteroaryl, heterocyclic, and the like. Also included are amines where the two or three substituents, together with the amino nitrogen, form a heterocyclic or heteroaryl group.

Specific examples of suitable amines include, by way of example only, isopropylamine, trimethyl amine, diethyl amine, tri(iso-propyl) amine, tri(n-propyl) amine, ethanolamine, 2-dimethylaminoethanol, tromediamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine, and the like.

Pharmaceutically acceptable acid addition salts may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluene-sulfonic acid, salicylic acid, and the like.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

### Pharmaceutical Compositions and Administration

The compounds of the invention are usually administered in the form of pharmaceutical compositions. This invention therefore provides pharmaceutical compositions that contain, as the active ingredient, one or more of the compounds of the invention, or a pharmaceutically acceptable salt or ester thereof, and one or more pharmaceutically acceptable excipients; carriers, including inert solid diluents and fillers; diluents, including sterile aqueous solution and various organic solvents; permeation enhancers; solubilizers; and adjuvants. The compounds of the invention may be administered alone or in combination with other therapeutic agents. Such compositions are prepared in a manner well known in the pharmaceutical art (see, e.g., Remington's Pharmaceutical Sciences, Mace Publishing Co., Philadelphia, PA 17th Ed. (1985) and "Modern Pharmaceutics", Marcel Dekker, Inc. 3rd Ed. (G.S. Banker & C.T. Rhodes, Eds.).

The compounds of the invention may be administered in either single or multiple doses by any of the accepted modes of administration of agents having similar utilities, for example as described in the above-mentioned patents and patent applications, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

One preferred mode for administration is parental, particularly by injection. The forms in which the novel compositions of the present invention may be incorporated for administration by injection include aqueous or oil suspensions, or emulsions, with sesame oil, com oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present invention. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating the compound of the invention in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Oral administration is another route for administration of the compounds of Formula I. Administration may be via capsule or enteric coated tablets, or the like. In making the pharmaceutical compositions that include at least one compound of Formula I, the active ingredient is usually diluted by an excipient and/or enclosed within such a carrier that can be in the form of a capsule, sachet, paper or other container. When the excipient serves as a diluent, it can be a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents.

The compositions of the invention can be formulated so as to provide quick, sustained, delayed release or any combination of these release means of the active ingredient after administration to the patient by employing procedures known in the art. Controlled release drug delivery systems for oral administration include osmotic pump systems and diffusion/dissolution systems including polymer-coated reservoirs or drug-polymer matrix formulations. Examples of controlled release systems are given in U.S. Patent Nos. 3,845,770; 4,326,525; 4,902,514; and 5,616,345 and WO 0013687. Another formulation for use in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art See, e.g., U.S. Patent Nos. 5,023,252, 4,992,445 and 5,001,139. Such patches may be constructed for continuous, pulsatile, or on demand delivery of pharmaceutical agents.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., a tablet, capsule, ampoule). The compounds of Formula I are effective over a wide dosage range and is generally administered in a pharmaceutically effective amount Preferably, for oral administration, each dosage unit contains from 10 mg to 2 g of a compound of Formula I, more preferably from 10 to 700 mg, and for parenteral administration, preferably from 10 to 700 mg of a compound of Formula I, more preferably about 50 to about 200 mg. It will be understood, however, that the amount of the compound of Formula I actually administered will be determined by a physician, in the light of the relevant circumstances, including the condition to be treated, the chosen route of administration, the actual compound administered and its relative activity, the age, weight, and response of the individual patient, the severity of the patient's symptoms, and the like.

For preparing solid compositions such as tablets, the principal active ingredient is mixed with a pharmaceutical excipient to form a solid pre-formulation composition containing a homogeneous mixture of a compound of the present invention. When referring to these pre-formulation compositions as homogeneous, it is meant that the active ingredient is dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

In one embodiment, the preferred compositions of the invention are formulated so as to provide quick, sustained or delayed release of the active ingredient after administration to the patient, especially sustained release formulations. The most preferred compound of the invention is ranolazine, which is named (±)-N-(2,6-dimethyl-phenyl)-4-[2-hydroxy-3-(2 methoxyphenoxy)propyl]-1-piperazine-acetamide. Unless otherwise stated, the ranolazine plasma concentrations used in the specification and examples refers to ranolazine free base.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner.

The intravenous formulation of ranolazine is manufactured via an aseptic fill process as follows. In a suitable vessel, the required amount of Dextrose Monohydrate is dissolved in Water for Injection (WFI) at approximately 78% of the final batch weight. With continuous stirring, the required amount of ranolazine free base is added to the dextrose solution. To facilitate the dissolution of ranolazine, the solution pH is adjusted to a target of 3.88-3.92 with 0.1N or 1N Hydrochloric Acid solution. Additionally, 0.1N HCl or 1.0N NaOH may be utilized to make the final adjustment of solution to the target pH. of 3.88-3.92. After ranolazine is dissolved, the batch is adjusted to the final weight with WFI. Upon confirmation that the in-process specifications have been met, the ranolazine bulk solution is sterilized by sterile filtration through two 0.2 µm sterile filters. Subsequently, the sterile ranolazine bulk solution is aseptically filled into sterile glass vials and aseptically stoppered with sterile stoppers. The stoppered vials are then sealed with clean flip-top aluminum seals.

Compounds of the invention may be impregnated into a stent by diffusion, for example, or coated onto the stent such as in a gel form, for example, using procedures known to one of skill in the art in light of the present disclosure.

The intravenous formulation of ranolazine is manufactured via an aseptic fill process as follows. In a suitable vessel, the required amount of Dextrose Monohydrate is dissolved in Water for Injection (WFI) at approximately 78% of the final batch weight. With continuous stirring, the required amount of ranolazine free base is added to the dextrose solution. To facilitate the dissolution of ranolazine, the solution pH is adjusted to a target of 3.88-3.92 with O.1N or 1N Hydrochloric Acid solution. Additionally, 0.1N HCl or 1.0N NaOH may be utilized to make the final adjustment of solution to the target pH of 3.88-3.92. After ranolazine is dissolved, the batch is adjusted to the final weight with WFI. Upon confirmation that the in-process specifications have been met, the ranolazine bulk solution is sterilized by sterile filtration through two 0.2 µm sterile filters. Subsequently, the sterile ranolazine bulk solution is aseptically filled into sterile glass vials and aseptically stoppered with sterile stoppers. The stoppered vials are then sealed with clean flip-top aluminum seals.

The preferred sustained release formulations of this invention are preferably in the form of a compressed tablet comprising an intimate mixture of compound and a partially neutralized pH-dependent binder that controls the rate of dissolution in aqueous media across the range of pH in the stomach (typically approximately 2) and in the intestine (typically approximately about 5.5).

To provide for a sustained release of compound, one or more pH-dependent binders may be chosen to control the dissolution profile of the compound so that the formulation releases the drug slowly and continuously as the formulation passed through the stomach and gastrointestinal tract The dissolution control capacity of the pH-dependent binder(s) is particularly important in a sustained release formulation because a sustained release formulation that contains sufficient compound for twice daily administration may cause untoward side effects if the compound is released too rapidly ("dose-dumping").

Accordingly, the pH-dependent binders suitable for use in this invention are those which inhibit rapid release of drug from a tablet during its residence in the stomach (where the pH is below about 4.5), and which promotes the release of a therapeutic amount of compound from the dosage form in the lower gastrointestinal tract (where the pH is generally greater than about 4.5). Many materials known in the pharmaceutical art as "enteric" binders and coating agents have the desired pH dissolution properties. These include phthalic acid derivatives such as the phthalic acid derivatives of vinyl polymers and copolymers, hydroxyalkylcelluloses, alkylcelluloses, cellulose acetates, hydroxyalkylcellulose acetates, cellulose ethers, alkylcellulose acetates, and the partial esters thereof, and polymers and copolymers of lower alkyl acrylic acids and lower alkyl acrylates, and the partial esters thereof.

Preferred pH-dependent binder materials that can be used in conjunction with the compound to create a sustained release formulation are methacrylic acid copolymers. Methacrylic acid copolymers are copolymers of methacrylic acid with neutral acrylate or methacrylate esters such as ethyl acrylate or methyl methacrylate. A most preferred copolymer is methacrylic acid copolymer, Type C, USP (which is a copolymer of methacrylic acid and ethyl acrylate having between 46.0% and 50.6% methacrylic acid units). Such a copolymer is commercially available, from Röhm Pharma as Eudragit® L 100-55 (as a powder) or L30D-55 (as a 30% dispersion in water). Other pH-dependent binder materials which may be used alone or in combination in a sustained release formulation dosage form include hydroxypropyl cellulose phthalate, hydroxypropyl methylcellulose phthalate, cellulose acetate phthalate, polyvinylacetate phthalate, polyvinylpyrrolidone phthalate, and the like. One or more pH-dependent binders are present in the dosage forms of this invention in an amount ranging from about 1 to about 20 wt%, more preferably from about 5 to about 12 wt% and most preferably about 10 wt%.

One or more pH-independent binders may be in used in sustained release formulations in oral dosage forms. It is to be noted that pH-dependent binders and viscosity enhancing agents such as hydroxypropyl methylcellulose, hydroxypropyl cellulose, methylcellulose, polyvinylpyrrolidone, neutral poly(meth)acrylate esters, and the like, do not themselves provide the required dissolution control provided by the identified pH-dependent binders. The pH-independent binders are present in the formulation of this invention in an amount ranging from about I to about 10 wt%, and preferably in amount ranging from about 1 to about 3 wt% and most preferably about 2.0 wt%.

As shown in Table 1, the preferred compound of the invention, ranolazine, is relatively insoluble in aqueous solutions having a pH above about 6.5, while the solubility begins to increase dramatically below about pH 6. In the following examples solutions of ranolazine in water or solutions with a pH above 6 are made up of ranolazine dihydrochloride. In the discussion portions of the following examples, concentrations of ranolazine found as a result of experiments are calculated as ranolazine free base.

**Table 1**

| Solution pH | Solubility (mg/mL) | USP Solubility Class |
|---|---|---|
| 4.81 | 161 | Freely Soluble |
| 4.89 | 73.8 | Soluble |
| 4.90 | 76.4 | Soluble |
| 5.04 | 49.4 | Soluble |
| 5.35 | 16.7 | Sparingly Soluble |
| 5.82 | 5.48 | Slightly soluble |
| 6.46 | 1.63 | Slightly soluble |
| 6.73 | 0.83 | Very slightly soluble |
| 7.08 | 0.39 | Very slightly soluble |
| 7.59 (unbuffered water) | 0.24 | Very slightly soluble |
| 7.79 | 0.17 | Very slightly soluble |
| 12.66 | 0.18 | Very slightly soluble |

Increasing the pH-dependent binder content in the formulation decreases the release rate of the sustained release form of the compound from the formulation at pH is below 4.5 typical of the pH found in the stomach. The enteric coating formed by the binder is less soluble and increases the relative release rate above pH 4.5, where the solubility of compound is lower. A proper selection of the pH-dependent binder allows for a quicker release rate of the compound from the formulation above pH 4.5, while greatly affecting the release rate at low pH. Partial neutralization of the binder facilitates the conversion of the binder into a latex like film which forms around the individual granules. Accordingly, the type and the quantity of the pH-dependent binder and amount of the partial neutralization composition are chosen to closely control the rate of dissolution of compound from the formulation.

The dosage forms of this invention should have a quantity of pH-dependent binders sufficient to produce a sustained release formulation from which the release rate of the compound is controlled such that at low pHs (below about 4.5) the rate of dissolution is significantly slowed. In the case of methacrylic acid copolymer, type C, USP (Eudragit® L 100-55), a suitable quantity of pH-dependent binder is between 5% and 15%. The pH dependent binder will typically have from about 1 to about 20 % of the binder methacrylic acid carboxyl groups neutralized. However, it is preferred that the degree of neutralization ranges from about 3 to 6%. The sustained release formulation may also contain pharmaceutical excipients intimately admixed with the compound and the pH-dependent binder. Pharmaceutically acceptable excipients may include, for example, pH-independent binders or film-forming agents such as hydroxypropyl methylcellulose, hydroxypropyl cellulose, methylcellulose, polyvinylpyrrolidone, neutral poly(meth)acrylate esters (e.g. the methyl methacrylate/ethyl acrylate copolymers sold under the trademark Eudragit® NE by Röhm Pharma, starch,gelatin, sugars carboxymethyl cellulose, and the like. Other useful pharmaceutical excpients include diluents such as lactose, mannitol, dry starch, microcrystalline cellulose and the like; surface active agents such as polyoxyethylene sorbitan esters, sorbitan esters and the like; and coloring agents and flavoring agents. Lubricants (such as tale and magnesium stearate) and other tableting aids are also optionally present.

The sustained release formulations of this invention preferably have a compound content of about 50% by weight to about 95% or more by weight, more preferably between about 70% to about 90% by weight and most preferably from about 70 to about 80% by weight; a pH-dependent binder content of between 5% and 40%, preferably between 5% and 25%, and more preferably between 5% and 15%; with the remainder of the dosage form comprising pH-independent binders, fillers, and other optional excipients. Some preferred sustained release formulations of this invention are shown below in Table 2.

**Table 2.**

| Ingredient | Weight | Preferred Weight | Most Preferred |
|---|---|---|---|
| | Range (%) | Range (%) | Weigh Range (%) |
| Active ingredient | 0-95 | 70-90 | 75 |
| Microcrystalline cellulose (filler) | 1-35 | 5-15 | 10.6 |
| Methacrylic acid copolymer | 1-35 | 5-12.5 | 10.0 |
| Sodium hydroxide | 0.1-1.0 | 0.2-0.6 | 0.4 |
| Hydroxypropyl methylcellulose | 0.5-5.0 | 1-3 | 2.0 |
| Magnesium stearate | 0.5-5.0 | 1-3 | 2.0 |

The sustained release formulations of this invention are prepared as follows: compound and pH-dependent binder and any optional excipients are intimately mixed(dry-blended). The dry-blended mixture is then granulated in the presence of an aqueous solution of a strong base that is sprayed into the blended powder. The granulate is dried, screened, mixed with optional lubricants (such as talc or magnesium stearate), and compressed into tablets. Preferred aqueous solutions of strong bases are solutions of alkali metal hydroxides, such as sodium or potassium hydroxide, preferably sodium hydroxide, in water (optionally containing up to 25% of water-miscible solvents such as lower alcohols).

The resulting tablets may be coated with an optional film-forming agent, for identification, taste-masking purposes and to improve ease of swallowing. The film forming agent will typically be present in an amount ranging from between 2% and 4% of the tablet weight. Suitable film-forming agents are well known to the art and include hydroxypropyl. methylcellulose, cationic methacrylate copolymers (dimethylaminoethyl methacrylate/ methyl-butyl methacrylate copolymers - Eudragit® E - Röhm. Pharma), and the like. These film-forming agents may optionally contain colorants, plasticizers, and other supplemental ingredients.

The compressed tablets preferably have a hardness sufficient to withstand 8 Kp compression. The tablet size will depend primarily upon the amount of compound in the tablet. The tablets will include from 300 to 1100 mg of compound free base. Preferably, the tablets will include amounts of compound free base ranging from 400-600 mg, 650-850 mg, and 900-1100 mg.

In order to influence the dissolution rate, the time during which the compound containing powder is wet mixed is controlled. Preferably the total powder mix time, i.e. the time during which the powder is exposed to sodium hydroxide solution, will range from 1 to 10 minutes and preferably from 2 to 5 minutes. Following granulation, the particles are removed from the granulator and placed in a fluid bed dryer for drying at about 60°C.

It has been found that these methods produce sustained release formulations that provide lower peak plasma levels and yet effective plasma concentrations of compound for up to 12 hours and more after administration, when the compound used as its free base, rather than as the more pharmaceutically common dihydrochloride salt or as another salt or ester. The use of free base affords at least one advantage: The proportion of compound in the tablet can be increased, since the molecular weight of the free base is only 85% that of the dihydrochloride. In this manner, delivery of an effective amount of compound is achieved while limiting the physical size of the dosage unit

### Utility and Testing

The method is effective in the treatment of conditions that respond to concurrent inhibition of I_{Kr}, I_{Ks} and late I_{Na} channels. Such conditions include VT, as exemplified by idiopathic ventricular tachycardia, ventricular fibrillation, pre-excitation syndrome, and Torsade de Pointes

Activity testing is conducted as described in the Examples below, and by methods apparent to one skilled in the art

The Examples that follow serve to illustrate this invention. The Examples are intended to show how to make and use the compounds of this invention. In the Examples, all temperatures are in degrees Centigrade.

The following examples illustrate the preparation of representative pharmaceutical formulations containing a compound of Formula I.

### EXAMPLE 1

Hard gelatin capsules containing the following ingredients are prepared:

| Quantity | |
|---|---|
| Ingredient | (mg/capsule) |
| Active Ingredient | 30.0 |
| Starch | 305.0 |
| Magnesium stearate | 5.0 |

The above ingredients are mixed and filled into hard gelatin capsules.

### EXAMPLE 2

A tablet formula is prepared using the ingredients below:

| INGREDIENT | (mg/TABLET) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets.

### EXAMPLE 3

A dry powder inhaler formulation is prepared containing the following components:

| Ingredient | Weight % |
|---|---|
| Active Ingredient | 5 |
| Lactose | 95 |

The active ingredient is mixed with the lactose and the mixture is added to a dry powder inhaling appliance.

### EXAMPLE 4

Tablets, each containing 30 mg of active ingredient, are prepared as follows:

| Quantity | |
|---|---|
| Ingredient | (mg/tablet) |
| Active Ingredient | 30.0 mg |
| Starch | 45.0 mg |
| Microcrystalline cellulose | 35.0 mg |
| Polyvinylpyrrolidone (as 10% solution in sterile water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50 °C to 60 °C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

### EXAMPLE 5

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### EXAMPLE 6

Suspensions, each containing 50 mg of active ingredient per 5.0 mL dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 mL |

The active ingredient, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### EXAMPLE 7

A subcutaneous formulation may be prepared as follows:

| Ingredient | Quantity |
|---|---|
| Active Ingredient | 5.0 mg |
| Com Oil | 1.0 mL |

### EXAMPLE 8

An injectable preparation is prepared having the following composition:

| Ingredients | Amount |
|---|---|
| Active ingredient | 2.0 mg/ml |
| Mannitol, USP | 50 mg/ml |
| Gluconic acid, USP | q.s. (pH 5-6) |
| water (distilled, sterile) | q.s. to 1.0 ml |
| Nitrogen Gas, NF | q.s. |

### EXAMPLE 9

A topical preparation is prepared having the following composition:

| Ingredients | grams |
|---|---|
| Active ingredient | 0,2-10 |
| Span 60 | 2.0 |
| Tween 60 | 2.0 |
| Mineral oil | 5.0 |
| Petrolatum | 0.10 |
| Methyl paraben | 0.15 |
| Propyl paraben | 0.05 |
| BHA (butylated hydroxy anisole) | 0.01 |
| Water | q.s. to100 |

All of the above ingredients, except water, are combined and heated to 60⁾ C with stirring. A sufficient quantity of water at 60⁾ C is then added with vigorous stirring to emulsify the ingredients, and water then added q.s. 100 g.

The following examples demonstrate the utility of the compounds of the invention.

### EXAMPLE 10

### I. Electrophysiologic Effects of Ranolazine in Isolated Myocytes, Tissues and Arterially-Perfused Wedge Preparations from the Canine Left Ventricle

### A. Material and Methods

Dogs weigthing 20-25 kg were anticoagulated with heparin (180 IU/kg) and anesthetized with pentobarbital (30-35 mg/kg, i.v.). The chest was opened via a left thoracotomy, the heart excised and placed in a cold cardioplegic solution ([K⁺]₀= 8 mmol/L, 4°C). All protocols were in conformance with guidelines established by the Institutional Animal Care and Use Committee.

### 1. Voltage Clamp Studies in Isolated Canine Ventricular Myocytes

Myocytes were isolated by enzymatic dissociation from a wedge-shaped section of the left ventricular free wall supplied by the left circumflex coronary artery. Cells from the epicardial and midmyocardial regions of the left ventricle were used in this study.

Tyrode's solution used in the dissociation contained (in mM): 135 NaCl, 5.4 KCl, 1 MgCl₂, 0 or 0.5 CaCl₂, 10 glucose, 0.33 NaH₂PO₄, 10 N-2-hydroxyethylpiperazine-N'-2-ethanesulfonic acid (HEPES), and the pH was adjusted to 7.4 with NaOH.

Inward rectifier potassium current (I_{K1}), slow delayed rectifier potassium current (I_{Ks}), and rapid delayed rectifier potassium current (I_{Kr}) were recorded at 37° C using conventional whole cell voltage clamp configuration. The composition of the external and pipette solutions used to isolate specific ionic currents is summarized in Table 3.

**Table 3**

| External Solutions | | Pipette Solution |
|---|---|---|
| I_{Kr} and I_{KI} (mM) | I_{Ks} (mM) | I_{Ks}, I_{Kr} and Iₖₗ (mM) |
| 11 glucose | 11 glucose | 20 KCl |
| 4 KCl | 4 KCl | 125 K-Aspartate |
| 1.2 MgSO₄ | 1.8 MgCl₂ | 1 MgCl₂ |
| 2 CaCl₂ | 1.8 CaCl₂ | 10 EGTA |
| 132 NaCl | 145 Nad | 5 MgATP |
| | 1 NaH₂PO₄ | |
| 20 HEPES | 10 HEPES | 5 HEPES |
| pH 7.4 with NaOH | pH 7.4 with NaOH | pH 7.1 with KOH |

I_{K1} was measured using an external solution containing 3 µM ouabain and 5 µM nifedipine to block the sodium-potassium pump and L-type calcium current (I_{Ca,L}), respectively. I_{Ks} was measured in the presence of 5 µM E-4031 and 5 µM nifedipine to block I_{Kr} and I_{Ca}. 5 µM nifedipine was present in the external solution when I_{Kr} was being recorded.

Isolated myocytes were placed in a temperature controlled 0.5 ml chamber (Medical Systems, Greenvale, NY) on the stage of an inverted microscope and superfused at a rate of 2 ml/min. An eight-barrel quartz micromanifold (ALA Scientific Instruments Inc., Westbury, NY) placed 100 µm from the cell was used to apply ranolazine at concentrations of (in µM): 0.1, 0.5, 1.0, 5.0, 10 and 100.0. An Axopatch ID amplifier (Axon Instruments, Foster City, CA) was operated in voltage clamp mode to record currents. Whole cell currents were filtered with a 3-pole low-pass Bessel filter at 5 kHz, digitized between 2 - 5 kHz (Digidata 1200A, Axon Instruments) and stored on a computer. Clampex 7 acquisition and analysis software (Axon Instruments) was used to record and analyze ionic currents. Pipette tip resistance was 1.0- 2.0 M Ω and seal resistance was greater than 5 GΩ. Electronic compensation of series resistance averaged 76 %. Voltages reported in the text were corrected for patch electrode tip potentials. The seal between cell membrane and patch pipette was initially formed in external solution containing 1 mM CaCl₂. A 3 M KCl-agar bridge was used between the Ag/AgCl ground electrode and external solution to avoid development of a ground potential when switching to experimental solution.

I_{Ks} was elicited by depolarization to 40 mV for 3 sec from a holding potential of -50 mV followed by a repolarization step to 0 mV (4.5 sec). The time-dependent tail current elicited by the repolarization was termed I_{Ks}. This protocol was repeated 5 times every 20 sec. Iₜₒ was not blocked, but it had little influence on our measurement of I_{Ks} because of its fast and complete inactivation. All measurements were obtained 5-12 min after patch rupture since no significant run-down of I_{Ks} is observed during this interval.

I_{Kr} was measured as the time-dependent tail current elicited at a potential of - 40 mV following a short 250 ms depolarizing pulse to 30 mV. Data are presented as mean ± S.E.M. I_{K1} was recorded during 900 msec voltage steps applied from a holding potential of - 40 mV to test potentials ranging from -100 mV to 0 mV, and was characterized as the 5 msec average of the steady state current at the end of the test pulse.

### 2. Action Potential Studies in Isolated Canine Ventricular Epicardial and M region Tissues

Epicardial and midmyocardial (M) cell preparations (strips approximately 1 x 0.5 x 0.15 cm) were isolated from the left ventricle. The tissue slices were placed in a tissue bath (5 ml volume with flow rate of 12 ml/min) and allowed to equilibrate for at least 4 hours while superfused with an oxygenated Tyrode's solution (pH=7.35, t° =37 ± 0.5°C) and paced at a basic cycle length (BCL) of 2 Hz using field stimulation. The composition of the Tyrode's solution was (in mM): NaCl 129, KCl 4, NaH₂PO₄ 0.9, NaHCO₃ 20, CaCl₂ 1.8, MgSO₄ 0.5, and D-glucose 5.5.

Action potential recordings: Transmembrane potentials were recorded using standard glass microelectrodes filled with 2.7 M KCl (10 to 20 MΩ DC resistance) connected to a high input-impedance amplification system (World Precision Instruments, Sarasota, FL, USA). Amplified signals were displayed on Tektronix (Beaverton, OR, USA) oscilloscopes and amplified (model 1903-4 programmable amplifiers [Cambridge Electronic Designs (C.E.D.), Cambridge, England]), digitized (model 1401 AD/DA system [C.E.D.]), analyzed (Spike 2 acquisition and analysis module [C.E.D.], and stored on magnetic media.

Study protocols: Action potentials were recorded from epicardial and M cell preparations. Control recordings were obtained after a 4-6 hour equilibrium period. The effects of ranolazine were determined at concentrations of 1, 5,10, 50, and 100 µM, with recordings started 30 minutes after the addition of each concentration of the drug. Rate-dependence of ranolazine's actions were determined by recording transmembrane action potentials at basic pacing cycle lengths (BCL) of 300, 500, 800, 1000, 2000, 5000 msec. Data recorded at BCLs of 500 and 2000 msec are presented.
The following action potential parameters were measured:
1) action potential duration at 50% and 90% repolarization.
2) Amplitude
3) Overshoot
4) Resting membrane potential
5) Rate of rise of the upstroke of the action potential (Vₘₐₓ)

Vₘₐₓ was recorded under control conditions and in the presence of 10 and 100 µM of ranolazine. Vₘₐₓ was measured at a BCL of 500 msec. Because low extracellular K⁺ is known to promote drug-induced APD prolongation and early afterdepolarization, two separate sets of experiments were performed, one at normal [K⁺]ₒ (4 mM) and the other with low [K⁺]ₒ (2 mM).

### 3. Action Potential Studies in Arterially-Perfused Canine Left Ventricular Wedge Preparations

Transmural left ventricular wedges with dimensions of approximately 12 mm x 35 mm x 12 mm were dissected from the mid-to-basal anterior region of the left ventricular wall and a diagonal branch of the left anterior descending coronary artery was cannulated to deliver the perfusate (Tyrode's solution). The composition of the Tyrode's solution was (in mM): NaCl 129, KCl 4, NaH₂PO₄ 0.9, NaHCO₃ 20, CaCl₂ 1.8, MgSO₄ 0.5, and D-glucose 5.5; pH=7.4. A separate set of experiments were performed using Tyrode's solution containing 2 mM KCl.

Transmembrane action potentials were recorded from epicardial (EPI) and Subendocardial regions (M) using floating microelectrodes. A transmural pseudo-electrocardiogram (ECG) was recorded using two K-Agar electrodes (1.1 mm, i.d.) placed at approx. 1 cm. from the epicardial (+) and endocardial(-) surfaces of the preparation and along the same axis as the transmembrane recordings.

Ventricular wedges were allowed to equilibrate in the chamber for 2 hrs while paced at basic cycle lengths of 2000 msec using silver bipolar electrodes contacting the endocardial surface. A constant flow rate was set before ischemia to reach a perfusion pressure of 40-50 mmHg. The temperature was maintained at 37±0.5 °C by heating the perfusate and a contiguous water-chamber that surrounded the tissue-chamber with the same heater/circulating bath. The top-uncovered part of the tissue-chamber was covered in each experiment to ~75% of its surface with plastic sheets to further prevent heat loss; the remainder 25% was kept uncovered to position and maneuver the ECG electrodes and the floating microelectrodes. The preparations were fully immersed in the extracellular solution throughout the course of the experiment.

The QT interval was defined as the time interval between the initial deflection of the QRS complex and the point at which a tangent drawn to the steepest portion of the terminal part of the T wave crossed the isoelectric line.

### B. Study Protocols

Experimental Series 1: To determine the changes in repolarization time (action potential duration at 50 and 90% repolarization [APD₅₀ and APD₉₀, respectively] and QT interval [ECG]) as well as the vulnerability of the tissues to arrhythmogenesis after perfusing the preparations with ranolazine at concentrations ranging from 1 to 100 µM. [K+]ₒ= 4 mM.
   Transmembrane action potentials were recorded from epicardial (Epi), subendocardial regions (M region) using glass floating microelectrodes. A transmural ECG was recorded concurrently.
   a. Steady-state stimulation: Basic cycle length (BCL) was varied from 300 to 2000 msec to examine the rate-dependent changes in repolarization time (APD and ECG) at the following concentrations of ranolazine: 1, 5, 10, 50 and 100 µM.
   b. Programmed electrical stimulation (PES): Premature stimulation was applied to the epicardial surface before and after each concentration of drug in an attempt to induce arrhythmias. Single pulses (S2) were delivered once after every fifth or tenth basic beat (S1) at cycle lengths of 2000 msec. The S1-S2 coupling interval was progressively reduced until refractoriness was encountered (S2 stimuli were of 2-3 msec duration with an intensity equal to 3-5 times the diastolic threshold).
Experimental Series 2: To determine the changes in repolarization time (action potential duration at 50 and 90% repolarization [APD₅₀ and APD₉₀, respectively] and QT interval [ECG]) as well as the vulnerability of the preparation to arrhythmogenesis after perfusing the preparations with ranolazine at concentrations ranging from 1 to 100 µM. [K⁺]ₒ = 2 mM.
   a. Steady-state stimulation: Performed at basic cycle lengths (BCL) of 500 and 2000.
   b. Programmed electrical stimulation-(PES): See above.

Drugs: Ranolazine dihydrochloride was diluted in 100% distilled water as a stock solution of 50 mM. The drug was prepared fresh for each experiment.

Statistics: Statistical analysis was performed using one way repeated measures analysis of variance (ANOVA) followed by Bonferroni's test.

### EXAMPLE 11.

### Effect of Ranolazine on I_{Kr}, I_{Ks} and I_{K1}

Ranolazine inhibited I_{Kr} and I_{Ks} in a concentration-dependent manner, but did not alter I_{K1} . I_{Kr} was measured as the time-dependent tail current at - 40 mV, after a 250 msec activating pulse to 30 mV. Figure 3A shows currents recorded in control solution and after 50 µM ranolazine. I_{Kr} was almost completely blocked by this concentration of ranolazine. Figure 3B shows the concentration-response relationship for inhibition of I_{Kr} tail current, with an IC₅₀ of 11.5 µM.

I_{Ks} was elicited by a 3 sec step to +40 mV and measured as the peak time-dependent tail current recorded after stepping back to 0 mV. Shown in Figure 4A are currents recorded under control conditions, after 100 µM ranolazine, and after washout of the drug. Ranolazine (100 µM) largely eliminated the tail current recorded at 0 mV and this effect was completely reversed upon washout The concentration-response relationship for inhibition of I_{Ks} tail current is illustrated in Figure 4B, indicating an IC₅₀ of 13.4 µM.

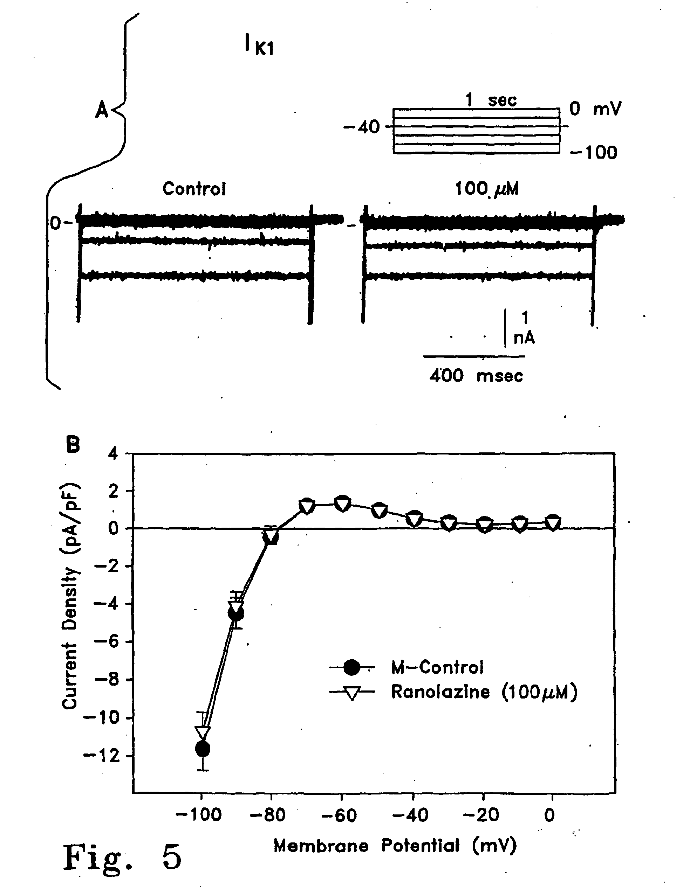

The inward rectifier, I_{K1} was recorded using perforated-patch voltage clamp techniques. Figure 5A shows I_{K1} recorded at voltages between -100 and 0 mV, incremented in 10 mV steps, under control conditions (left panel) and in the presence of 100 µM ranolazine. In this and five similar experiments, ranolazine produced no change in the inward rectifier current Panel B plots composite data illustrating the current-voltage relations constructed from the average current measured at the end of each test pulse

### EXAMPLE 12

### Action Potential Studies in Isolated Canine Ventricular Tissues

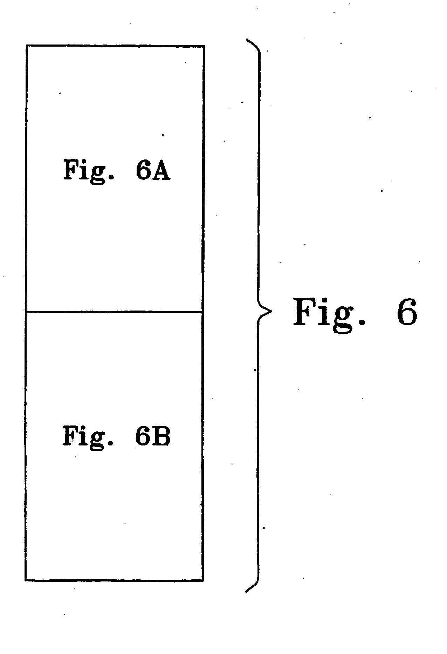

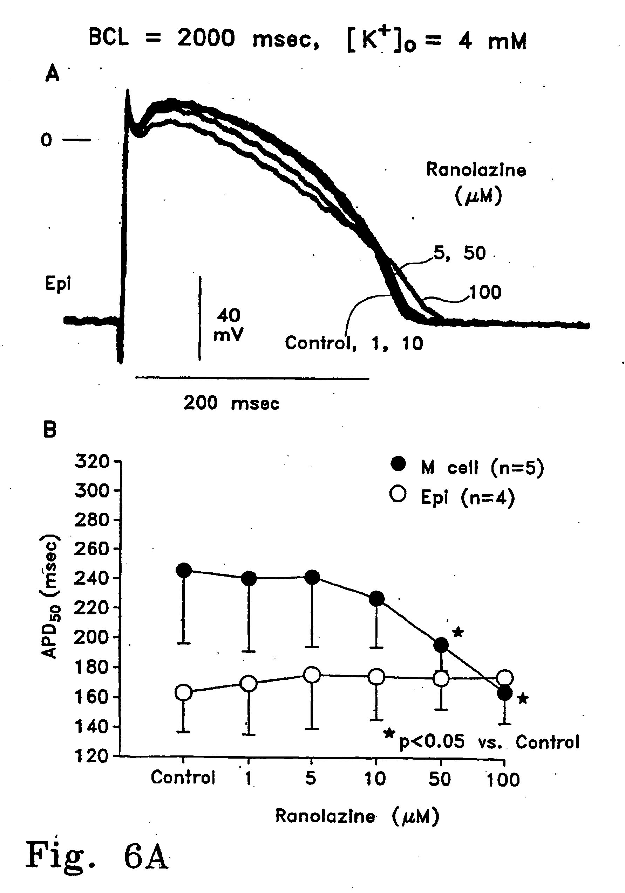

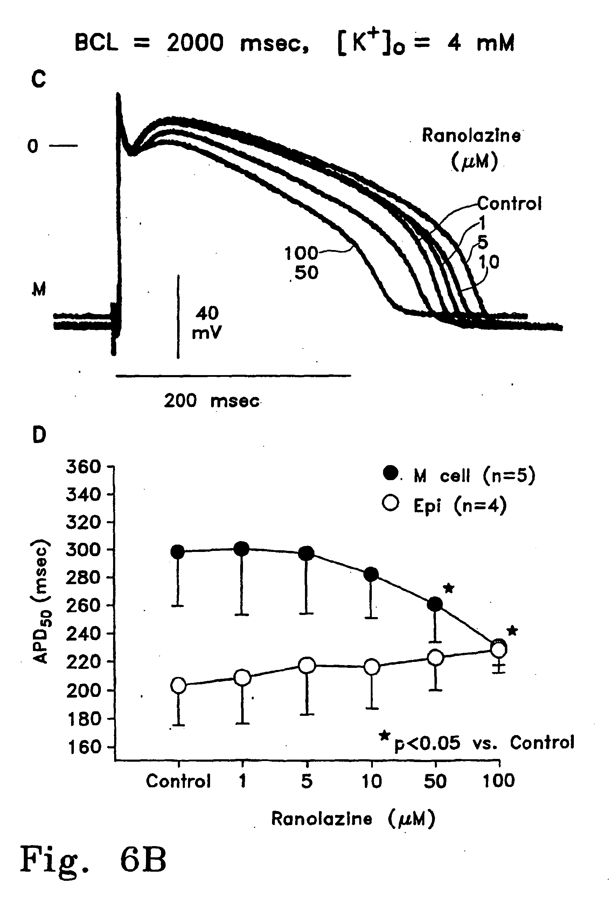

Ranolazine produced a concentration-dependent abbreviation of both APD₅₀ and APD₉₀ in M cell preparations at a [K⁺]o = 4 mM and BCL=2000 msec (Fig. 6). In some preparations, ranolazine produces a biphasic effect, prolonging APD at low concentrations and abbreviating APD at high concentrations (Fig 4A). Epicardial repolarization was less affected by the drug, showing a tendency towards APD prolongation. Transmural dispersion of repolarization was reduced at moderate concentrations of ranolazine and practically eliminated at higher concentrations.

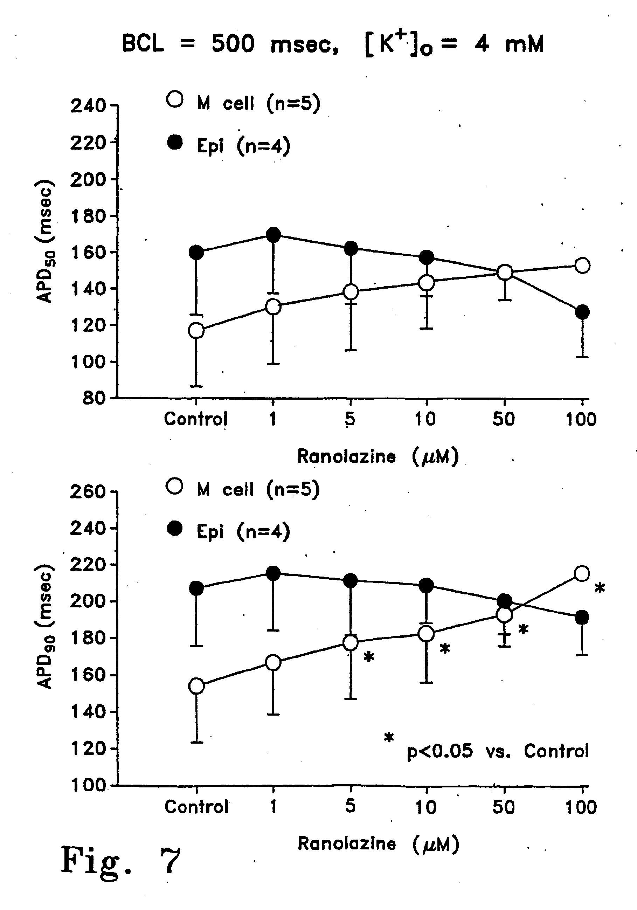

At a BCL of 500 msec, ranolazine caused a concentration-dependent prolongation of APD in epicardial tissues and abbreviation in M cell preparations. At a concentration of 100 µM, epicardial APD exceeded that of the M cell. As a result, transmural dispersion of repolarization was reduced or eliminated. At the highest concentration of ranolazine (100 µM), the transmural repolarization gradient reversed. It is noteworthy that ranolazine induced a use-dependent prolongation of APD₉₀ in epicardial preparations, i.e., prolongation was greater at faster rates (Figures 6 and 7).

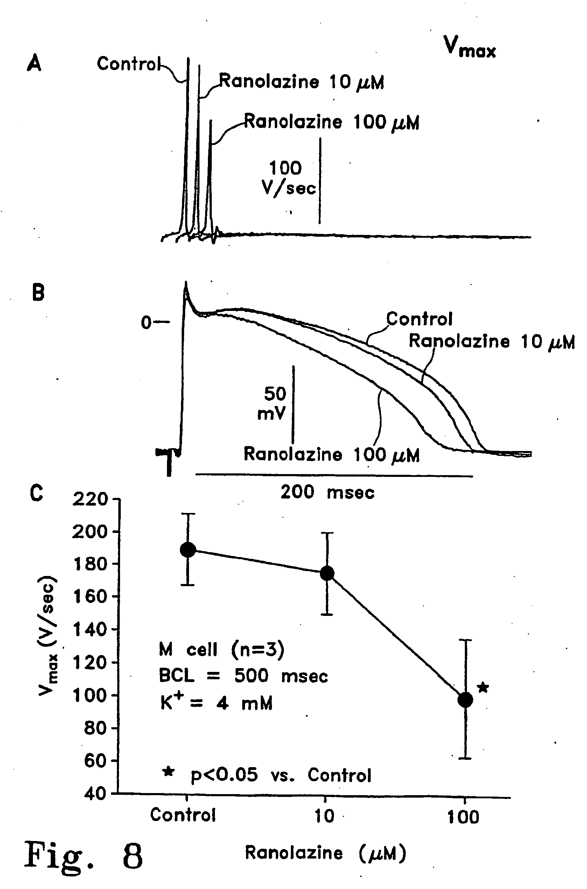

To assess ranolazine actions on I_{Na}, the rate of rise of the upstroke of the action potential (Vₘₐₓ) was measured. Ranolazine caused a reduction of Vₘₐₓ. This effect was modest (n.s.) at 10 µM, but more substantial with 100 µM ranolazine (Figure 8).

At concentrations of up to 50 µM, ranolazine produced little to no effect on amplitude, overshoot, and resting membrane potential in M cell preparations (Table 4).

**Table 4**

| Ranolazine (in µM) BCL = 500 msec. | | | | | | |
|---|---|---|---|---|---|---|
| | Control | 1.0 | 5.0 | 10.0 | 50.0 | 100.0 |
| Amplitude | 107±14 | 109±9 | 114±8 | 113±9 | 104±7 | 91±19* |
| RMP | -86±5 | -86±3 | -86±3 | -86±2 | -86±5 | -86±7 |
| Overshoot | 21±13 | 23±10 | 27±7 | 25±8 | 19±3 | 9±13 |

| | | | | | | |
|---|---|---|---|---|---|---|
| Data are expressed as mean ±SD, n=5 for all, * -p<0.05 vs. control | | | | | | |

At the highest dose tested (100 µM), ranolazine caused a decrease in phase 0 amplitude. Overshoot of the action potential as well as a resting membrane potential were reduced, although these did not reach statistical significance.

In epicardial preparations, ranolazine produced little to no change in resting membrane potential, overshoot and phase 0 amplitude (Table 5).

**Table 5**

| Ranolazine (in µM) BCL = 500 msec. | | | | | | |
|---|---|---|---|---|---|---|
| | Control | 1.0 | 5.0 | 10.0 | 50.0 | 100.0 |
| Amplitude | 95±3 | 93±5 | 101±2 | 94±5 | 86±12 | 93±3 |
| RMP | -84±3 | -84±4 | -89±1 | -88±2 | -86±1 | -85±3 |
| Overshoot | 11±2 | 10±4 | 12±3 | 8±4 | 0±11 | 8±4 |

| | | | | | | |
|---|---|---|---|---|---|---|
| Data are expressed as meantSD, n=4 for all but 100.0 µM ranolazine (n=2). In the remained two epicardial preparations, 100.00 µM ranolazine produced an excessive APD prolongation, resulting to repolarization altemans and/or 2:1 responses. | | | | | | |

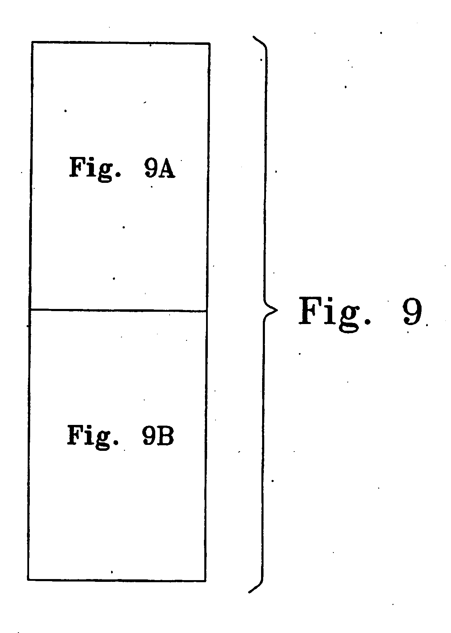

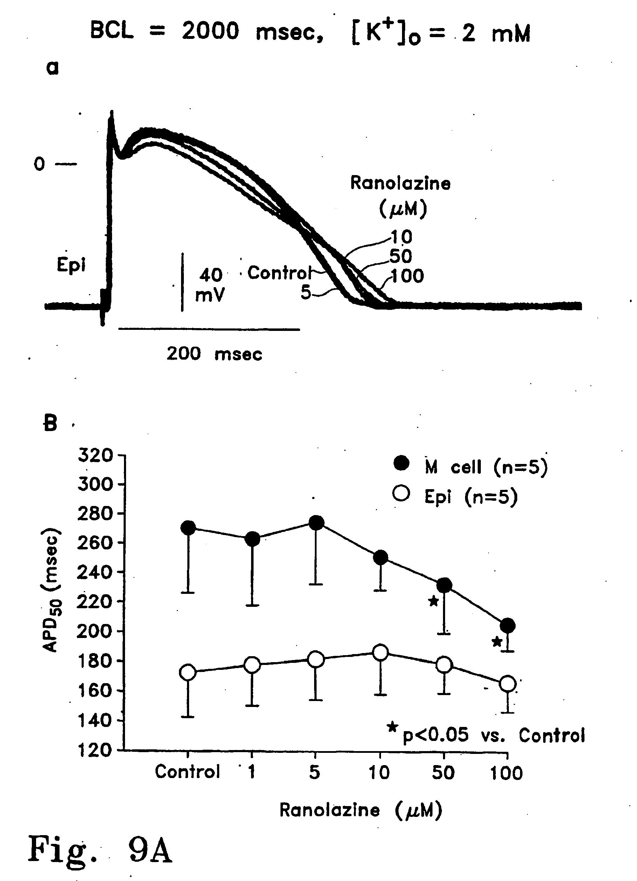

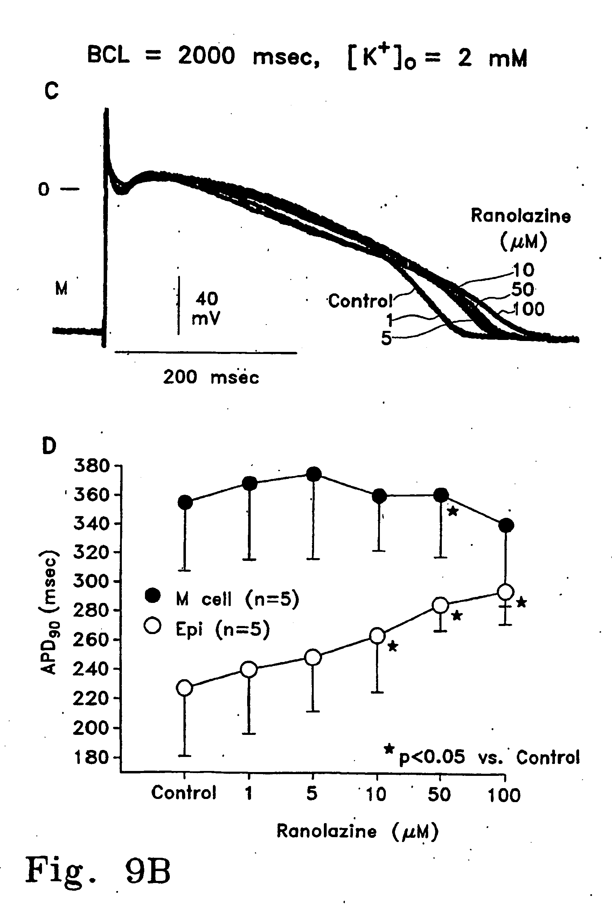

In the presence of low [K⁺]ₒ and slow rates ( BCL=2000 msec), ranolazine caused no significant change in APD₉₀ of the M cell, but a concentration-dependent abbreviation of APD₅₀ (Figure 9). In contrast, in epicardium the drug produced little change in APD₅₀, but a concentration-dependent prolongation of APD₉₀. Transmural dispersion of repolarization was importantly diminished.

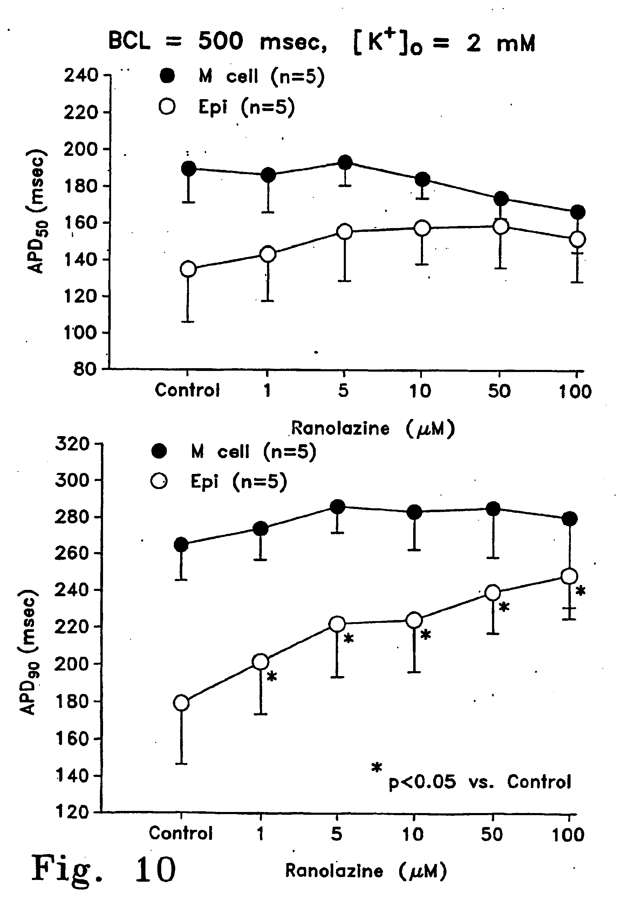

At a BCL of 500 msec, ranolazine caused little change in repolarization of the M cell, but a prominent concentration-dependent prolongation of APD₉₀ in epicardium (Figure 10).

### EXAMPLE 13.

### Action Potential Studies in Arterially-Perfused Canine Left Ventricular Wedge

### Preparations

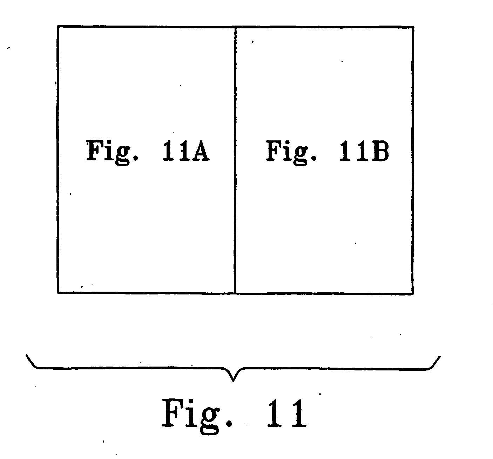

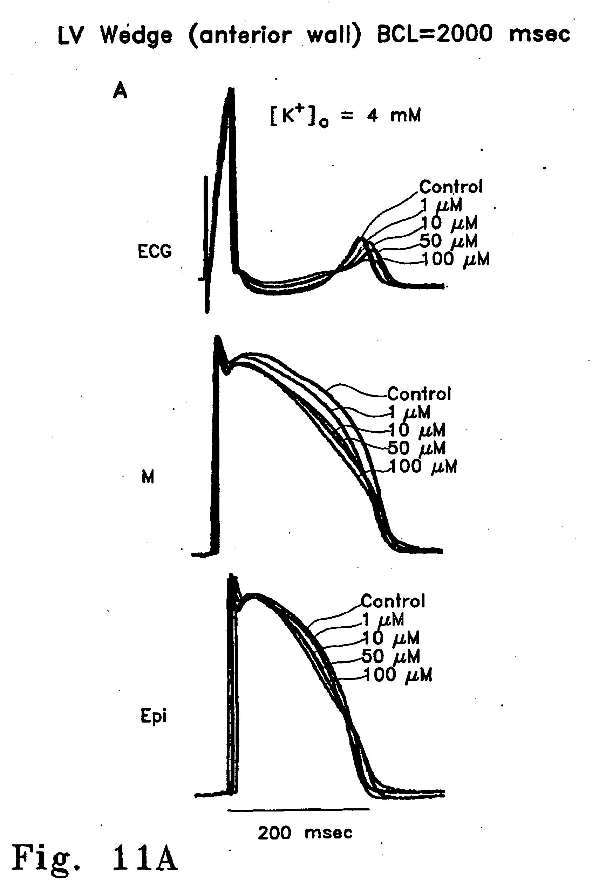

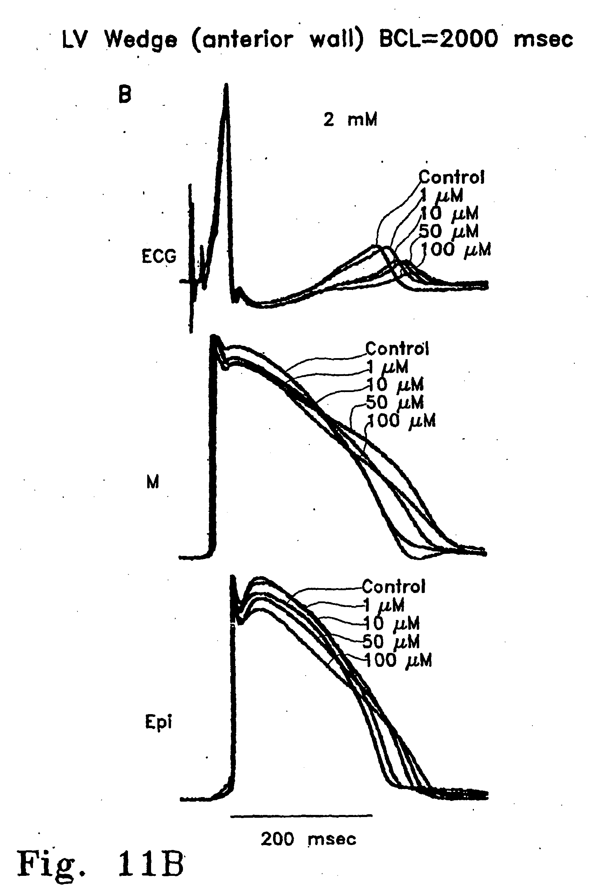

Each panel in Figure 11 shows an ECG and transmembrane action potentials recorded from the midmyocardium (M region) and epicardium (Epi) of the arterially perfused canine left ventricular wedge preparation at a basic cycle length (BCL) of 2000 msec in the absence and presence of ranolazine (1-100 µM). The effects of the drug were studied with coronary perfusate containing either 4 mM (left panels) or 2 mM (right panels) KC1.

In the presence of 4 mM KCl, ranolazine did not significantly alter APD₉₀, but significantly reduced APD₅₀ at high concentrations of the drug (50 and 100 µM). In contrast, in the presence of 2 mM KCl, ranolazine significantly prolonged APD₉₀ at concentrations of 5-100 µM, but did not significantly alter APD₅₀ at any concentration (Table 6).

Ranolazine prolonged APD₉₀ of epicardium more than that of M cells at [K⁺]ₒ of 4 mM. As a consequence, transmural dispersion of repolarization was reduced, although this did not reach significance. At a [K⁺]ₒ of 2 mM, ranolazine prolonged APD₉₀ of M cells more than those of epicardium, resulting in an increase in transmural dispersion of repolarization, which also failed to reach significance (Table 7).

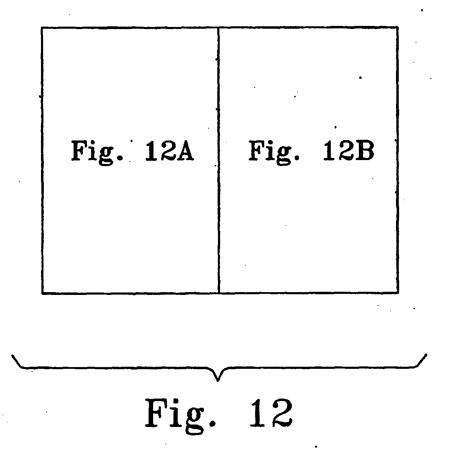

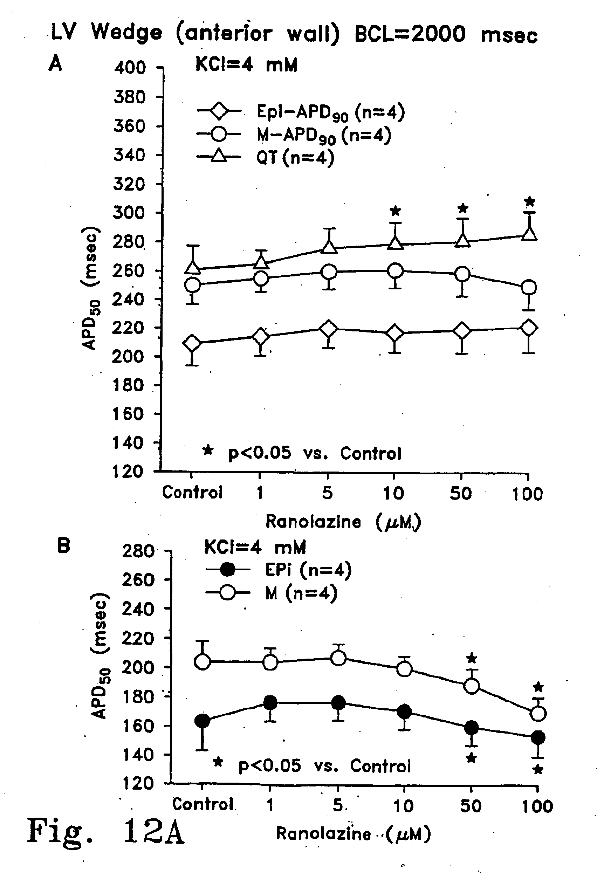

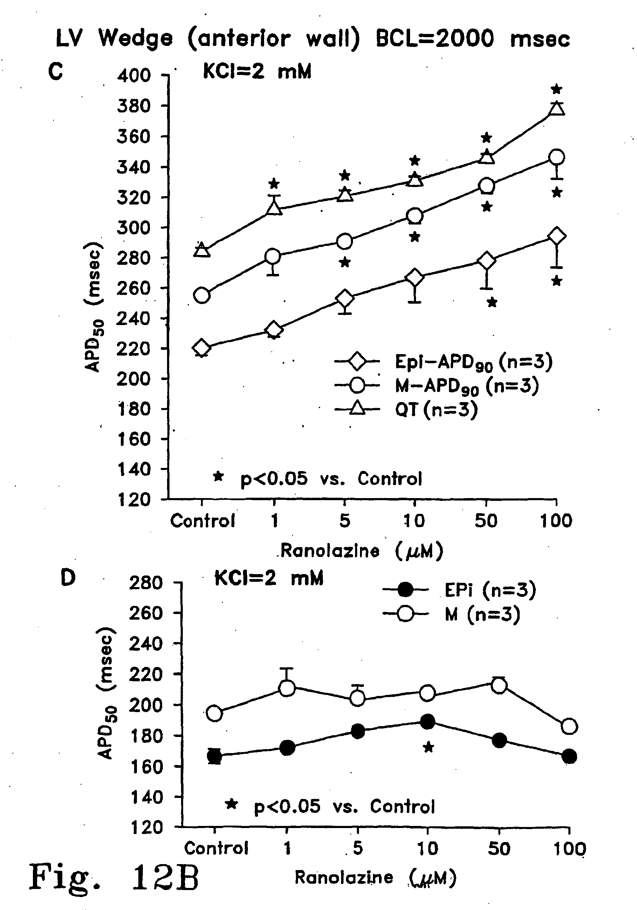

Figure 12 shows composite data of the concentration-dependent effect of ranolazine on APD₉₀ and QT interval (top panels) and on APD₅₀ (bottom panels). With a [K⁺]ₒ of 4 mM, QT and APD₉₀ were little affected at any drug concentration; APD₅₀ significantly abbreviated at 50 and 100 µM concentrations. With a [K⁺]ₒ of 2 mM, QT and APD₉₀ of the M cell prolonged at ranolazine concentrations greater than 5 µM slightly, whereas APD₅₀ was little affected.

**Table 6**

| Canine left Ventricular Wedge: 4 mM (KCl]₀, BCL=2000 | | | | | | | |
|---|---|---|---|---|---|---|---|
| | Epicardium | | M region | | | | |
| Concentration | APD50±SE | APD90±SB | APD50±SE | APD90±SE | QT_{end} | Tₚₑₐₖ - T_{end} | TDR |
| Control | 164±21 | 209.3±15.76 | 204.5±13.9 | 250±13.93 | 261.1±15.76 | 3.25±2.56 | 43±6 |
| 1 µM | 176.3±12.25 | 213.8±13.28 | 203.3±9.621 | 254.3±9.15 | 263.5±10.56 | 34.5±3.202 | 26.75±8.045 |
| 5µM | 176.5±11.85 | 219±12.12 | 207.5±8.627 | 258.3±11.08 | 274.5±13.73 | 37.75±4.09 | 36±2.449 |
| 10µM | 170.5±12.03 | 216.5±13.41 | 199±9.083 | 260.3±12.66 | 277.8±14.99* | 39.25±5.54 | 30.75±10.46 |
| 50 µM | 159±12.82* | 218±15.91 | 1 87.8±11.21* | 257.5±15.47 | 279.3±17.21* | 41.25±8.37 | 32.5±6.278 |
| 100µM | 152.5±14.44* | 220.5±18.26 | 169±10.5* | 247.8±15.32 | 284.5±14.39* | 40.5±4.94 | 23.75±2.689 |

| | | | | | | | |
|---|---|---|---|---|---|---|---|
| *p<0.05 vs. control n≤4 | | | | | | | |

**Table 7**

| Canine Left Ventricular Wedge: 2 mM [KCl]₀, BCL=2000 | | | | | | | |
|---|---|---|---|---|---|---|---|
| | Epicardium | | M region | | | | |
| Concentration | APD50±SE | APD90±SE | APD50±SE | APD90±SE | QT_{end} | Tₚₑₐₖ- T_{end} | TDR |
| control | 167±5.548 | 220±5.568 | 195.3±3.283 | 254.3±0.882 | 283±2.08 | 24±12.57 | 16±9.238 |
| 1µM | 173±2 | 232±5.508 | 210.7±13.53 | 280.3±12.72 | 311±9.5 | 35±4.70 | 28.33±11.46 |
| 5 µM | 183.5±1.5 | 252.5±10.5 | 205.7±7.881 | 289.7±2.848* | 319±4.58 | 33±1.33 | 15±7 |
| 10 µM | 190±2* | 265.5±16.5 | 208.3±3.48 | 305.3±4.978* | 329±2.33 | 36±4.09 | 23.5±1.5 |
| 50 µM | 179±1 | 276.5±18,5* | 214.3±6.333 | 315.5±5.5* | 343±2.84 | 41±6.35 | 35.5±3.5 |
| 100 µM | 167.5±0.5 | 293.5±21.5* | 187.7±4.978 | 345±14.36* | 376±4.48 | 55±1.00 | 35±11 |

| | | | | | | | |
|---|---|---|---|---|---|---|---|
| *p<0.05 vs. control n≤4 | | | | | | | |

Table 8 highlights the fact that Torsade de Pointes arrhythmias are not observed to develop spontaneously, nor could they be induced by programmed electrical stimulation under any of the protocols involving the canine left ventricular wedge preparation. No arrhythmias were observed under control conditions or following any concentration of ranolazine.

**Table 8**

| Ranolazine-induced Torsade de Pointes | | |
|---|---|---|
| | Spontaneous | Stimulation-induced |
| Ranolazine (1-100 µM) | 0/4 | 0/4 |
| 4 mM [K⁺]₀ | | |
| Ranolazine (1-100 µM) | 0/3 | 0/3 |
| 2 mM [K⁺]₀ | | |

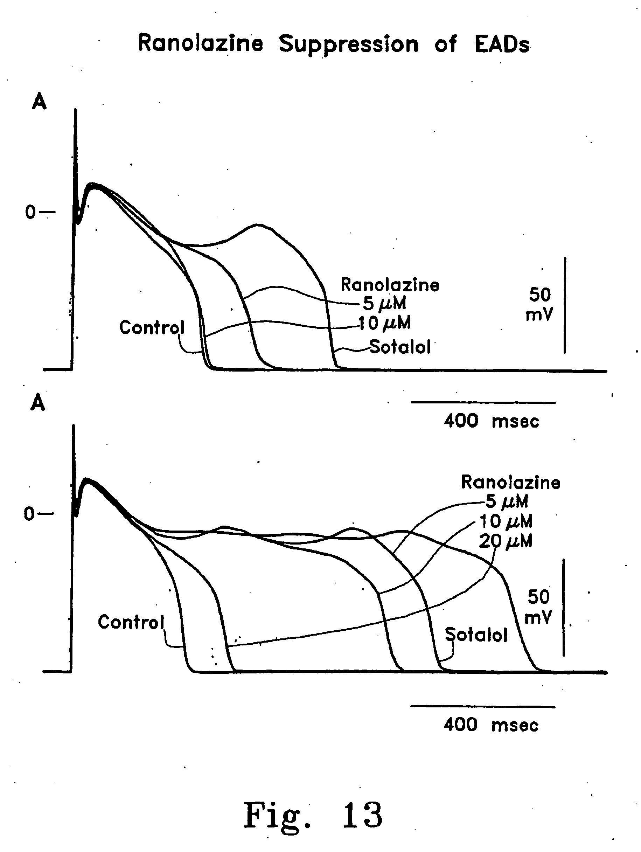

Neither early nor delayed afterdepolarizations were observed in either tissue or wedge preparations pretreated with any concentration of ranolazine. Indeed, ranolazine proved to be effective in suppressing EADs induced by exposure of M cell preparations to other I_{Kr} blockers such as d-sotalol, as illustrated in Figure 13. D-Sotalol produced a remarkable prolongation of repolarization and induced EADs in the M cell preparations. Ranolazine concentration-dependently abbreviated the action potential and abolished the EADs. A similar effect of ranolazine (5-20 µM) to suppress EAD activity and abbreviate APD was observed in 4/4 M cell preparations.

### EXAMPLE 14

### II. Electrophysicologic Effects of Ranolazine on Late I_{Na}, I_{Ca}, Iₜₒ and I _{Na-Ca} IN Isolated Canine Left Ventricular Myocytes.

### A. Materials and Methods

### 1. Voltage Clamp Studies in Isolated Canine Ventricular Mvocvtes

Adult male mongrel dogs were given 180 IU/kg heparin (sodium salt) and anesthetized with 35 mg/kg i.v. pentobarbital sodium, and their hearts were quickly removed and placed in Tyrode's solution. Single myocytes were obtained by enzymatic dissociation from a wedge-shaped section of the ventricular free wall supplied by the left circumflex coronary artery. Cells from the epicardial and midmyocardial regions of the left ventricle were used. All procedures were in accordance with guidelines established by the Institutional Animal Care and Use Committee.

Tyrode's solution used in the dissociation contained (mM): 135 NaCl, 5.4 KCl, 1 MgCl₂, 0 or 0.5 CaCl₂, 10 glucose, 0.33 NaH₂PO₄, 10 N-2-hydroxyethylpiperazine-N'-2-ethanesulfonic acid (HEPES), and pH was adjusted to 7.4 with NaOH.

L-type calcium current (I_{Ca}), transient outward current (Iₜₒ), and sodium-calcium exchange current (I_{Na-Ca}) were recorded at 37° C using standard patch electrodes. The composition of the external and pipette solutions is shown in Tables 9 and 10, respectively. Late I_{Na} was recorded using perforated patch techniques.

**Table 9**

| External Solutions | |
|---|---|
| I_{NaCa}, I_{Na}, late and I_{Ca} | Iₜₒ |
| Whole cell/Perf patch (mM) | Whole Cell (mM) |
| 10 glucose | 10 glucose |
| - | 4 KCl |
| 1 MgCl₂ | 1 MgCl₂ |
| 2 CaCl₂ | 2 CaCl₂ |
| 140 Na-methanesulfonate | 140 N-methyl-D-glucamine-Cl |
| 10 HEPES | 10 HEPES |
| pH 7.4 with methane sulfonic acid | pH 7.4 with HCl |

**Table 10**

| Internal Solutions | | | |
|---|---|---|---|
| I_{Na,late} | I_{Ca} | I_{NaCa} | Iₜₒ |
| Perf-patch (mM) | Whole cell (mM) | Whole cell (mM) | Whole cell (mM) |
| 135 Cs-aspartate | 140 Cs-aspartate | 140 Cs-aspartate | 130 K-aspartate |
| 0.010 CaCl₂ | - | - | 20 KCl |
| 10 NaOH | 10 NaOH | 10 NaOH | - |
| 1 MgCl₂ | 1MgCl₂ | 1MgCl₂ | 1 MgCl₂ |
| - | 5 MgATP | 5 MgATP | 5 MgATP |
| 10 HEPES | 10 HEPES | 10 HEPES | 10 HEPES |
| - | 10 EGTA | 0.1 EGTA | 5 EGTA |
| pH 7.1 with CsOH | pH 7.1 with CsOH | pH 7.1 with CsOH | pH 7.1 with KOH |

Dissociated cells were placed in a temperature controlled 0.5 ml chamber (Medical Systems, Greenvale, NY) on the stage of an inverted microscope and superfused at 2 ml/min. A ten-barrel quartz micro-manifold (ALA Scientific Instruments Inc., Westbury, NY) placed 100 µm from the cell was used to apply ranolazine, tetrodotoxin (TTX), or cadmium. An Axopatch 200A amplifier (Axon Instruments, Foster City, CA) was operated in voltage clamp mode to record currents at 3 7° C. Whole cell currents were filtered with a 4-pole low-pass Bessel filter at 5 kHz, digitized between 2 - 5 kHz (Digidata 1200A, Axon Instruments) and stored on a computer. pClamp 8.2 software (Axon Instruments) was used to record and analyze ionic currents. Pipette tip resistance was 1.0-1.5 MΩ and seal resistance was greater than 5 GΩ. Electronic compensation of series resistance averaged 76 %. Voltages reported were corrected for patch electrode tip potentials. The seal between cell membrane and patch pipette was initially formed in Tyrode's solution containing 1 mM CaCl₂. A 3 M KCl-agar bridge was used between the Ag/AgCl ground electrode and external solution to avoid development of a ground potential when switching to experimental solution.

Tetrodotoxin (TTX) was prepared in water and diluted 1:100 for a final concentration of 10 µM in external solution. Ranolazine was prepared in water at a concentration of 50 mM and diluted in external solution to final concentrations ranging from 1 - 800 µM.

I_{Ca} was defined as peak inward current minus the current at the end of the test pulse. External solution contained 10 µM TTX to block the steady state component of late I_{Na}. Cells were rested for 20 seconds at -90 mV before evoking an 800 ms ramp to -60 mV and a 15 ms step to -50 mV to inactivate sodium channels and maintain voltage control, immediately followed by a 500 ms step to 0 mV to record I_{Ca} in control solutions. This protocol was repeated 5 times at a rate of 0.5 Hz for each of the drug concentrations. The steady state effects of the Ranolazine were measured as the fractional change in I_{Ca} during the 5^{th} pulse of the train. Changes in I_{Ca} were plotted against drug concentration on a semi-log scale and fitted to a logistic equation.

Late I_{Na} was defined as the average TTX-sensitive current measured in the final 5 ms of the test pulse to -30 mV. The transient loss of voltage control that occurred at the beginning of the 500 ms pulse did not affect currents measured at the end of the pulse³. A train of 500 ms pulses repeated at a rate of 1 Hz was used to determine steady state block. Reduction of late INa during the 10th pulse was plotted as a function of drug concentration on a semi-log scale and fitted to a logistic equation.

Iₜₒ was recorded in the presence of 300 µM CdCl₂ to block I_{Ca}, and was defined as the peak outward current minus the steady state current at the end of the test pulse. Holding potential was -80 mV and a 5 ms pulse to -50 mV was taken before evoking 100 ms pulses to-10, 0, and 10 mV, which were repeated at a rate of 0.1 Hz. The effects ofranolazine were evaluated 4 min after addition of each drug concentration. Results were not plotted as a logistic function as ranolazine had a minimal effect on Iₜₒ. Instead, all results are presented as means ± standard error. A two-tailed Student's t-test was used to determine differences among means.

To trigger I_{Na-Ca} by means of the normal calcium transient, a 3-ms pulse to -50 mV was followed by a 5 ms step to 0 mV to activate I_{Ca}. and a calcium transient This two step protocol was immediately followed by a pulse to -80 mV to record I_{Na-Ca}. I_{Na-Ca} was quantified as total charge transported (pA x ms). Voltage clamp protocols were preceded by a train of ten pulses to 20 mV delivered at a rate of 0.5 Hz followed by a rest of 6 sec to maintain calcium loading of the SR. Reduction of I_{Na-Ca} was plotted as a function of drug concentration on a semi-log scale and fitted to a logistic equation.

### EXAMPLE 15

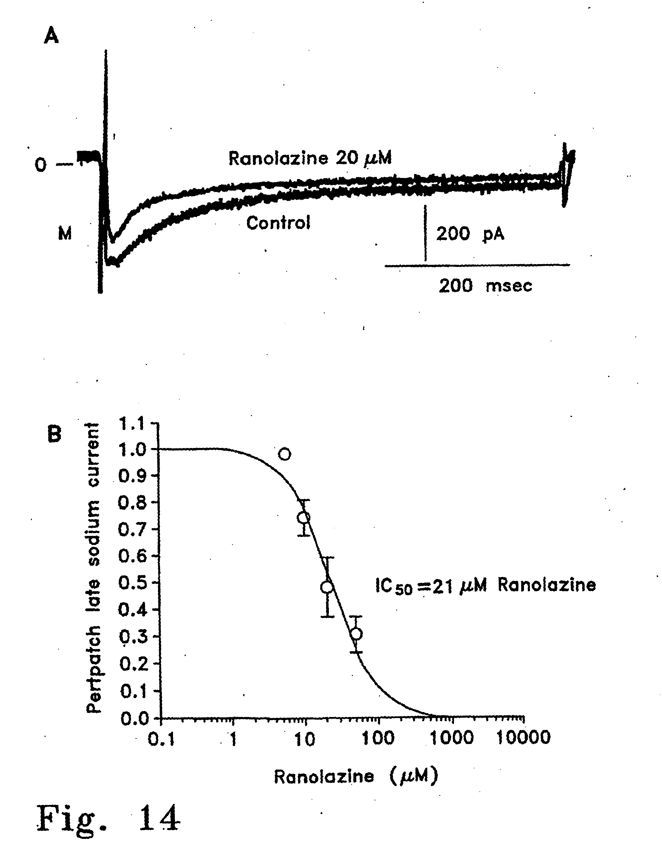

Figure 14A shows TTX-sensitive currents in control solution and 4 min after addition of 20 µM ranolazine to the external solution. Figure 14B shows the summary results of similar experiments in which ranolazine (5 - 50 µM) was added to the external solution. Half-inhibition of late I_{Na} occurred at a drug concentration of 21 µM.

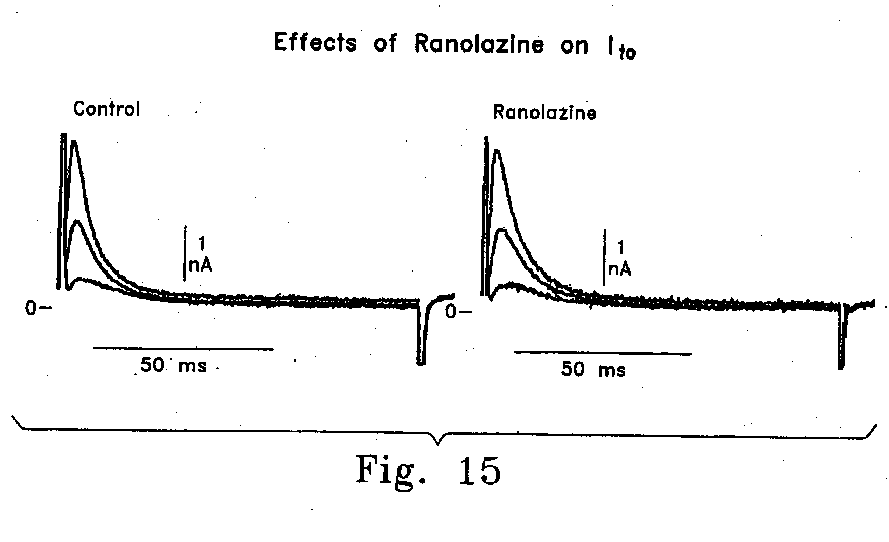

The effect of Ranolazine on Iₜₒ was determined at test potentials of -10, 0, and 10 mV. Iₜₒ was quite resistant to inhibition by ranolazine. Figure 15 shows currents recorded in control solution (left panel) and 4 min after addition of 50 µM ranolazine. The drug reduced peak Iₜₒ by less than 10

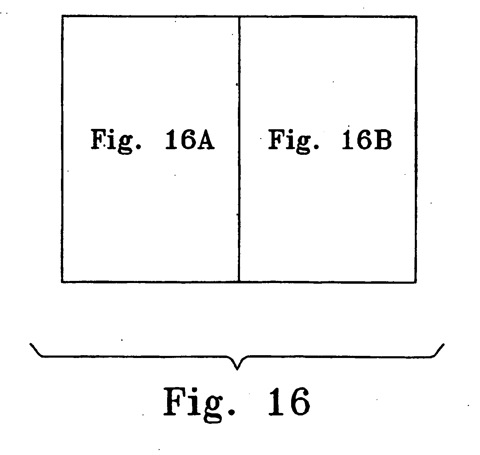

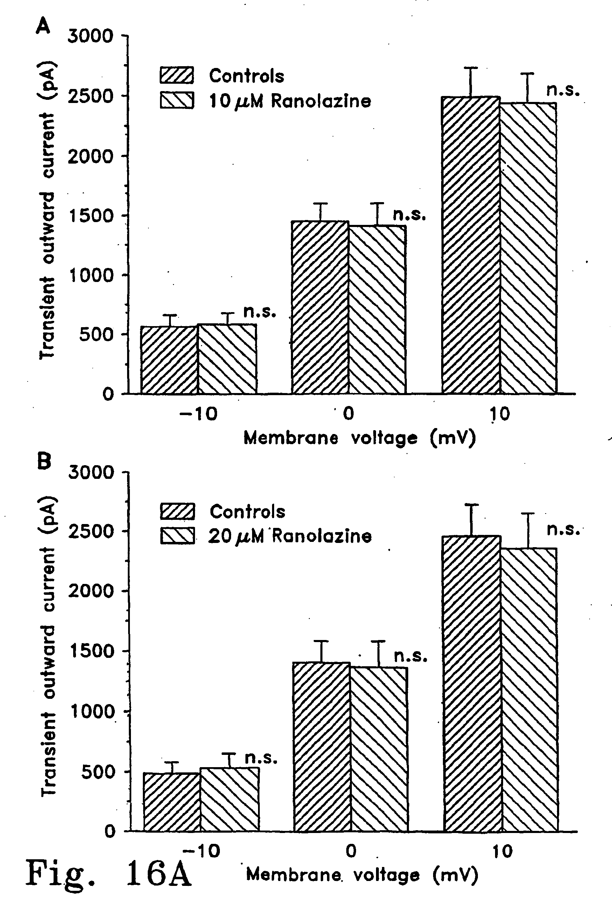

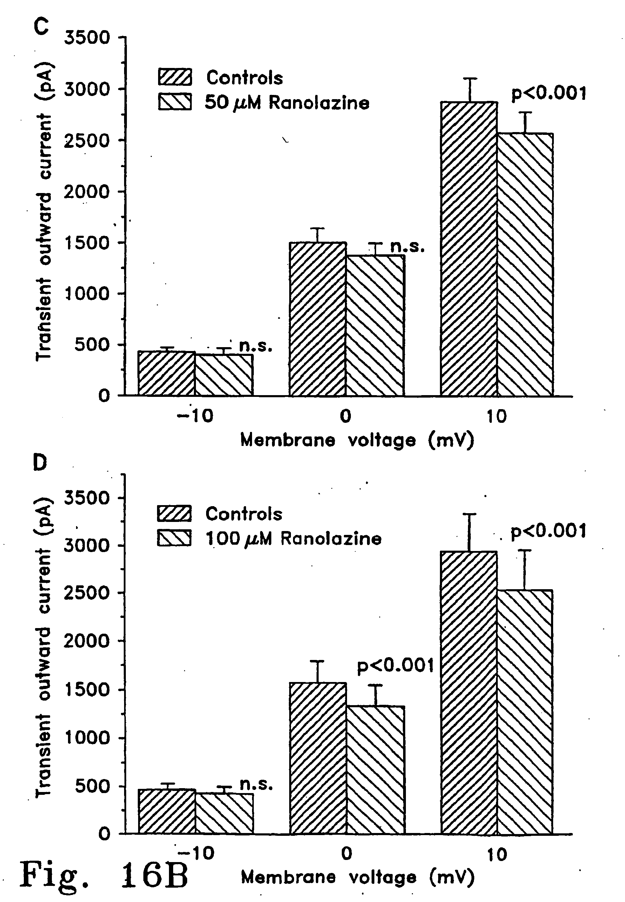

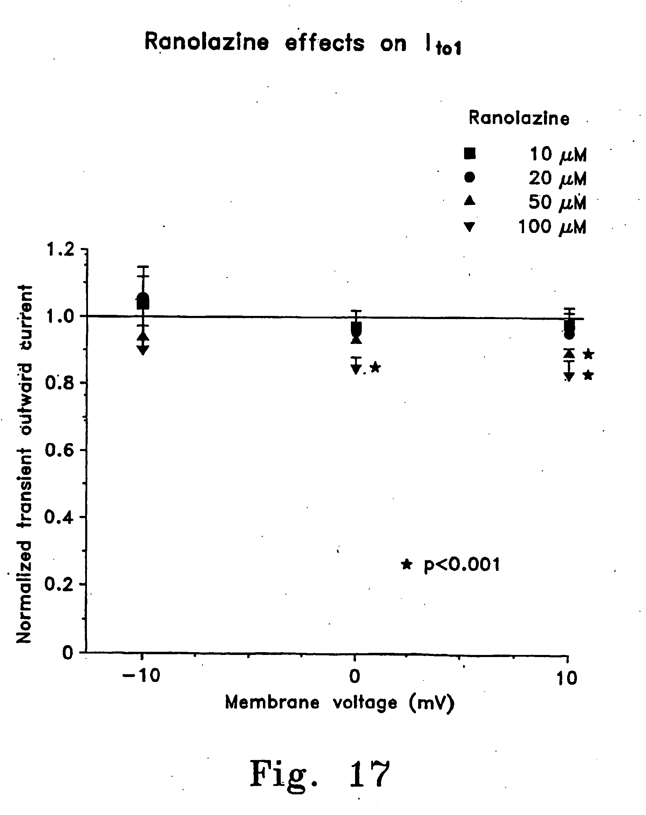

Ranolazine at a concentration of 50 µM reduced Iₜₒ by 10 ± 2 % at 10 mV (6 cells, p < 0.001). The effects of ranolazine at-10 and 0 mV did not reach significance. Ranolazine at a concentration of 100 µM reduced Itₒ by 16 ± 3 % and 17 ± 4 % at test potentials of 0 and 10 mV, respectively (7 cells, p < 0.001). Ranolazine had no effect at concentrations of 10 µM (9 cells) and 20 µM (9 cells) at any of the test voltages. Results presented in Figure 16 were normalized to each control current and summarized in Figure 17.

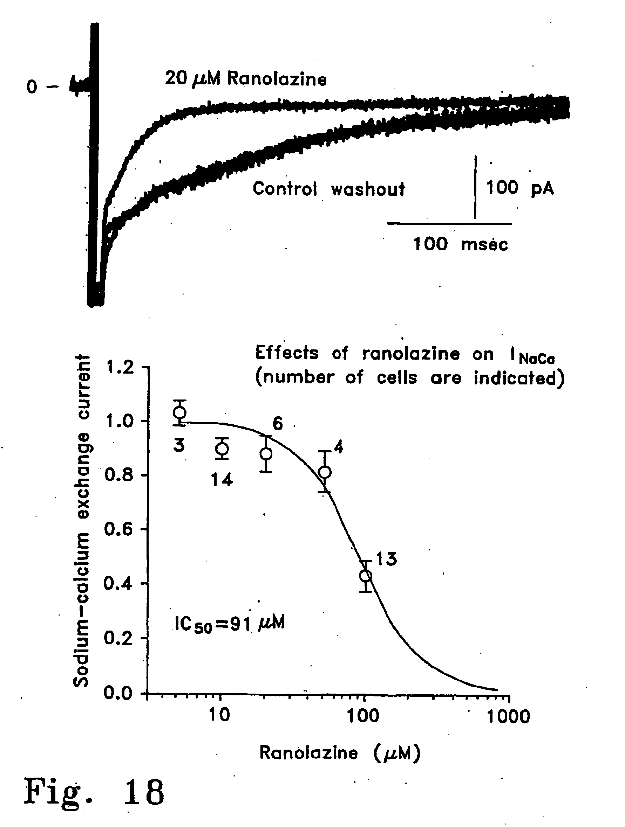

The top panel of Figure 18 shows superimposed traces of I_{Na-Ca} in control solution, 4 min after addition of 100 µM ranolazine, and after returning to the control solution. The lower panel of Figure 18 shows the concentration-response curve obtained from 3-14 cells. The IC₅₀ for ranolazine inhibit I_{Na-Ca} is 91 µM.

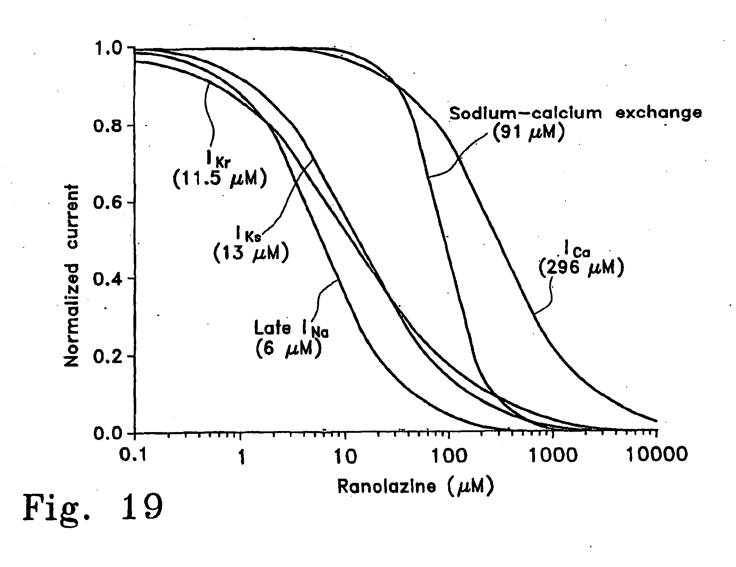

Figure 19 shows the concentration-response curves for I_{Kr}, I_{Ks}, I_{Ca}, late I_{Na}, and I_{Na}-Cₐ in a single plot Inhibition of Itₒ at the highest concentration tested (100 µM) was insufficient to develop a complete curve. I_{Kr,} I_{Ks}, and late I_{Na} showed similar sensitivities to ranolazine.

### EXAMPLE 16

### III. Electrophysiological Effects of Ranolazine in Isolated Canine Purkinje Fibers.

### A. Material and Methods.

Dogs weighing 20-25 kg were anticoagulated with heparin and anesthetized with pentobarbital (30-35 mg/kg, i.v.). The chest was opened via a left thoracotomy, the heart excised and placed in a cold cardioplegic solution ([K⁺]₀=8 mmol/L, 4°C). Free running Purkinje fibers were isolated from the left and right ventricles. The preparations were placed in a tissue bath (5 ml volume with flow rate of 12 ml/min) and allowed to equilibrate for at least 30 min while superfused with an oxygenated Tyrode's solution (pH=7.35, t° =37 ± 0.5°C) and paced at a basic cycle length (BCL) of 1 Hz using point stimulation. The composition of the Tyrode's solution was as following (in mM): NaCl 129, KCl 4, NaH₂PO₄ 0.9, NaHCO₃ 20, CaCl₂ 1.8, MgSO₄ 0.5, and D-glucose 5.5.

Action potential recordings: Transmembrane potentials were recorded using standard glass microelectrodes filled with 2.7 M KCl (10 to 20 MΩ DC resistance) connected to a high input-impedance amplification system (World Precision Instruments, Sarasota, FL, USA). Amplified signals were displayed on Tektronix (Beaverton, OR, USA) oscilloscopes and amplified (model 1903-4 programmable amplifiers [Cambridge Electronic Designs (C.E.D.), Cambridge, England]), digitized (model 1401 AD/DA system [C.E.D.]), analyzed (Spike 2 acquisition and analysis module [C.E.D.], and stored on magnetic media (personal computer).

### B. Study protocols.

Control recordings were obtained after a 30 min equilibration period. Increasing concentrations of ranolazine (1, 5, 10, 50, and 100 µM) were evaluated, with recordings started 20 minutes after the addition of each concentration of the drug. The rate-dependence of ranolazine's actions were evaluated by recording action potentials at basic cycle lengths (BCL) of 300, 500, 800, 1000, 2000, and 5000 msec. In this report only BCLs of 500 and 2000 msec are presented as representative of relatively rapid and slow pacing rates.
The following action potential parameters were measured:
a. Action potential duration at 50% (APD₅₀) and 90% (APD₉₀) repolarization.
b. Amplitude
c. Overshoot
d. Resting membrane potential
e. Rate of rise of the upstroke of the action potential (Vₘₐₓ).

Because low extracellular K⁺ is known to promote drug-induced APD prolongation and early afterdepolarizations, we determined the effects of ranolazine in the presence of normal (4 mM) and low (3 mM) [K⁺]ₒ. In the final phase, we evaluate the effects of ranolazine on EADs induced by d-sotalol (100 µM), a fairly specific I_{Kr} blocker.

Ranolazine dihydrochloride was diluted in distilled water to make a stock solution of 50 mM. The drug was freshly prepared for each experiment. Statistics. Statistical analysis was performed using one way repeated measures analysis of variance (ANOVA) followed by Bonferroni's test.

### EXAMPLE 17

### Normal concentration of extracellular K⁺ (4 mM)

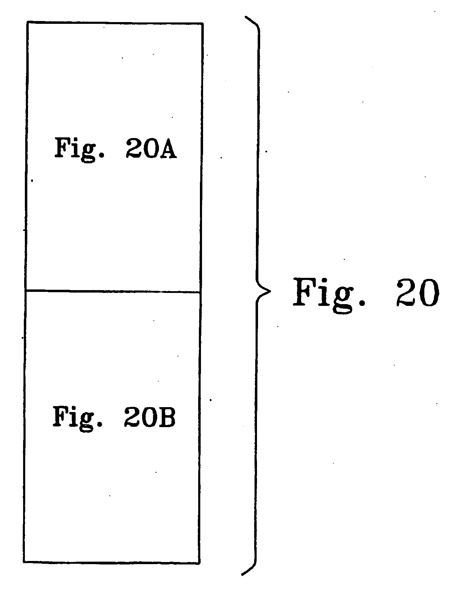

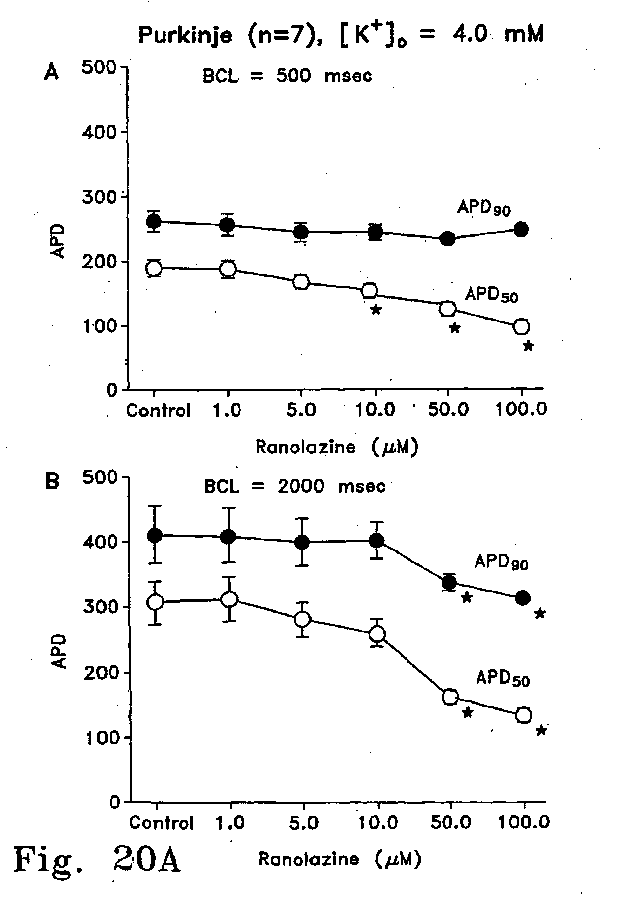

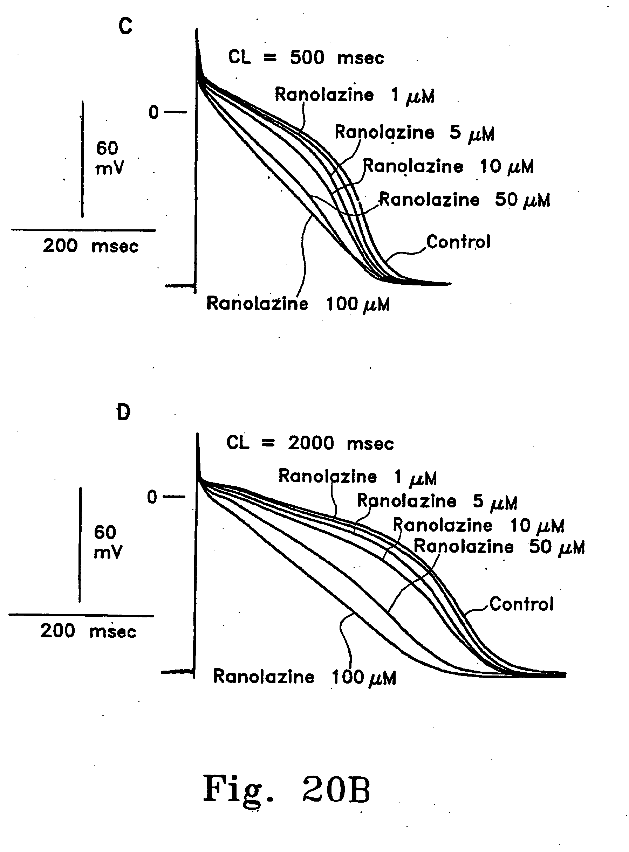

Ranolazine (1-100 µM) produced concentration- and rate-dependent effects on repolarization in Purkinje fibers (Fig. 20). Low concentrations of ranolazine (1-10 µM) produced either no effect or a relatively small abbreviation of APD. High concentrations of ranolazine (50 and 100 µM) significantly abbreviated APD₅₀ at both rapid and slow rates. In contrast, APD₉₀ was markedly abbreviated at slow, but not at rapid pacing rates (Fig .20). No sign of an EAD was observed at any concentration of the drug.

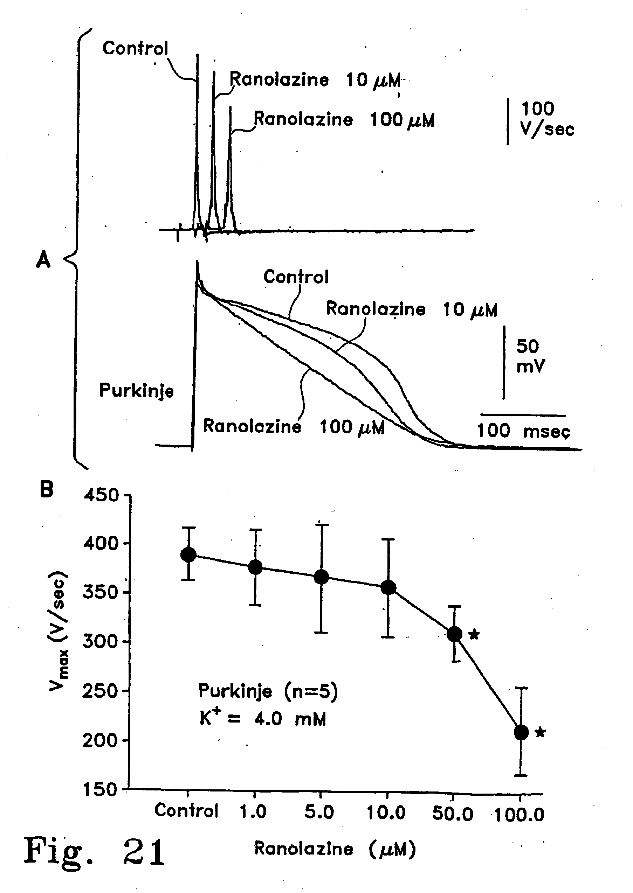

To assess the effect of ranolazine on I_{Na}, we determined the effect of the drug on the rate of rise of the upstroke of the action potential (Vₘₐₓ). Ranolazine caused a significant reduction of Vₘₐₓ at concentrations of 50 and 100 µM (Fig. 21), indicating inhibition of I_{Na} by the drug.

Ranolazine, in concentrations of 1-50 µM, produced little to no effect on the amplitude, overshoot, or resting membrane potential (Table 11).

**Table 11**

| Effects of Ranolazine on phase 0 amplitude, resting membrane Potential (RMP), and overshoot of action potential in Purkinje fibers In the presence of normal [K⁺]₀ Ranolazine (in µM) | | | | | | |
|---|---|---|---|---|---|---|
| | Control | 1.0 | 5.0 | 10.0 | 50.0 | 100.0 |
| Amplitude | 122±5 | 120±9 | 124±3 | 122±7 | 117±7 | 106±12* |
| RMP | -91±1 | -90±2 | -90±2 | -90±3 | -89±3 | -87±3* |
| Overshoot | 32±4 | 32±7 | 34±7 | 32±6 | 28±7 | 19±11 * |

| | | | | | | |
|---|---|---|---|---|---|---|
| [K⁺]₀ = 4.0 mM; BCL = 500 msec Data are expressed as mean ±SD, n=7, *p<0.05 vs. control | | | | | | |

At the highest concentration tested (100 µM), ranolazine caused a statistically significant reduction of phase 0 amplitude and overshoot, consistent with the effect of the drug to reduce Vₘₐₓ and I_{Na}.

### Low concentration of extracellular K⁺ (3 mM)

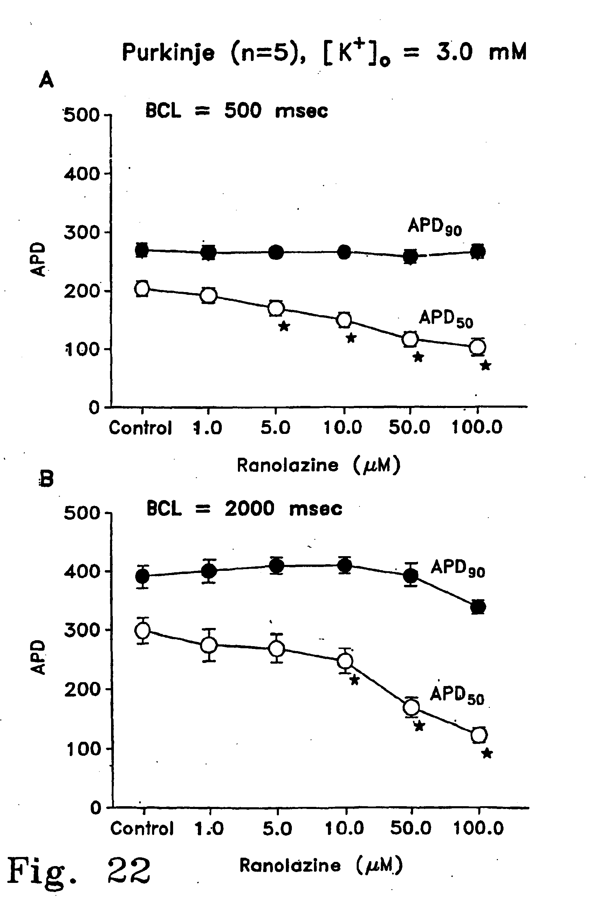

Lowering extracellular K⁺ did not modify the effects of ranolazine substantially. The most obvious differences include the tendency of the drug to prolong APD₉₀ at moderate concentrations and the induction of a smaller abbreviation of APD by highest concentration of the drug at a BCL of 2000 msec (Figure 22, Table 12).

**Table 12**

| Effects ofranolazine on phase 0 amplitude, resting membrane Potential (RMP), and overshoot of action potential in Purkinje fibers in the Presence of low [K⁺]₀ Ranolazine (in µM) | | | | | | |
|---|---|---|---|---|---|---|
| | Control | 1.0 | 5.0 | 10.0 | 50.0 | 100.0 |
| Amplitude | 130±9 | 132±6 | 130±5 | 128±4 | 121±7* | 114±7* |
| RMP | -92±1 | -92±1 | -92±1 | -92±1 | -92±1 | -90±2 |
| Overshoot | 38±9 | 40±5 | 38±4 | 37±4 | 29±6* | 24±7* |

| | | | | | | |
|---|---|---|---|---|---|---|
| [K⁺]₀ =3.0 mM; BCL = 500 msec Data are expressed as mean ±SD, n=5, *p<0.05 vs. control | | | | | | |

Concentrations greater than 5-10 µM significantly abbreviated APD₅₀. As with the higher level of [K⁺]ₒ, the amplitude of phase 0 and overshoot of the action potential were significantly reduced by high concentrations of ranolazine (50 and 100 µM). EADs were never observed.

### Ranolazine suppression of d-sotalol-induced EADs

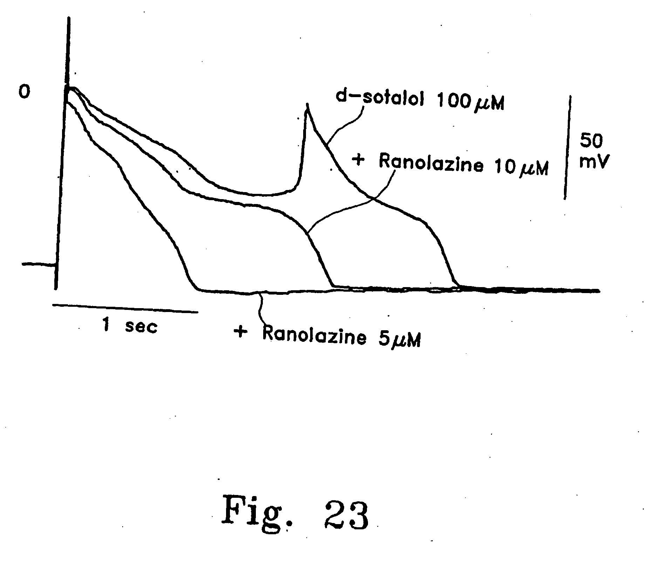

The specific I_{Kr} blocker d-sotalol (100 µM) induced EAD activity in 4 out of 6 Purkinje fiber preparations. Ranolazine, in a concentration as low as 5 µM, promptly abolished the d-sotalol-induced EADs in 4 out of 4 Purkinje fibers (Figure 23). Higher levels of Ranolazine (10 µM) produced a greater abbreviation of the action potential.

### EXAMPLE 18

### IV. Effects of ranolazine on QT prolongation and arrhythmia induction in anesthetized dog: comparison with sotalol

### A. Materials and Methods

Dogs were pretreated with Atravet (0.07 mg/kg sc) and then 15 minutes later anesthetized with ketamine (5.3 mg/kg iv) and valium (0.25 mg/kg iv) followed by isoflurane (1-2%), intubated and subjected to mechanical ventilation. They were then subjected to AV block with radiofrequency ablation. A median stemotomy was performed and catheters were inserted into a femoral artery for blood pressure (BP) recording and into both femoral veins for infusion of test drugs. Bipolar electrodes were inserted into both ventricles for programmed stimulation determination of refractory periods (extrastimulus technique), as well as for evaluation of QT interval and QRS duration at various controlled basic cycle lengths (BCLs). TdP was induced by challenges of phenylephrine, which were given as bolus intravenous doses of 10, 20, 30, 40 and 50 µg/kg. After each dose, the ECG was monitored continuously to detect arrhythmias. The BP always rose after phenylephrine, and sufficient time (at least 10 minutes) was allowed for BP to normalize before giving the next dose of phenylephrine. Test drug effects were evaluated as per protocols below.

Data are presented as the mean ± S.E.M. Statistical comparisons were made with Student's t test. A 2-tailed probability <0.05 was taken to indicate statistical significance. In data tables, *denotes P<0.05, **P<0.01.

### B. Study Design (Protocols)

The test drug was infused as: Group 1 (5 dogs): Sotalol was administered iv at a loading dose of 8 mg/kg and a maintenance dose of 4 mg/kg/hr. Group 2 (6 dogs): Five dogs received ranolazine as a 0.5 mg/kg iv load followed by a first, a second and a third continuous iv infusion of 1.0, 3.0 and 15 mg/kg/hr, respectively. One dog received ranolazine as a 1.5 mg/kg iv load followed by infusions of 15 and 30 mg/kg/hr. Twenty minutes after starting the maintenance infusion (for sotalol) or 30 minutes after starting each iv infusion rate (for ranolazine) electrophysiological measurements (right and left ventricular ERP, QT and QRS) were obtained at BCLs of 300, 400, 600 and 1000 ms. The phenylephrine challenges were then given, with all doses given at each drug infusion rate, and any arrhythmias monitor.

### EXAMPLE 19

Table 13 summarizes the proarrhythmic effects (bigeminy, trigeminy, torsades de pointes and torsades de pointes degenerating to ventricular fibrillation) of sotalol in the model.

**Table 13**

| Arrhythmia occurrence in sotalol group | | | | | | |
|---|---|---|---|---|---|---|
| ID | Sot 8+4 | PE10 | PE20 | PE30 | PE40 | PE50 |
| Sot1 | - | - | - | -bigeminy | -tdp 30 beats | |
| | | | | -trigeminy | CL-206.9 | |
| | | | | | -tdp 16 beats | |
| | | | | | CL-194.7 | |
| | | | | | -tdp VF | |
| | | | | | -tdp 7 beats | |
| | | | | | CL-230 | |
| | | | | | -tdp VF death | |
| Sot2 | -S1=1000, | -VT | | | | |
| | S2=275 | mono | | | | |
| | VT 4 | tdpVF | | | | |
| | beats | death | | | | |
| | CL=186.7 | | | | | |
| | -S1=1000, | | | | | |
| | S2=270 | | | | | |
| | VT 4 | | | | | |
| | beats | | | | | |
| | CL=173.7 | | | | | |
| | -S1-1000, | | | | | |
| | S2=265 | | | | | |
| | tdp 21 | | | | | |
| | beats | | | | | |
| | CL=144 | | | | | |
| | -S1=300, | | | | | |
| | S2=230 | | | | | |
| | tdp VF | | | | | |
| | -tdp VF | | | | | |
| Sot3 | - | -tdp 13 | -bigeminy | -bigeminy | -VT mono 5 | |
| | | beats | -trigeminy | -trigeminy | beats CL=250 | |
| | | CL=201.7 | | | -tdp 21 beats | |
| | | | | | CL=195 | |
| | | | | | -tdp VF | |
| | | | | | -tdp VF | |
| | | | | | -tdp VF | |
| | | | | | death | |
| Sot4 | -S1=1000, | - | -bigeminy | -bigeminy | -bigeminy | - |
| | S2=235 | | | | | trigeminy |
| | VT 7 | | | | | -VT |
| | beats | | | | | mono 19 |
| | CL=137 | | | | | beats |
| | | | | | | CL=300 |
| | | | | | | -tdp VF |
| | | | | | | -tdp VF |
| | | | | | | death |
| Sot5 | - | - | - | -tdp VF | | |
| | | | | death | | |
| VT = ventricular tachycardia, VF = ventricular fibrillation, mono = monomorphic, tdp = torsade de pointes, CL = cycle length, sot = sotalol, PE 10,20,3 0,40,50 = phenylephrine at 10,20,30,40,50 µg/kg respectively | | | | | | |

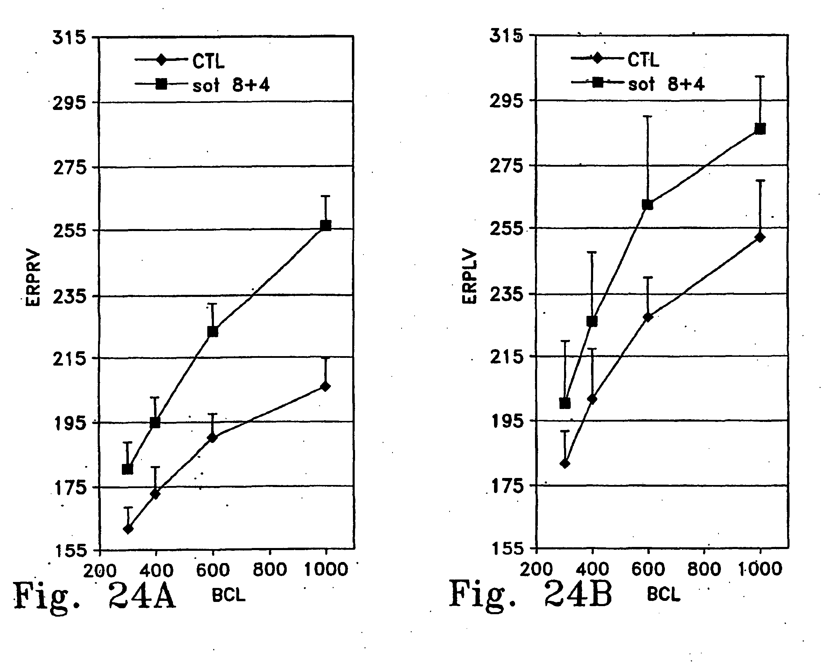

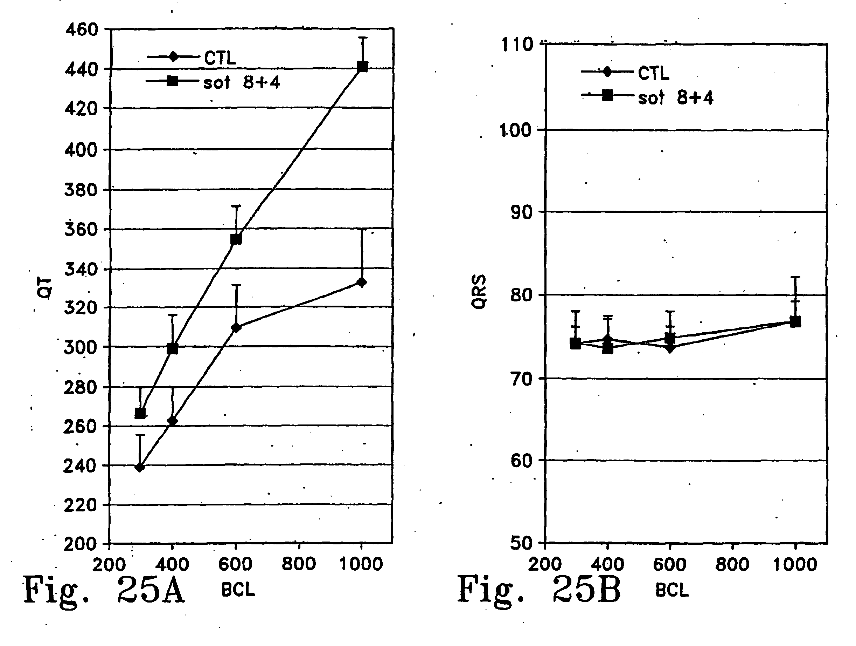

Two of five dogs had proarrhythmia without phenylephrine challenge, and all 5 had proarrhythmia upon phenylephrine challenge. All the dogs eventually died from torsade de pointes degenerating to ventricular fibrillation induced by the combination of sotalol infusion and a phenylephrine bolus. Sotalol increased right ventricular (RV) and left ventricular (LV) effective refractory period in a reverse use-dependent fashion (Table 14 and Figures 24A and B). Sotalol increased QT interval in a strikingly reverse use-dependent fashion and did not affect QRS duration (Table 15 and Figures 25A and B).

**Table 14: Effects of Sotalol on Right and Left Ventricular ERP (ms)**

| | Mean ERP RV | |
|---|---|---|
| BCL | CTL | sot 8+4 |
| 1000 | 206.00±8.86 | 255.50+9.56** |
| 600 | 191.00±7.1 | 223.50±9.07** |
| 400 | 174.00±7.85 | 1 95.67±7.53** |
| 300 | 162.00±6.82 | 1 81 .33±8.21 ** |
| 1000 | 252.50±17.5 | 286.25±16.25* |
| 600 | 227.50±12.5 | 262.50±27.5* |
| 400 | 202.50±15 | 226.25±21.25 |
| 300 | 182.50±10 | 201.25±18.75 |

| | | |
|---|---|---|
| BCL= Basic Cycle Length Sot 8+4=sotalol IV laoding dose of 8 CTL= Control mg/kg + maintenance dose of 0 mg/kglhr * p<0.05 ** p<0,01 | | |

**Table 15: Effects on QT and QRS Intervals (ms):**

| BCL | QT | QT | BCL | SE CTL | SE sot8+4 |
|---|---|---|---|---|---|
| | CTL | sot 8+4 | | | |
| 1000 | 332.70±77.00 | 440.93**±76.93 | *1000* | *26.7±2.37* | *14.06±5.39* |
| 600 | 309.85±73.60 | 354.67**±74.73 | *600* | *21.33±2.50* | *15.54±3.11* |
| 400 | 262.73±74.53 | 299.14*±73.53 | *400* | *77.37±2.38* | *16.75±3.76* |
| 300 | 238.40±74.07 | 266.40*±74.07 | *300* | *16.95±1.86* | *13.11±3.68* |

Results are available for the 5 dogs receiving the standard ranolazine infusion protocol. The high-dose dog died of pump failure during the 30 mg/kg/hr infusion, with no ventricular arrhythmias and electrophysiological study of this dog could not be performed. Table 16 summarizes arrhythmia occurrence in the presence of ranolazine, alone and in combination with phenylephrine boluses (10-50 µg/kg) according to an identical protocol as for sotalol above. We were unable to induce any torsades de pointes and/or ventricular fibrillation during ranolazine infusion with or without phenylephrine boluses.

**Table 16**

| Arrhythmia occurrence in ranolazine group | | | |
|---|---|---|---|
| ID | Ran 0.5 +1 | Rano3 | Rano 15 |
| Rano 1 | PE10- | PE10- | PE10- |
| | | | PE20- |
| | PE20- | PE20, fast IDV, 16 beats, CL=709.3 | PE30- |
| | | | PE40- |
| | PE30, 55 min inf., fast IDV, 12 beats, CL=512.7 | PE30, 56 min inf., fast IDV, 16 beats, CL=309.3 | PE50, fast mV, 5 beats, CL=575 fast IDV, 18 beats, CL=529.4 |
| Rano2 | PE10- | PE10- | PE10- |
| | PE20- | | PE20- |
| | PE30- | PE20- | PE30- |
| | PE40- | | PE40- |
| | PE50 bigeminy | PE30- | PE50- |
| Rano3 | PE10- | PE10- | PE10- |
| | PE20- | PE20- | PE20- |
| | PE30- | PE30- | PE30- |
| | PE40- | PE40- | PE40- |
| | PE50- | PE50- | PE50- |
| Ran04 | PE10 fast IDV, 37 beats, CL=633.9 | PE10- | PE10- |
| | | pE20- | |
| | PE20- | PE30- | PE20- |
| | PE30- | PE40- | PE30- |
| | PE40- | PE50- | PE40- |
| Rano6 | PE10- | S1=300, S2=180, VT 13 beats, CL=266.7 | PE10- |
| | PE20- | PE10- | PE20- |
| | PE30- | PE20- | PE30- |
| | | PE30- | PE40- |
| | PE40- | PE40- | PE50- |
| | | PE50- | |

| | | | |
|---|---|---|---|
| Rano = ranolazine, VT = ventricular tachycardia, IDV = idioventricular escape beat, CL = cycle length, PE = phenylephrine, inf. = infusion | | | |

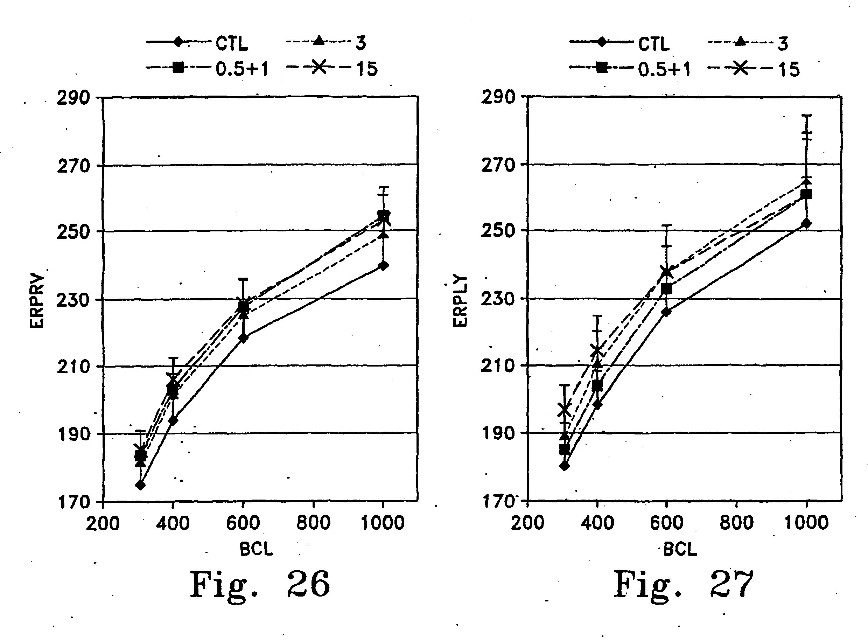

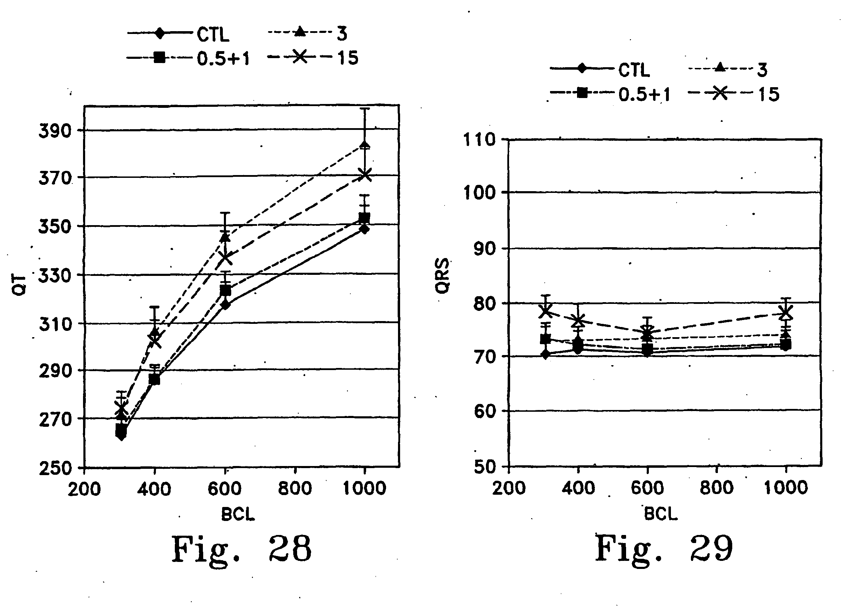

Ranolazine slightly increased ERP (mean increases not larger than about 10%), with no reverse use-dependence (Table 17 A and B and Figures 26 and 27). QT intervals were increased modestly (maxiumum increase was approximately 10%) but not significantly, with maximum effects at 3 mg/kg per hour and a decrease at the higher dose (Table 18 A and B and Figure 28 and 29).

**Table 17A: Effects of Ranolazine on Right and Left Ventricular ERP (ms)**

| Mean ERP-RV+SE | | | | |
|---|---|---|---|---|
| BCL | CTL | 0.5+1 | 3 | 15 |
| 1000 | 240.20±9.9 | 254.00*±9.31 | 249.50±6.19 | 253.16±7.77 |
| 600 | 218.50±8.93 | 227.50±8.87 | 224.50±4.83 | 229.50±6.19 |
| 400 | 194.00±6.83 | 201.50±6.45 | 199.66±3.75 | 206.50±5.79 |
| 300 | 175.00±5.25 | 182.84±6.67 | 181.00±2.32 | 185.00±5.76 |

**Table 17B: Effects of Ranolazine on Right and Left Ventricular ERP (ms)**

| Mean ERP-LV+SE | | | | |
|---|---|---|---|---|
| BCL | CTL | 0.5+1 | 3 | 15 |
| 1000 | 252.16±14.13 | 259.38±8.18 | 265.43±19.42 | 260.43±19.32 |
| 600 | 226.16±11.29 | 233.13±12.43 | 238.13±13.25 | 237.50±14.11 |
| 400 | 198.50±9.7 | 204.38±11.01 | 211.45±9.2 | 215.00±10.05 |
| 300 | 180.50±7.18 | 185.00±8.1 | 189.38±8.32 | 196.88*±7.53 |

**Table 18A: Effects of Ranolazine on QT Interval (ms):**

| Mean QT ± SE | | | | |
|---|---|---|---|---|
| BCL | CTL | 0.5+1 | 3 | 15 |
| 1000 | 348.40±9.07 | 352.52±9.05 | 384.02±13.9 | 369.80±11.6 |
| 600 | 318.20±8.58 | 323.50±7.74 | 345.00±10.04 | 336.34±11.43 |
| 400 | 285.40±6.02 | 286.50±5.76 | 306.46±10.38 | 302.18±9.33 |
| 300 | 263.60±6.61 | 266.16±6.36 | 272.72±6.09 | 274.82±6.48 |

**Table 18B : Effect of Ranolazine on QRS Interval**

| Mean QT ± SE | | | | |
|---|---|---|---|---|
| BCL | CTL | 0.5+1 | 3 | 15 |
| 1000 | 72.10±2.96 | 72.51±3.35 | 74.24±2.9 | 78.50±2.6 |
| 600 | 70.90±3.27 | 71.68±2.94 | 73.72±2.29 | 74.84*±2.56 |
| 400 | 71.37±3.53 | 72.36±3.39 | 73.18±2.57 | 76.82±3.06 |
| 300 | 70.65±3.52 | 73.60±2.8 | 73.26±2.33 | 78.48*±2.8 |

### EXAMPLE 20

### Effects of ranolazine on late I_{Na} during action potential voltage clamp

Adult male mongrel dogs were given 180 IU/kg heparin (sodium salt) and anesthetized with 35 mg/kg i.v. pentobarbital sodium, and their hearts were quickly removed and placed in Tyrode's solution. Single myocytes were obtained by enzymatic dissociation from a wedge-shaped section of the ventricular free wall supplied by the left circumflex coronary artery. Cells from the midmyocardial region of the left ventricle were used. All procedures were in accordance with guidelines established by the Institutional Animal Care and Use Committee.

Tyrode's solution used in the dissociation contained (mM0: 13 5 NaCl, 5.4 KCl, 1 MgCl₂, 0 or 0.5 CaCl₂, 10 glucose, 0.33 NaH₂PO₄, 10 N-2-hydroxyethylpiperazine N'-2-ethanesulfonic acid (HEPES) and pH was adjusted to 7.4 with NaOH. The compositions of the external and internal solutions used are summarized in Table 19.

**Table 19**

| External Solution | Internal Solution |
|---|---|
| I_{Na}, late Whole Cell (mM) | I_{Na,late} (mM) |
| 10 glucose | 135 Cs-aspartate |
| 1 MgCl₂ | 1 MgCl₂ |
| | 10 NaOH |
| 2 CaCl₂ | 10 EGTA |
| 150 Na-methanesulfonate | 5 Mg-ATP |
| 10 HEPES | 10 HEPES |
| pH 7.4 with methane sulfonic acid | pH 7.1 with CsOH |

Late I_{Na} was recorded at 37°C using standard patch electrodes. Dissociated cells were placed in a temperature controlled 0.5 ml chamber (Medical Systems, Greenvale, NY) on the stage of an inverted microscope and superfused at 2 ml/min. A four-barrel quartz micro-manifold (ALA Scientific Instruments Inc., Westbury, NY) placed 100 µm from the cell was used to apply ranolazine and tetrodotoxin (TTX). An inline heater (Harvard/Wamer, Holliston, MA) was used to maintain temperatures of solutions within the quartz manifold. An Axopatch 700A amplifier (Axon Instruments, Foster City CA) was operated in voltage clamp mode to record currents at 37°C. Whole cell currents were filtered with a 4-pole low-pass Bessel filter at 5 kHz, digitized between 2 - 5 kHz (Digidata 1200A, Axon Instruments) and stored on a computer. pClamp 8.2 software (Axon Instruments) was used to record and analyze ionic currents. Pipette tip resistance was 1.0-1.5 MΩ and seal resistance was greater than 5 GΩ. Electronic compensation of series resistance averaged 76%. Voltages reported were corrected for patch electrode tip potentials. The seal between cell membrane and patch pipette was initially formed in Tyrode's solution containing 1 mM CaCl₂. A 3 M KCl-agar bridge was used between the Ag/AgCl ground electrode and external solution to avoid development of a ground potential when switching to experimental solution.

Tetrodotoxin (TTX) was prepared in water and diluted 1:100 for a final concentration of 10 µM in external solution. Ranolazine dihydrochloride was prepared in water at a concentration of 5 mM and diluted in external solution to final concentrations ranging from 1-50 µM.

I_{Na,late} was recorded during a train of 30 pulses at repetition rates of 300 and 2000 ms. Currents during the last 5 pulses of the trains were averaged to reduce noise, and late I_{Na} was defined as the TTX-sensitive current. Protocols were repeated in drug-free solution, 2 to 4 minutes after adding ranolazine, and immediately after 10 µM TTX was added to completely block I_{Na}, late.

Action potentials, rather than square pulses were used to voltage clamp I_{Na}, late. At a BCL of 300 ms, measurements were made midway through the plateau at a voltage of 13 mV and during phase 3 repolarization at a voltage of -28 mV. At a BCL of 2000 ms, measurements were made at similar positions at voltages of 20 mV and -28 mV. Reduction of late IN. was plotted as a function of drug concentration on a semi-log scale and fitted to a logistic equation.

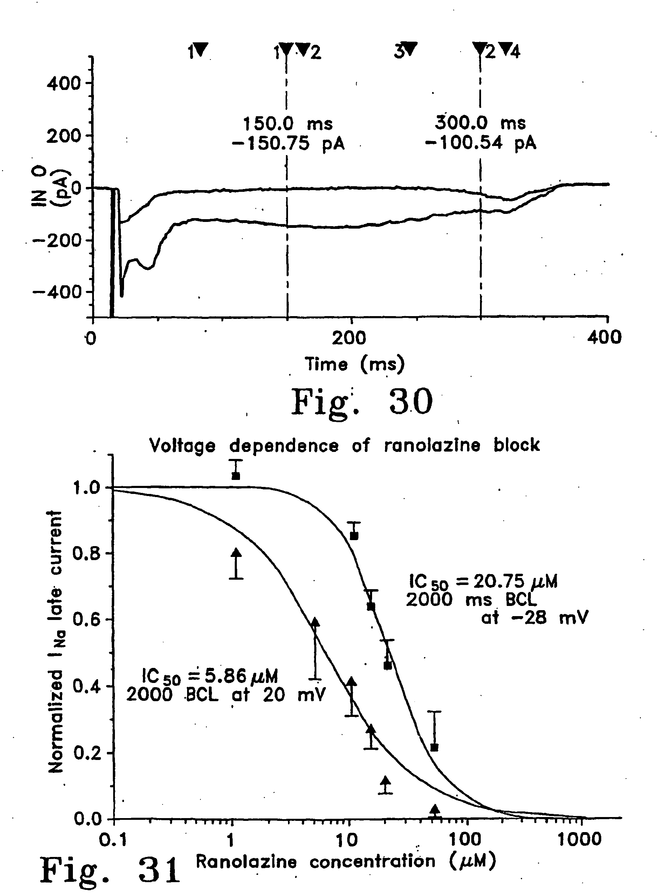

Figure 30 shows TTX-sensitive currents in control solution and 3 min after addition of 20 µM ranolazine to the external solution. The cell was pulsed every 2000 ms for 30 pulses. This figure shows that plateau currents were more sensitive to ranolazine than the sodium current recorded late in the action potential clamp. Inhibition was greatest at 20 mV, but some TTX-sensitive current remained at -28 mV in the presence of ranolazine.

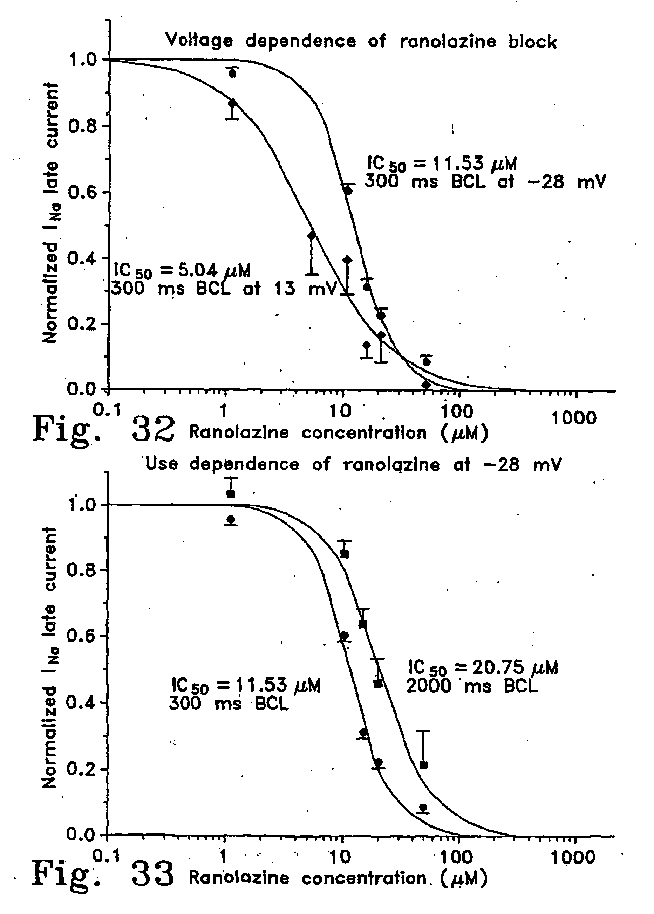

Figure 31 shows the summary results of similar experiments in which ranolazine (1 - 50 µM was added to the external solution. Half-inhibition of late I_{Na} occurred at drug concentrations of 5.9 µM and 20.8 µM, respectively. Figure 32 shows that inhibition was more potent during the plateau, even when cells were pulsed every 300 ms.

Figure 33 shows the composite data of similar experiments in which ranolazine was added to the external solution. Half-inhibition of I_{Na,late} occurred at a drug concentration of 20.8 µM and 11.5 µM when pulsed at basic cycle lengths of 2000 ms and 300 ms, respectively.

### EXAMPLE 21

### Effects of Ranolazine on the Duration of Action Potential of Guinea Pig Ventricular Myocytes

### Isolation of ventricular myocytes

Single ventricular myocytes were isolated from the hearts of adult, male guinea pigs (Harlan). In brief, the hearts were perfused with warm (35°C) and oxygenated solutions in the following order: 1) Tyrode solution containing (in mmol/L) 140 NaCl, 4.6 KCl, 1.8 CaCl₂, 1.1 MgSO₄, 10 glucose and 5 HEPES, pH 7.4, for 5 minutes; 2) Ca²⁺ -free solution containing (in mmol/L) 100 NaCl, 30 KCl, 2 MgSO₄, 10 glucose, 5 HEPES, 20 taurine, and 5 pyruvate, pH 7.4, for 5 minutes; and 3) Ca²⁺ -free solution containing sollagenase (120 units/ml) and albumin (2 mg/ml), for 20 minutes. At the end of the perfusion, the ventricles were removed, minced, and gently shaken for 10 minutes in solution #3. Isolated cells were harvested from the cell suspension.

### Measurement of action potential duration

Myocytes were placed into a recording chamber and superfused with Tyrode solution at 35°C. Drugs were applied via the superfusate. Action potentials were measured using glass microelectrodes filled with a solution containing (in mmol/L) 120 K-aspartate, 20 KCl, 1 MgCl₂, 4 Na₂ATP, 0.1 Na₃GTP, 10 glucose, 1 EGTA and 10 HEPES (pH 7.2). Microelectrode resistance was 1-3 MΩ. An Axopatch-200 amplifier, a DigiData-1200A interface and pCLAMP6 software were used to perform electrophysiological measurements. Action potentials were induced by 5-ms depolarizing pulses applied at various frequencies as indicated. The duration of action potential was measured at 50% (APD₅₀) and 90% (APD₉₀) repolarization. Measurements were made when the response to a drug had reached a stable maximum.

### Experimental Protocol

1) Ventricular myocytes were electrically stimulated at a frequency of 0.5, 1 or 2 Hz. Each myocyte was treated with 3, 10 and 30 µmol/L ranolazine. The effect of ranolazine on action potential duration at each pacing frequency was determined from 4 myocytes.
2) Action potentials were elicited at a frequency of 0.25 Hz, and the effect of ranolazine (10 µmol/L) on action potential duration was examined in the presence of 5 µmol/L quinidine. Experiments were performed on 4 myocytes.

### Statistical Analysis

Data are expressed as mean tSEM. The paired Student's t-test was used for statistical analysis of paired data, and the one-way repeated measures ANOVA followed by Student-Newman-Keuls test was applied for multiple comparisons. A p value < 0.05 was considered statistically significant.

### Effect of ranolazine at various pacing frequencies

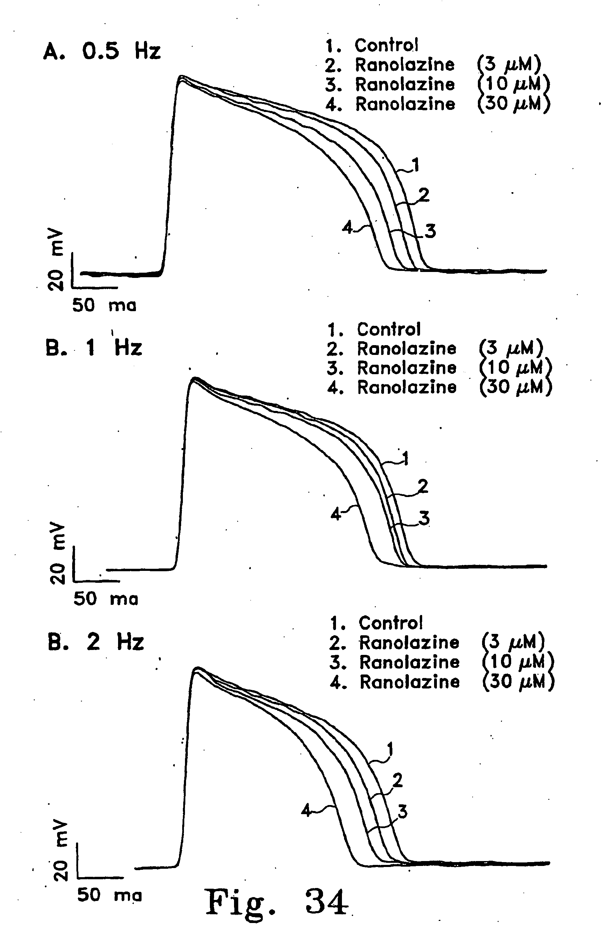

In the absence of drug, the APD₅₀ and APD₉₀ measured at stimulation frequencies of 0.5 (n=4), 1(n=4) and 2 (n=4) Hz were 250±20, 221±18, and 208±9 ms, and 284±22, 251±20 and 245±9 ms, respectively. Thus, increasing the pacing frequency resulted in a rate-dependent shortening of the action potential duration. Irrespective of the pacing frequency, ranolazine caused a moderate and concentration-dependent shortening of both the APD₅₀ and APD₉₀. Figure 34 shows that ranolazine at 3,10, and 30 µmol/L decreased the action potential duration of myocytes stimulated at 0.5, 1, and 2 Hz. The shortening of action potential duration caused by ranolazine was partially reversible after washout of the drug.

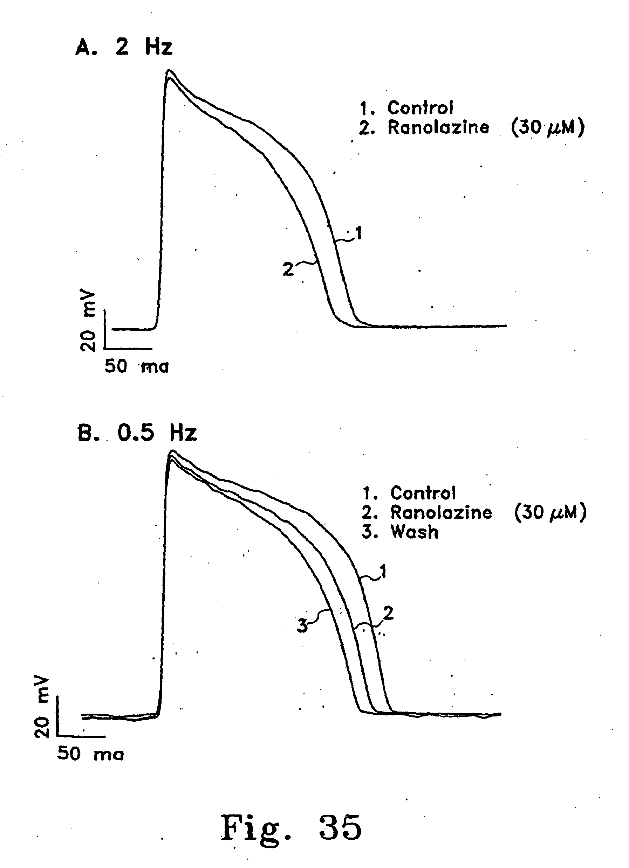

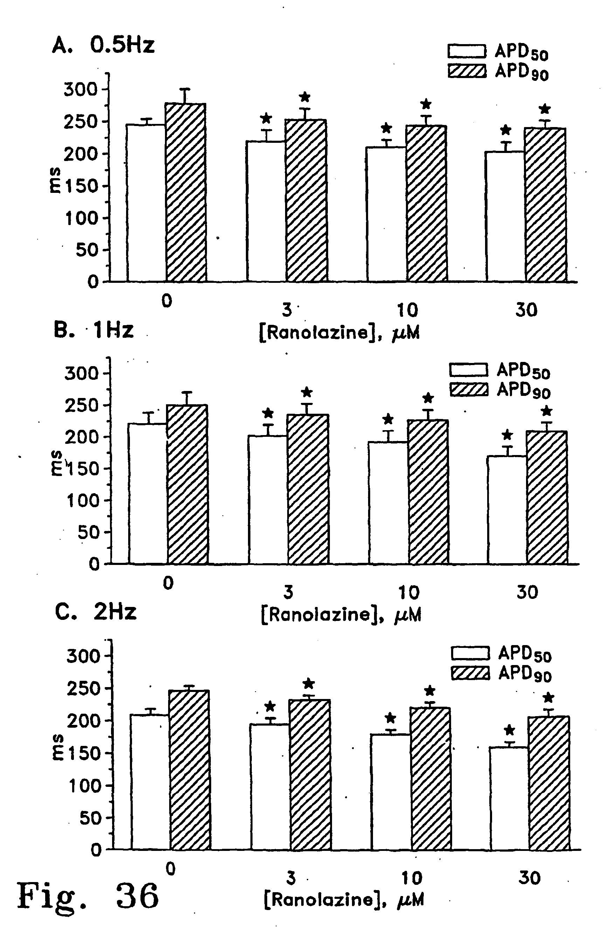

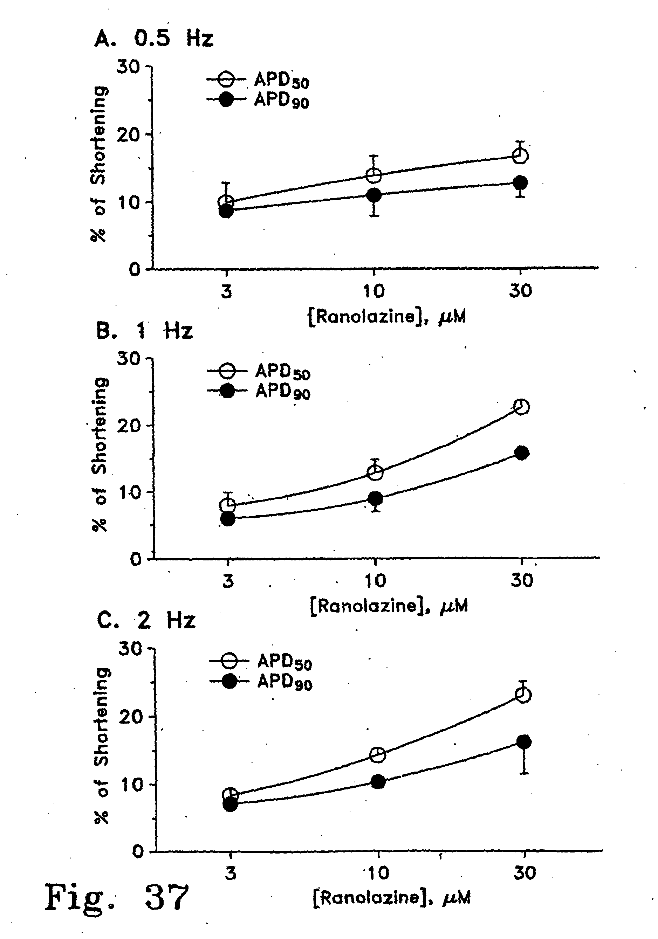

Figure 35 shows the results obtained from a single myocyte paced first at 2 Hz, and then at 0.5 Hz. At the two pacing frequencies, molazine (30 µmol/L) caused a similar shortening of the action potential duration. Comparisons of the APD₅₀ and APD₉₀ measured in the absence and presence of 3, 10 and 30 µmol/L ranolazine at pacing frequencies of 0.5*,* 1 and 2 Hz are shown in Figure 36. The shortening of APD₅₀ and APD₉₀ by ranolazine at various pacing frequencies is normalized as percentage of control, and is shown in Figure 37.

### Effect of ranolazine in the presence of quinidine

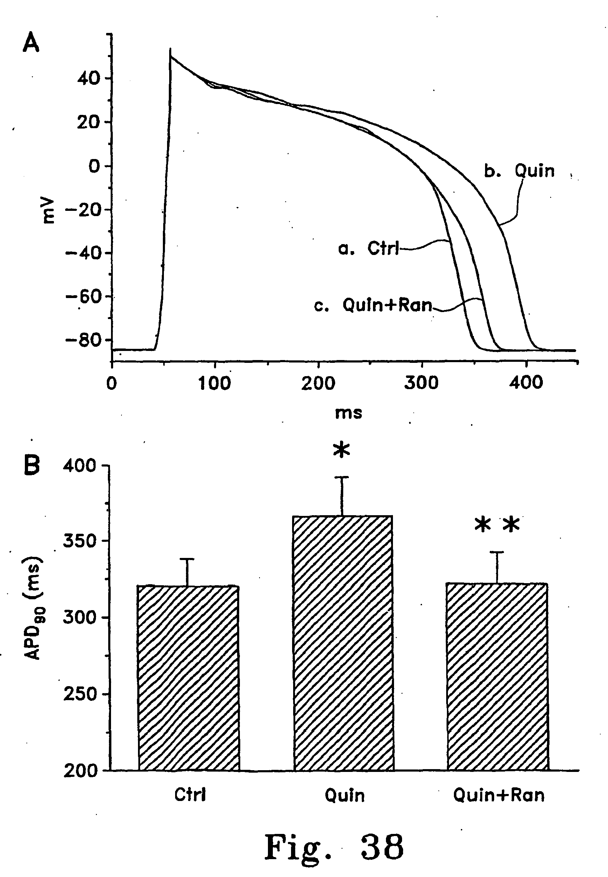

Figure 38A shows that quinidine (5 µmol/L) increased the duration of action potential of a myocyte paced at 0.25 Hz. Ranolazine (10 µmol/L) is shown to have attenuated the effect of quinidine.

Quinidine, in addition to prolonging the action potential duration, is known to induce early afterdepolarizations (EADs), triggered activity and torsade de pointes. As shown in Figures 39 and 40, quinidine (2.5 µmol/L) induced EADs and triggered activity. Ranolazine (10 µmol/L,) was found to be effective in suppressing EADs (Figure 39) and triggered activity (Figure 40) induced by quinidine.

### EXAMPLE 22

Following the procedures and protocols of Example 21, guinea pig ventricular myocytes were electrically stimulated in the presence of ranolazine either alone or in the presence of ATX II [a sea anemone toxin known to mimic LQT3 syndrome by slowing Na⁺ -channel inactivation from the open state and thereby increasing the peak and late Na⁺ current (I_{Na}) of cardiomyocytes]. ATXII is known to induce early afterdepolarizations (EADs) and triggered activity and ventricular tachycardia.

ATXII (10-40 nmol/L) was found to markedly increase the duration of action potentials measured at 50% repolarization (APD₅₀) from 273±9 ms to 1,154±61 ms (n=20, p<0.001) as shown in Figure 41, and induced EADs in all cells. Multiple EADs and resultant sustained depolarization were frequently observed. Ranolazine at a concentration as low as 1 µmol/L effectively abolished ATXII induced EADs and triggered activity. The prolongation of the APD₅₀ caused by ATXII was significantly (p<0.001) attenuated by ranolazine at concentrations of 1, 3, 10 and 30 µmol/L, respectively, by 60±4% (n=7), 80±2% (n=7), 86±2% (n=12) and 99±1% (n=8), as shown in Figures 42, 43, 44, 45, and 46. These figures depict 5 different experiments.

### EXAMPLE 23

To study the effect of ranolazine on ATXII induced MAP (monophasic action potential) duration prolongation, EADs and ventricular tachyarrhythmia (VT), the K-H buffer perfused guinea pig isolated heart model was used.

ATXII (10-20 nM) was found to prolong MAPD₉₀ by 6% in 4 hearts without rapid ventricular arrhythmia. ATXII markedly induced EADs and polymorphic VT in 10/14 guinea pig isolated hearts. Ranolazine at 5,10 and 30 µM significantly suppressed EADs and VT, especially sustained VT, in the presence of ATXII. The protective effect of ranolazine was reversible upon washout of ranolazine. These results are shown in Figures 47 through 50.

Figure 47 shows the MAP and ECG for control, ATXII (20 nM), and ATXII (20 nM) plus ranolazine (10 µM). This figure shows that ranolazine reduced the ATXII-induced EAD and MAP prolongation.

Figure 48 shows the MAP and ECG for ATXII (20 nM)-induced VT, either spontaneous VT or pacing-induced VT.

Figure 49 shows that ranolazine reduced ATXII-induced VT. This figure shows the MAP and ECG for both ATXII (20nM) alone and A TXII (20 nM) plus ranolazine (30 µM).

Figure 50 shows that ranolazine (10 µM) reversed ATXII-induced EAD and ΔMAP.

### Example 24

To determine whether ranolazine suppressed ATX-11 induced 1) EADs and triggered activity (TA), and 2) ventricular tachycardia (VT) guinea pig ventricular myocytes and isolated hearts, respectively, were used.

Action potentials were recorded using the whole-cell patch-electrode technique. Ventricular monophasic action potentials and electrograms were recorded from isolated hearts. ATX-II (10-20 nmol/L) increased the APD measured at 50% reporlarization (APD₅₀) from 271±7 ms to 1,1481±49 ms (n=24, p<0.001), and induced EADs in all cells. Multiple EADs and sustained depolarizations were frequently observed. Ranolazine at concentrations ≥ 1 µmol/L abolished ATX-II induced EADs and TA. Prolongation of the APD₅₀ caused by ATX-II was significantly (p<0.001) reduced by ranolazine at concentrations of 0.1, 0.3, 1, 3, 10 and 30 µmol/L by 29±1% (n=5), 47±1% (n=5), 63±3% (n=11), 79±1% (n=10), 86±2% (n=12) and 99±1% (n=8), respectively. Ranolazine (10 µmol/L) also suppressed EADs and TA induced by 2.5 µmol/L quinidine (n=2). ATX-II (10-20 nmol/L) caused EADs and VT in 10 of 14 isolated hearts; ATX-II induced EADs were significantly reduced and VTs were terminated by 5-30 µmol/L ranolazine.

### Example 25

To determine whether an increase by ATX-II (which mimics SCN5A mutation) of the I_{Na(L)} facilitates the effects of E-4031 and 293B (potassium channel blockers of the rapid and slow components of the delayed rectifier (I_{K}) to prolong the APD and to induce EADs, and whether ranolazine reverses the effects of ATX-II and the K⁺ blockers, guinea pig ventricular myocytes and isolated hearts were used.

The ventricular APD of guinea pigs isolated myocyytes and hearts was measured, respectively, at 50% (APD₅₀) and 90% (MAPD₉₀) repolarization. ATX-II at a low concentration (3 nmol/L) only slightly increased the APD₅₀ by 6±2%. However, when applied with either E-4031 or 293B, ATX-II greatly potentiated the effects of these K⁺ blockers to prolong the APD. In the absence and presence of ATX-II, the APD₅₀ was increased by 11±2% and 104±41% by E-4031 (1 µmol/L), and 40±7% and 202±59% by 293B (30 µmol/L), respectively. Moreover, E-4031 and 293B induced EADs in the presence, but not in the absence, of ATX-II. Ranolazine (10 µmol/L) completely abolished the EADs and significantly reversed the prolongation of the APD₅₀ by about 70% in the presence of ATX-H plus either E-4031 or 293B. ATX-11 (7 nmol/L), E-4031 (1 µmol/L) and 293B (1 µmol/L) alone increased the MAPD₉₀ by 32±0.1%, 30.1±0.1% and 6.3±0.2%, respectively. When applied with ATX-II, E-4031 and 293B increased the MAPD₉₀ by 127.1±0.4% and 31.6±0.1%, respectively. Ranolazine (10 µmol/L) significantly decreased the MAPS₉₀ by 24.5±0.1% in the presence of ATX-II plus E-4031 and by 8.3±0.1% in the presence of ATX-II plus 293B.

## Claims

1. A compound selected from the group consisting of:
a compound of Formula I: 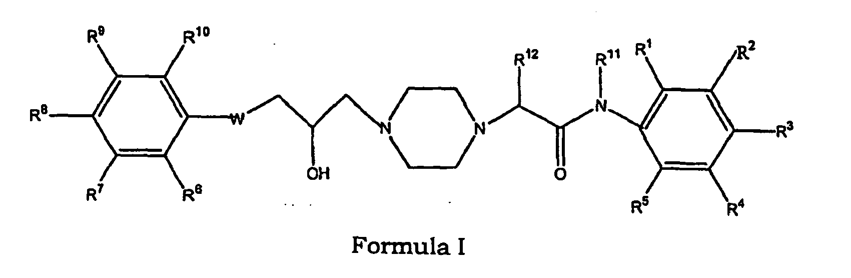 wherein:
R¹, R², R³, R⁴ and R⁵ are each independently hydrogen, lower alkyl, lower alkoxy, cyano, trifluoromethyl, halo, lower alkylthio, lower alkyl sulfinyl, lower alkyl sulfonyl, , or N-optionally substituted alkylamido, provided that when R¹ is methyl, R⁴ is not methyl; or R² and R³ together form -OCH₂O-R⁶, R⁷, R⁸, R⁹ and R¹⁰ are each independently hydrogen, lower acyl, aminocarbonylmethyl, cyano, lower alkyl, lower alkoxy, trifluoromethyl, halo, lower alkylthio, lower alkyl sulfinyl, lower alkyl sulfonyl, or di-lower alkyl amino; or
R⁶ and R⁷ together form -CH=CH-CH=CH-; or
R⁷ and R⁸ together form -OCH₂O-;
R¹¹ and R¹² are each independently hydrogen or lower alkyl; and
W is oxygen or sulfur;
wherein lower alkyl refers to an unbranched saturated hydrocarbon chain of 1-4 carbons and lower acyl refers to the group RC(O)A, where R is lower alkyl and A represents the point of attachment,
an isomer of a compound of Formula I, and a pharmaceutically acceptable salt or ester of a compound of Formula I or its isomer for use in suppressing early after depolarizations (EADs) in a mammal.

2. The compound for the use of claim 1 which is ranolazine, named N-(2,6-dimethylphenyl)-4-[2-hydroxy-3-(2-methoxyphenoxy)propyl]-1-piperazineacetamide, or an isomer thereof, or a pharmaceutically acceptable salt of ranolazine or its isomer.

3. The compound for the use of claim 1 which is for administration at dose levels that inhibit I_{Kr}, I_{Ks}, and late I_{Na} ion channels but do not inhibit calcium channels.

4. The compound for the use of claim 2 which is ranolazine in the form of a pharmaceutically acceptable salt.

5. The compound for the use of claim 4 wherein the pharmaceutically acceptable salt is the dihydrochloride salt.

6. The compound for the use of claim 2 which is ranolazine in the form of the free base.

7. The compound for the use of claim 1 which is for administration comprising a dose level that inhibits late I_{Na} ion channels.

8. The compound for the use of claim 1 which is for administration comprising a dose level that inhibits I_{Kr}, I_{Ks}, and late I_{Na} ion channels.

9. The compound for the use of claim 1 which is for administration comprising a dose level that inhibits I_{Kr}, I_{Ks}, and late I_{Na} ion channels but does not inhibit calcium channels.

10. The compound for the use of claim 1 which is for administration in a manner that provides plasma levels of the compound of Formula I of at least 350±30 ng/mL for at least 12 hours.

11. The compound for the use of claim 1 which is for administration in a sustained release formulation that maintains plasma concentrations of the compound at less than a maximum of 4000 ng/mL, preferably between about 350 to about 4000 ng/mL, for at least 12 hours.

12. The compound for the use of claim 1 which is for administration in a formulation that contains between about 10 mg and 700 mg of said compound.

13. The compound for the use of claim 12 which is ranolazine or an isomer thereof, or a pharmaceutically acceptable salt of ranolazine or its isomer.

14. The compound for the use of claim 1 which is for administration in a formulation that provides a dose level of about 1 to about 30 micromoles per liter of formulation.

15. The compound for the use of claim 14 wherein said formulation provides a dose level of about 1 to about 10 micromoles per liter of formulation.

16. The compound for the use of claim 1 which is for administration by bolus or sustained release composition.

17. The compound for the use of claim 1 which is for intravenous administration.

18. Use of a compound of Formula I: 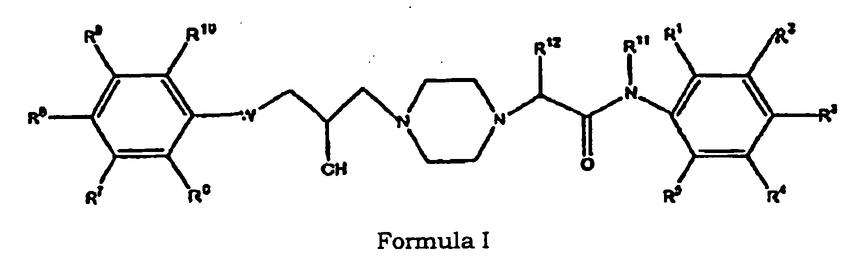 wherein:
R¹, R², R³, R⁴ and R⁵ are each independently hydrogen, lower alkyl, lower alkoxy, cyano, trifluoromethyl, halo, lower alkylthio, lower alkyl sulfinyl, lower alkyl sulfonyl, or N-optionally substituted alkylamido, provided that when R¹ is methyl, R⁴ is not methyl; or R² and R³ together form -OCH₂O-;
R⁶, R⁷, R⁸, R⁹ and R¹⁰ are each independently hydrogen, lower acyl, aminocarbonylmethyl, cyano, lower alkyl, lower alkoxy, trifluoromethyl, halo, lower alkylthio, lower alkyl sulfinyl, lower alkyl sulfonyl, or di-lower alkyl amino; or
R⁶ and R⁷ together form -CH=CH-CH=CH-; or
R⁷ and R⁸ together form -OCH₂O-;
R¹¹ and R¹² are each independently hydrogen or lower alkyl; and
W is oxygen or sulfur;
wherein lower alkyl refers to an unbranched saturated hydrocarbon chain of 1-4 carbons and lower acyl refers to the group RC(O)A, where R is lower alkyl and A represents the point of attachment,
or an isomer thereof, or a pharmaceutically acceptable salt or ester of a compound of Formula I or its isomer
for the preparation of a pharmaceutical composition for suppressing EADs in a mammal.

19. The use of claim 18 wherein the compound of Formula I is ranolazine, named N-(2,6-dimethylphenyl)-4-[2-hydroxy-3-(2-methoxyphenoxy)propyl]-1-piperazineacetamide, or an isomer thereof, or a pharmaceutically acceptable salt of ranolazine or its isomer.

20. The use of claim 18 wherein the pharmaceutical composition is for administering the compound of Formula I at dose levels that inhibit I_{Kr}, I_{Ks}, and late I_{Na} ion channels but do not inhibit calcium channels.

21. The use of claim 19 wherein ranolazine is in the form of a pharmaceutically acceptable salt.

22. The use of claim 21 wherein the pharmaceutically acceptable salt is the dihydrochloride salt.

23. The use of claim 19 wherein ranolazine is in the form of the free base.

24. The use of claim 18 wherein the pharmaceutical composition is for administration of the compound of Formula I that comprises a dose level that inhibits late I_{Na} ion channels.

25. The use of claim 18 wherein the pharmaceutical composition is for administration of the compound of Formula I that comprises a dose level that inhibits I_{Kr}, I_{Ks}, and late I_{Na} ion channels.

26. The use of claim 18 wherein the pharmaceutical composition is for administration of the compound of Formula I that comprises a dose level that inhibits I_{Kr}, I_{Ks}, and late I_{Na} ion channels but does not inhibit calcium channels.

27. The use of claim 18 wherein the pharmaceutical composition is for administering the compound of Formula I in a manner that provides plasma levels of the compound of Formula I of at least 350±30 ng/mL for at least 12 hours.

28. The use of claim 18 wherein the pharmaceutical composition is a sustained release formulation that maintains plasma concentrations of the compound at less than a maximum of 4000 ng/mL, preferably between about 350 to about 4000 ng/mL, for at least 12 hours.

29. The use of claim 18 wherein the pharmaceutical composition contains between about 10 mg and 700 mg of said compound.

30. The use of claim 29 wherein the compound of Formula I is ranolazine or an isomer thereof, or a pharmaceutically acceptable salt of ranolazine or its isomer.

31. The use of claim 18 wherein the pharmaceutical composition provides a dose level of about 1 to about 30 micromoles per liter of pharmaceutical composition.

32. The use of claim 31 wherein the pharmaceutical composition provides a dose level of about 1 to about 10 micromoles per liter of pharmaceutical composition.

33. The use of claim 18 wherein the pharmaceutical composition is to be administered by bolus or sustained release composition.

34. The use of claim 18 wherein the pharmaceutical composition is to be administered intravenously.

## Patentansprüche

1. Verbindung, die ausgewählt ist aus der Gruppe, bestehend aus:
einer Verbindung der Formel I: 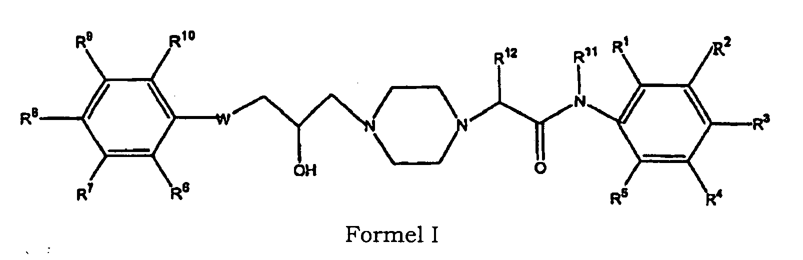 worin:
R¹, R², R³, R⁴ und R⁵ jeweils unabhängig voneinander Wasserstoffatom, Niederalkyl-, Niederalkoxy-, Cyan-, Trifluormethyl-, Halogen-, Niederalkylthio-, Niederalkylsulfinyl-, Niederalkylsulfonyl- oder N-gegebenenfalls substituierte Alkylamidgruppe sind, mit der Maßgabe, dass, wenn R¹ eine Methylgruppe ist, R⁴ keine Methylgruppe ist, oder R² und R³ zusammen eine -OCH₂O--Gruppe ausbilden,
R⁶, R⁷, R⁸, R⁹ und R¹⁰ jeweils unabhängig voneinander Wasserstoffatom, Niederacyl-, Aminocarbonylmethyl-, Cyan-, Niederalkyl-, Niederalkoxy-, Trifluormethyl-, Halogen-, Niederalkylthio-, Niederalkylsulfinyl-, Niederalkylsulfonyl- oder Diniederalkylaminogruppe sind oder
R⁶ und R⁷ zusammen eine -CH=CH-CH=CH--Gruppe ausbilden oder
R⁷ und R⁸ zusammen eine -OCH₂O--Grup ausbilden,
R¹¹ und R¹² jeweils unabhängig voneinander Wasserstoffatom oder Niederalkylgruppe sind und
W ein Sauerstoff- oder Schwefelatom ist,
wobei Niederalkylgruppe eine unverzweigte gesättigte Kohlenwasserstoffkette mit 1-4 Kohlenstoffatomen betrifft und Niederacylgruppe die Gruppe RC(O)A betrifft, worin R eine Niederalkylgruppe ist und A den Anbindungspunkt darstellt,
einem Isomer einer Verbindung der Formel I und einem pharmazeutisch verträglichen Salz oder einem pharmazeutisch verträglichen Ester einer Verbindung der Formel I oder dessen Isomer
für eine Verwendung bei einer Supprimierung von frühen Nachpolarisationen (EADs) bei einem Säuger.

2. Verbindung für die Verwendung nach Anspruch 1, die Ranolazin, namentlich N-(2,6-Dimethylphenyl)-4-[2-hydroxy-3-(2-methoxyphenoxy)propyl]-1-piperazinacetamid, oder ein Isomer davon oder ein pharmazeutisch verträgliches Salz von Ranolazin oder dessen Isomer ist.

3. Verbindung für die Verwendung nach Anspruch 1, die einer Verabreichung bei Dosismengen dient, die I_{Kr}-, I_{Ks}- und späte I_{Na}-Ionenkanäle hemmen, jedoch nicht Calciumkanäle hemmen.

4. Verbindung für die Verwendung nach Anspruch 2, die Ranolazin in der Form eines pharmazeutisch verträglichen Salzes ist.

5. Verbindung für die Verwendung nach Anspruch 4, wobei das pharmazeutisch verträgliche Salz das Dihydrochloridsalz ist.

6. Verbindung für die Verwendung nach Anspruch 2, die Ranolazin in der Form der freien Base ist.

7. Verbindung für die Verwendung nach Anspruch 1, die einer Verabreichung dient, die eine Dosismenge umfasst, die späte I_{Na}-Ionenkanäle hemmt.

8. Verbindung für die Verwendung nach Anspruch 1, die einer Verabreichung dient, die eine Dosismenge umfasst, die I_{Kr}-, I_{Ks}- und späte I_{Na}-Ionenkanäle hemmt.

9. Verbindung für die Verwendung nach Anspruch 1, die einer Verabreichung dient, die eine Dosismenge umfasst, die I_{Kr}-, I_{Ks}- und späte I_{Na}-Ionenkanäle hemmt, jedoch nicht Calciumkanäle hemmt.

10. Verbindung für die Verwendung nach Anspruch 1, die einer Verabreichung in einer Weise dient, die Plasmaspiegel der Verbindung der Formel I von mindestens 350±30 ng/ml für mindestens 12 Stunden bereitstellt.

11. Verbindung für die Verwendung nach Anspruch 1, die einer Verabreichung in einer Formulierung mit anhaltender Freisetzung dient, die Plasmakonzentrationen der Verbindung bei weniger als einem Maximum von 4000 ng/ml, vorzugsweise zwischen etwa 350 bis etwa 4000 ng/ml für mindestens 12 Stunden aufrechterhält.

12. Verbindung für die Verwendung nach Anspruch 1, die einer Verabreichung in einer Formulierung dient, die etwa 10 mg bis 700 mg der Verbindung enthält.

13. Verbindung für die Verwendung nach Anspruch 12, die Ranolazin oder ein Isomer davon oder ein pharmazeutisch verträgliches Salz von Ranolazin oder dessen Isomer ist.

14. Verbindung für die Verwendung nach Anspruch 1, die einer Verabreichung in einer Formulierung dient, die eine Dosismenge von etwa 1 bis etwa 30 Mikromol pro Liter Formulierung bereitstellt.

15. Verbindung für die Verwendung nach Anspruch 14, wobei die Formulierung eine Dosismenge von etwa 1 bis etwa 10 Mikromol pro Liter Formulierung bereitstellt.

16. Verbindung für die Verwendung nach Anspruch 1, die einer Verabreichung durch eine Boluszusammensetzung oder eine Zusammensetzung mit anhaltender Freisetzung dient.

17. Verbindung für die Verwendung nach Anspruch 1, die einer intravenösen Verabreichung dient.

18. Verwendung einer Verbindung der Formel I: 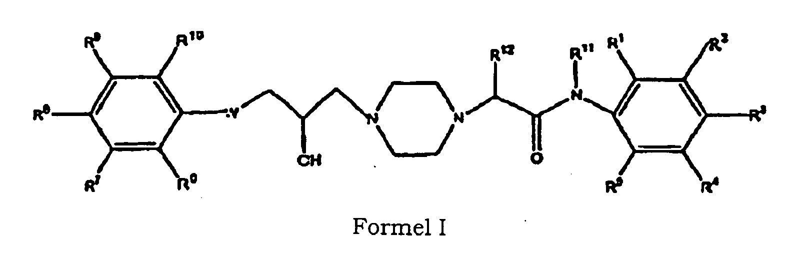 worin:
R¹, R², R³, R⁴ und R⁵ jeweils unabhängig voneinander Wasserstoffatom, Niederalkyl-, Niederalkoxy-, Cyan-, Trifluormethyl-, Halogen-, Niederalkylthio-, Niederalkylsulfinyl-, Niederalkylsulfonyl- oder N-gegebenenfalls substituierte Alkylamidgruppe sind, mit der Maßgabe, dass, wenn R¹ eine Methylgruppe ist, R⁴ keine Methylgruppe ist, oder R² und R³ zusammen eine -OCH₂O--Gruppe ausbilden,
R⁶, R⁷, R⁸, R⁹ und R¹⁰ jeweils unabhängig voneinander Wasserstoffatom, Niederacyl-, Aminocarbonylmethyl-, Cyan-, Niederalkyl-, Niederalkoxy-, Trifluormethyl-, Halogen-, Niederalkylthio-, Niederalkylsulfinyl-, Niederalkylsulfonyl- oder Diniederalkylaminogruppe sind oder
R⁶ und R⁷ zusammen eine -CH=CH-CH=CH--Gruppe ausbilden oder
R⁷ und R⁸ zusammen eine -OCH₂O--Grup ausbilden,
R¹¹ und R¹² jeweils unabhängig voneinander Wasserstoffatom oder Niederalkylgruppe sind und
W ein Sauerstoff- oder Schwefelatom ist,
wobei Niederalkylgruppe eine unverzweigte gesättigte Kohlenwasserstoffkette mit 1-4 Kohlenstoffatomen betrifft und Niederacylgruppe die Gruppe RC(O)A betrifft, worin R eine Niederalkylgruppe ist und A den Anbindungspunkt darstellt,
oder eines Isomers davon oder eines pharmazeutisch verträglichen Salzes oder eines pharmazeutisch verträglichen Esters einer Verbindung der Formel I oder dessen Isomer
für die Herstellung einer pharmazeutischen Zusammensetzung für eine Supprimierung von EADs bei einem Säuger.

19. Verwendung nach Anspruch 18, wobei die Verbindung der Formel I Ranolazin, namentlich N-(2,6-Dimethylphenyl)-4-[2-hydroxy-3-(2-methoxyphenoxy)propyl]-1-piperazinacetamid, oder ein Isomer davon oder ein pharmazeutisch verträgliches Salz von Ranolazin oder dessen Isomer ist.

20. Verwendung nach Anspruch 18, wobei die pharmazeutische Zusammensetzung einer Verabreichung der Verbindung der Formel I bei Dosismengen dient, die I_{Kr}-, I_{Ks}- und späte I_{Na}-Ionenkanäle hemmen, jedoch nicht Calciumkanäle hemmen.

21. Verwendung nach Anspruch 19, wobei Ranolazin in der Form eines pharmazeutisch verträglichen Salzes vorliegt.

22. Verwendung nach Anspruch 21, wobei das pharmazeutisch verträgliche Salz das Dihydrochloridsalz ist.

23. Verwendung nach Anspruch 19, wobei Ranolazin in der Form der freien Base vorliegt.

24. Verwendung nach Anspruch 18, wobei die pharmazeutische Zusammensetzung einer Verabreichung der Verbindung der Formel I dient, die eine Dosismenge umfasst, die späte I_{Na}-Ionenkanäle hemmt.

25. Verwendung nach Anspruch 18, wobei die pharmazeutische Zusammensetzung einer Verabreichung der Verbindung der Formel I dient, die eine Dosismenge umfasst, die I_{Kr}-, I_{Ks}- und späte I_{Na}-Ionenkanäle hemmt.

26. Verwendung nach Anspruch 18, wobei die pharmazeutische Zusammensetzung einer Verabreichung der Verbindung der Formel I dient, die eine Dosismenge umfasst, die I_{Kr}-, I_{Ks}- und späte I_{Na}-Ionenkanäle hemmt, jedoch nicht Calciumkanäle hemmt.

27. Verwendung nach Anspruch 18, wobei die pharmazeutische Zusammensetzung einer Verabreichung der Verbindung der Formel I in einer Weise dient, die Plasmaspiegel der Verbindung der Formel I von mindestens 350±30 ng/ml für mindestens 12 Stunden bereitstellt.

28. Verwendung nach Anspruch 18, wobei die pharmazeutische Zusammensetzung eine Formulierung mit anhaltender Freisetzung ist, die Plasmakonzentrationen der Verbindung bei weniger als einem Maximum von 4000 ng/ml, vorzugsweise zwischen etwa 350 bis etwa 4000 ng/ml für mindestens 12 Stunden aufrechterhält.

29. Verwendung nach Anspruch 18, wobei die pharmazeutische Zusammensetzung etwa 10 mg bis 700 mg der Verbindung enthält.

30. Verwendung nach Anspruch 29, wobei die Verbindung der Formel I Ranolazin oder ein Isomer davon oder ein pharmazeutisch verträgliches Salz von Ranolazin oder dessen Isomer ist.

31. Verwendung nach Anspruch 18, wobei die pharmazeutische Zusammensetzung eine Dosismenge von etwa 1 bis etwa 30 Mikromol pro Liter an pharmazeutischer Zusammensetzung bereitstellt.

32. Verwendung nach Anspruch 31, wobei die pharmazeutische Zusammensetzung eine Dosismenge von etwa 1 bis etwa 10 Mikromol pro Liter an pharmazeutischer Zusammensetzung bereitstellt.

33. Verwendung nach Anspruch 18, wobei die pharmazeutische Zusammensetzung durch eine Boluszusammensetzung oder durch eine Zusammensetzung mit anhaltender Freisetzung zu verabreichen ist.

34. Verwendung nach Anspruch 18, wobei die pharmazeutische Zusammensetzung intravenös zu verabreichen ist.

## Revendications

1. Composé choisi dans le groupe consistant en:
un composé de Formule I: 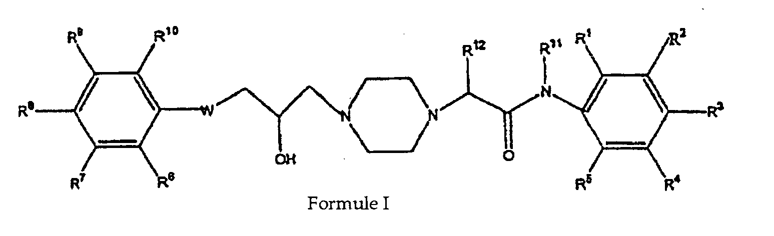 où:
R¹, R², R³, R⁴ et R⁵ sont chacun indépendamment l'hydrogène ou un groupe alkyle inférieur, alcoxy inférieur, cyano, trifluorométhyle, halogéno, alkylthio inférieur, alkyl sulfinyle inférieur, alkyl sulfonyle inférieur, ou alkylamido N-substitué en option, à condition que quand R¹ est le méthyle, R⁴ n'est pas le méthyle; ou R² et R³ forment ensemble -OCH₂O-;
R⁶, R⁷, R⁸, R⁹ et R¹⁰ sont chacun indépendamment l'hydrogène ou un groupe acyle inférieur, aminocarbonylméthyle, cyano, alkyle inférieur, alcoxy inférieur, trifluorométhyle, halogéno, alkylthio inférieur, alkyl sulfinyle inférieur, alkyl sulfonyle inférieur, ou di-(alkyl inférieur) amino; ou
R⁶ et R⁷ forment ensemble -CH=CH-CH=CH-; ou
R⁷ et R⁸ forment ensemble -OCH₂O-;
R¹¹ et R¹² sont chacun indépendamment l'hydrogène ou un groupe
alkyle inférieur; et
W est l'oxygène ou le soufre;
tandis que alkyle inférieur désigne une chaîne hydrocarbonée saturée non-ramifiée ayant 1-4 atomes de carbone et acyle inférieur désigne le groupe RC(O)A, où R est un alkyle inférieur et A représente le point de fixation,
un isomère d'un composé de Formule I, et un sel ou ester pharmaceutiquement acceptable d'un composé de Formule I ou son isomère
pour utilisation dans la suppression des post-dépolarisations précoces (EADs) chez un mammifère.

2. Composé pour l'utilisation selon la revendication 1, qui est la ranolazine, dénommée N-(2,6-diméthylphényl)-4-[2-hydroxy-3-(2-méthoxyphénoxy)propyl]-1-pipérazineacétamide, ou un isomère de celle-ci, ou un sel de ranolazine pharmaceutiquement acceptable ou son isomère.

3. Composé pour l'utilisation selon la revendication 1, qui est destiné à l'administration à des niveaux de dose qui inhibent les canaux ioniques I_{Kr}, I_{Ka}, et I_{Na} tardif, mais n'inhibent pas les canaux calciques.

4. Composé pour l'utilisation selon la revendication 2, qui est la ranolazine sous la forme d'un sel pharmaceutiquement acceptable.

5. Composé pour l'utilisation selon la revendication 4, dans lequel le sel pharmaceutiquement acceptable est le sel de dichlorhydrate.

6. Composé pour l'utilisation selon la revendication 2, qui est la ranolazine sous la forme de la base libre.

7. Composé pour l'utilisation selon la revendication 1, qui est destiné à l'administration comprenant un niveau de dose qui inhibe les canaux ioniques I_{Na} tardifs.

8. Composé pour l'utilisation selon la revendication 1, qui est destiné à l'administration comprenant un niveau de dose qui inhibe les canaux ioniques I_{Kr}, I_{Ka}, et I_{Na} tardif.

9. Composé pour l'utilisation selon la revendication 1, qui est destiné à l'administration comprenant un niveau de dose qui inhibe les canaux ioniques I_{Kr}, I_{Ka}, et I_{Na} tardif, mais n'inhibe pas les canaux calciques.

10. Composé pour l'utilisation selon la revendication 1, qui est destiné à l'administration d'une manière qui procure des niveaux plasmatiques du composé de Formule I d'au moins 350 ± 30 ng/mL pendant au moins 12 heures.

11. Composé pour l'utilisation selon la revendication 1, qui est destiné à l'administration dans une formulation à libération prolongée qui maintient les concentrations plasmatiques du composé à moins d'un maximum de 4000 ng/mL, de préférence entre environ 350 et environ 4000 ng/mL, pendant au moins 12 heures.

12. Composé pour l'utilisation selon la revendication 1, qui est destiné à l'administration dans une formulation qui contient entre environ 10 mg et 700 mg du dit composé.

13. Composé pour l'utilisation selon la revendication 12, qui est la ranolazine ou un isomère de celle-ci, ou un sel de ranolazine pharmaceutiquement acceptable ou son isomère.

14. Composé pour l'utilisation selon la revendication 1, qui est destiné à l'administration dans une formulation qui procure un niveau de dose d'environ 1 à environ 30 micromoles par litre de formulation.

15. Composé pour l'utilisation selon la revendication 14, dans lequel ladite formulation procure un niveau de dose d'environ 1 à environ 10 micromoles par litre de formulation.

16. Composé pour l'utilisation selon la revendication 1, qui est destiné à l'administration par embol ou par composition à libération prolongée.

17. Composé pour l'utilisation selon la revendication 1, qui est destiné à l'administration intraveineuse.

18. Utilisation d'un composé de Formule I: 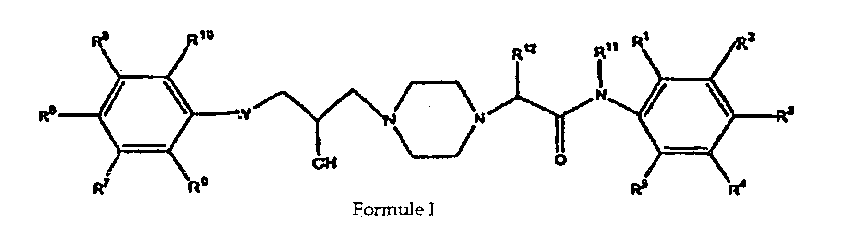 où:
R¹, R², R³, R⁴ et R⁵ sont chacun indépendamment l'hydrogène ou un groupe alkyle inférieur, alcoxy inférieur, cyano, trifluorométhyle, halogéno, alkylthio inférieur, alkyl sulfinyle inférieur, alkyl sulfonyle inférieur, ou alkylamido N-substitué en option, à condition que quand R¹ est le méthyle, R⁴ n'est pas le méthyle; ou R² et R³ forment ensemble -OCH₂O-;
R⁶, R⁷, R⁸, R⁹ et R¹⁰ sont chacun indépendamment l'hydrogène ou un groupe acyle inférieur, aminocarbonylméthyle, cyano, alkyle inférieur, alcoxy inférieur, trifluorométhyle, halogéno, alkylthio inférieur, alkyl sulfinyle inférieur, alkyl sulfonyle inférieur, ou di-(alkyl inférieur) amino; ou
R⁶ et R⁷ forment ensemble -CH=CH-CH=CH-; ou
R⁷ et R⁸ forment ensemble -OCH₂O-;
R¹¹ et R¹² sont chacun indépendamment l'hydrogène ou un groupe alkyle inférieur; et
W est l'oxygène ou le soufre;
tandis que alkyle inférieur désigne une chaîne hydrocarbonée saturée non-ramifiée ayant 1-4 atomes de carbone et acyle inférieur désigne le groupe RC(O)A, où R est un alkyle inférieur et A représente le point de fixation,
ou un de ses isomères, ou un sel ou ester pharmaceutiquement acceptable d'un composé de Formule I ou son isomère pour la préparation d'une composition pharmaceutique pour la suppression des EADs chez un mammifère.

19. Utilisation selon la revendication 18, dans laquelle le composé de Formule 1 est la ranolazine, dénommée N-(2,6-diméthylphényl)-4-[2-hydroxy-3-(2-méthoxyphénoxy)propyl]-1-pipérazineacétamide, ou un isomère de celle-ci, ou un sel de ranolazine pharmaceutiquement acceptable ou son isomère.

20. Utilisation selon la revendication 18, dans laquelle la composition pharmaceutique est destinée à l'administration du composé de Formule I à des niveaux de dose qui inhibent les canaux ioniques I_{Kr}, I_{Ka}, et I_{Na} tardif, mais n'inhibent pas les canaux calciques.

21. Utilisation selon la revendication 19, dans laquelle la ranolazine est sous la forme d'un sel pharmaceutiquement acceptable.

22. Utilisation selon la revendication 21, dans laquelle le sel pharmaceutiquement acceptable est le sel de dichlorhydrate.

23. Utilisation selon la revendication 19, dans laquelle la ranolazine est sous la forme de la base libre.

24. Utilisation selon la revendication 18, dans laquelle la composition pharmaceutique destinée à l'administration du composé de Formule I comprend un niveau de dose qui inhibe les canaux ioniques I_{Na} tardifs.

25. Utilisation selon la revendication 18, dans laquelle la composition pharmaceutique est destinée à l'administration du composé de Formule I comprenant un niveau de dose qui inhibe les canaux ioniques I_{Kr}, I_{Ka}, et I_{Na} tardif.

26. Utilisation selon la revendication 18, dans laquelle la composition pharmaceutique est destinée à l'administration du composé de Formule I comprenant un niveau de dose qui inhibe les canaux ioniques I_{Kr}, I_{Ka}, et I_{Na} tardif, mais n'inhibe pas les canaux calciques.

27. Utilisation selon la revendication 18, dans laquelle la composition pharmaceutique est destinée à l'administration du composé de Formule I d'une manière qui procure des niveaux plasmatiques du composé de Formule I d'au moins 350 ± 30 ng/mL pendant au moins 12 heures.

28. Utilisation selon la revendication 18, dans laquelle la composition pharmaceutique est une formulation à libération prolongée qui maintient les concentrations plasmatiques du composé à moins d'un maximum de 4000 ng/mL, de préférence entre environ 350 et environ 4000 ng/mL, pendant au moins 12 heures.

29. Utilisation selon la revendication 18, dans laquelle la composition pharmaceutique contient entre environ 10 mg et 700 mg du dit composé.

30. Utilisation selon la revendication 29, dans laquelle le composé de Formule I est la ranolazine ou un isomère de celle-ci, ou un sel de ranolazine pharmaceutiquement acceptable ou son isomère.

31. Utilisation selon la revendication 18, dans laquelle la composition pharmaceutique procure un niveau de dose d'environ 1 à environ 30 micromoles par litre de composition pharmaceutique.

32. Utilisation selon la revendication 31, dans laquelle la composition pharmaceutique procure un niveau de dose d'environ 1 à environ 10 micromoles par litre de composition pharmaceutique.

33. Utilisation selon la revendication 18, dans laquelle la composition pharmaceutique est destinée à l'administration par embol ou par composition à libération prolongée.

34. Utilisation selon la revendication 18, dans laquelle la composition pharmaceutique est destinée à être administrée par voie intraveineuse.
